# EUROPÄISCHE PATENTSCHRIFT

(11) **EP 2 438 067 B1**
(45) Veröffentlichungstag und Bekanntmachung des Hinweises auf die Patenterteilung: **26.08.2015**
(21) Anmeldenummer: 10724458.4
(22) Anmeldetag: 02.06.2010
(51) Int. Cl.: C07D 471/04, C07D 471/20, C07D 491/10, C07D 519/00, A61K 31/499, A61P 25/06

(54) **SPIROLACTAME ALS CGRP-ANTAGONISTEN**
SPIROLACTAMS AS CGRP-ANTAGONISTS
SPIROLACTAMES COMME ANTAGONISTES DU CGRP

(30) Priorität: 05.06.2009 EP 09162068; 29.10.2009 EP 09174459
(43) Veröffentlichungstag der Anmeldung: 11.04.2012
(73) Patentinhaber: Boehringer Ingelheim International GmbH, 55216 Ingelheim am Rhein (DE)
(72) Erfinder: DREYER, Alexander, 55216 Ingelheim am Rhein (DE); DOODS, Henri, 55216 Ingelheim am Rhein (DE); GERLACH, Kai, 55216 Ingelheim am Rhein (DE); GOTTSCHLING, Dirk, 55216 Ingelheim am Rhein (DE); HEIMANN, Annekatrin, 55216 Ingelheim am Rhein (DE); MUELLER, Stephan Georg, 55216 Ingelheim am Rhein (DE); RUDOLF, Klaus, 55216 Ingelheim am Rhein (DE); SCHAENZLE, Gerhard, 55216 Ingelheim am Rhein (DE)
(74) Vertreter: Simon, Elke Anna Maria
(86) Internationale Anmeldenummer: PCT/EP2010/057693
(87) Internationale Veröffentlichungsnummer: WO 2010/139717

(56) Entgegenhaltungen:
- WO-A1-2008/020902
- DOODS ET AL: "CGRP antagonists: unravelling the role of CGRP in migraine" TRENDS IN PHARMACOLOGICAL SCIENCES, ELSEVIER, HAYWARTH, GB, Bd. 28, Nr. 11, 25. Oktober 2007 (2007-10-25), Seiten 580-587, XP022327127 ISSN: 0165-6147

## Beschreibung

Gegenstand der vorliegenden Erfindung sind neue CGRP-Antagonisten der allgemeinen Formel I 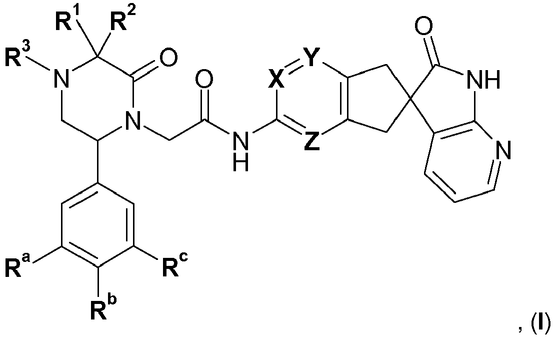 in der **R¹**, **R²**, **R³**, **R^{a}**, **R^{b}**, **R^{c}**, **X, Y** und .**Z** wie in den Ansprüchen definiert sind, deren individuelle Diastereomere, deren individuelle Enantiomere und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Im Stand der Technik werden bereits strukturähnliche Verbindungen mit CGRP-antagonistischen Eigenschaften beschrieben: WO 2008/020902. Die Verbindungen der vorliegenden Anmeldung unterscheiden sich vom Stand der Technik im Wesentlichen durch die Wahl der Reste **R¹** und **R²**.

Sie weisen aufgrund dieses strukturellen Unterschieds potentiell verbesserte mikrosomale Stabilitäten auf. Das führt zu längeren Halbwertszeiten, d.h. zu längeren Verweildauern der Substanzen im Blutkreislauf.

### DETAILLIERTE BESCHREIBUNG DER ERFINDUNG

Die Erfindung ist durch die beigefügten Ansprüche definiert. Die folgende Beschreibung unterliegt dieser Einschränkung. Alle Ausführungsformen, die im folgenden z.B. als "Ausführungsformen der vorliegenden Erfindung" bezeichnet sind aber nicht unter den Schutzumfang der Ansprüche fallen sind lediglich Ausführungsformen der vorliegenden Offenbarung und nicht Teil der Erfindung. In der allgemeinen Formel **I** bedeutet in einer ersten Ausführungsform
- **X**: C-H, C-Cl oder N,
- **Y, Z**: unabhängig voneinander jeweils CH oder N,
wobei maximal nur ein Substituent **X, Y** oder **Z** ein Stickstoffatom bedeutet,
(a)
   - **R¹**: H,
   - **R²**: **R**^{2.1}-C₀₋₁-alkylen-,
   - **R^{2.1}**: eine Gruppe ausgewählt aus 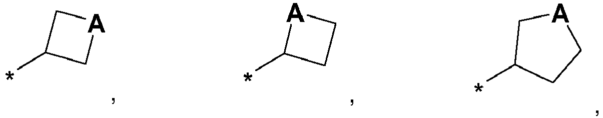 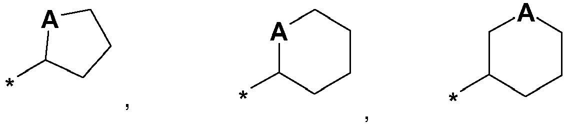 oder 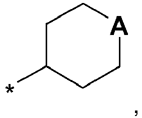 und
   - **A**: -O-, -S-, -S(O)- oder -S(O₂)-; oder
(b)
   - **R¹**: H,
   - **R²**: **R^{2.1}**-CH₂- und
   - **R^{2.1}**: eine Gruppe 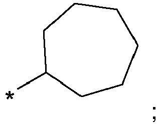 oder
(c) **R¹** und **R²** zusammen mit dem Kohlenstoffatom, an das sie gebunden sind, eine C₄₋₆-Cycloalkylgruppe, die jeweils mit einem Oxetan- oder Tetrahydropyranring spirocyclisch verknüpft ist; oder
(d) **R¹** und **R²** zusammen mit dem Kohlenstoffatom, an das sie gebunden sind, einen Rest 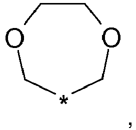 und
   - **R³**: H, -C(O)-O-C₁₋₄-Alkyl oder eine C₁₋₆-Alkylgruppe, die mit 1, 2, 3, 4 oder 5 Fluor-atomen substituiert sein kann,
   - **R^{a}**: H, F, -OCH₃ oder -OCF₃,
   - **R^{b}**: H, F, -OCH₃ oder -OCF₃, und
   - **R^{c}**: H, F, -OCH₃ oder -OCF₃,
deren individuelle Diastereomere, deren individuelle Enantiomere und deren Salze, insbesondere deren physiologisch verträgliche Salze mit anorganischen oder organischen Säuren oder Basen.

Eine zweite Ausführungsform der vorliegenden Erfindung besteht in den Verbindungen der obigen allgemeinen Formel **I,** in denen
**X, Y, Z** jeweils C-H oder N, wobei maximal nur ein N im Ring enthalten ist,
(a)
   - **R¹**: H und
   - **R²**: eine Gruppe ausgewählt aus 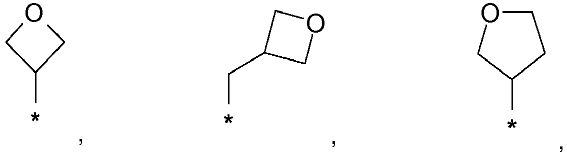 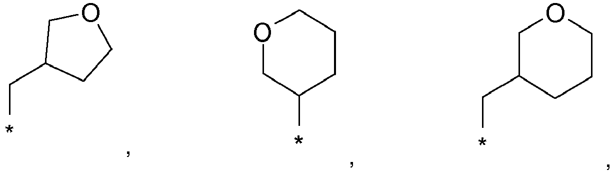 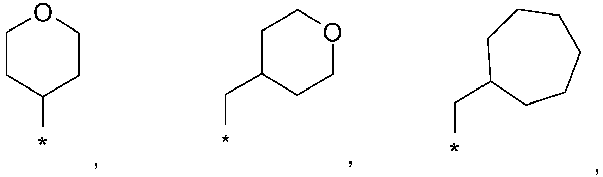 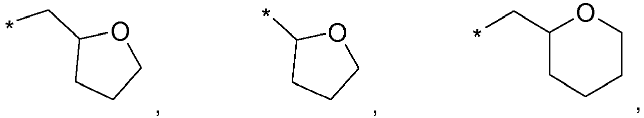 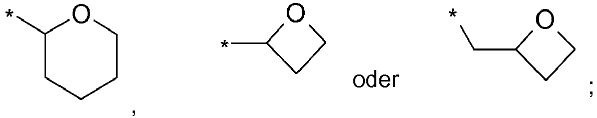 oder
(b) **R¹** und **R²** zusammen mit dem Kohlenstoffatom, an das sie gebunden sind, eine Gruppe ausgewählt aus 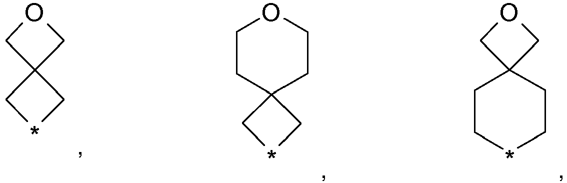 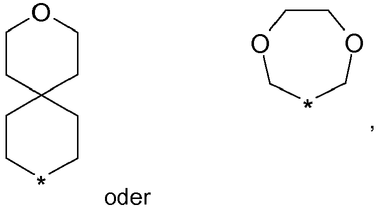 und
   - **R³**: H, -C(O)-O-C₁₋₄-Alkyl oder eine C₁₋₆-Alkylgruppe, die mit 1, 2, 3, 4 oder 5 Fluor-atomen substituiert sein kann,
   - **R^{a}**: H, F, -OCH₃ oder -OCF₃,
   - **R^{b}**: H, F, -OCH₃ oder -OCF₃, und
   - **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel **I**, in denen
- **X, Y, Z**: jeweils C-H oder N, wobei maximal nur ein N im Ring enthalten ist,
- **R¹**: H,
- **R²**: eine Gruppe ausgewählt aus 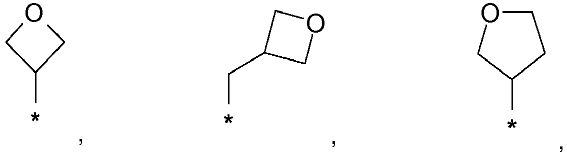 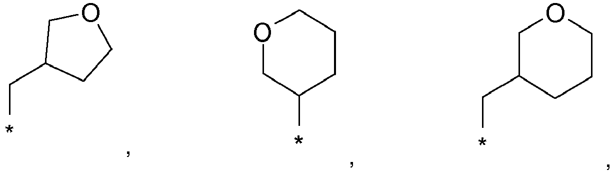 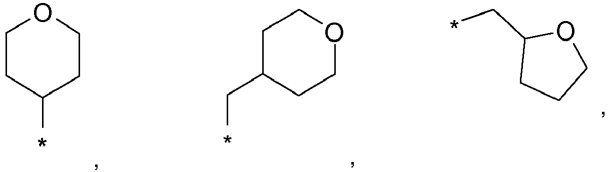 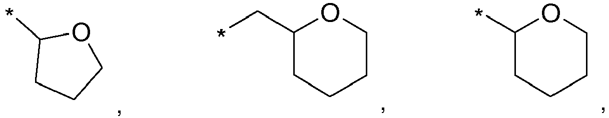 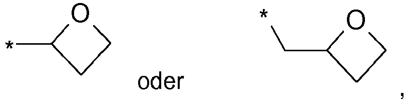
- **R³**: H, -C(O)-O-C₁₋₄-Alkyl oder eine C₁₋₆-Alkylgruppe, die mit 1, 2, 3, 4 oder 5 Fluor-atomen substituiert sein kann,
- **R^{a}**: H, F, -OCH₃ oder -OCF₃,
- **R^{b}**: H, F, -OCH₃ oder -OCF₃, und
- **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine vierte Ausführungsform der vorliegenden Erfindung besteht in den Verbindungen der obigen allgemeinen Formel **I**, in denen
- **X, Y, Z**: jeweils C-H oder N, wobei maximal nur ein N im Ring enthalten ist,
- **R¹**: H,
- **R²**: eine Gruppe 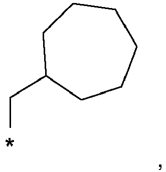
- **R³**: H, -C(O)-O-C₁₋₄-Alkyl oder eine C₁₋₆-Alkylgruppe, die mit 1, 2, 3, 4 oder 5 Fluor-atomen substituiert sein kann,
- **R^{a}**: H, F, -OCH₃ oder -OCF₃,
- **R^{b}**: H, F, -OCH₃ oder -OCF₃, und
- **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine fünfte Ausführungsform der vorliegenden Erfindung besteht in den Verbindungen der obigen allgemeinen Formel **I**, in denen
**X, Y, Z** jeweils C-H oder N, wobei maximal nur ein N im Ring enthalten ist,
**R¹** und **R²** zusammen mit dem Kohlenstoffatom, an das sie gebunden sind, eine Gruppe ausgewählt aus 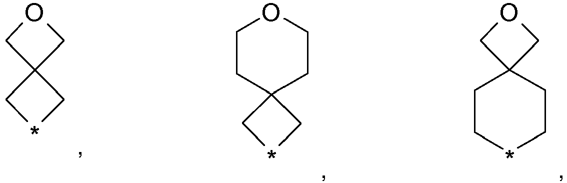 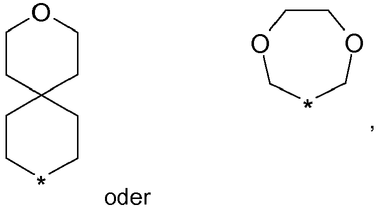
   - **R³**: H, -C(O)-O-C₁₋₄-Alkyl oder eine C₁₋₆-Alkylgruppe, die mit 1, 2, 3, 4 oder 5 Fluor-atomen substituiert sein kann,
   - **R^{a}**: H, F, -OCH₃ oder -OCF₃,
   - **R^{b}**: H, F, -OCH₃ oder -OCF₃, und
   - **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine sechste Ausführungsform der vorliegenden Erfindung besteht in den Verbindungen der obigen allgemeinen Formel **I**, in denen
**X, Y, Z** jeweils C-H oder N, wobei maximal nur ein N im Ring enthalten ist,
**R¹** und **R²** zusammen mit dem Kohlenstoffatom, an das sie gebunden sind, eine Gruppe 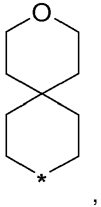
   - **R³**: H, -C(O)-O-C₁₋₄-Alkyl oder eine C₁₋₆-Alkylgruppe, die mit 1, 2, 3, 4 oder 5 Fluor-atomen substituiert sein kann,
   - **R^{a}**: H, F, -OCH₃ oder -OCF₃,
   - **R^{b}**: H, F, -OCH₃ oder -OCF₃, und
   - **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine siebte Ausführungsform der vorliegenden Erfindung besteht in den Verbindungen der obigen allgemeinen Formel **I**, in denen
**X, Y, Z** jeweils C-H oder N, wobei maximal nur ein N im Ring enthalten ist,
**R¹** und **R²** zusammen mit dem Kohlenstoffatom, an das sie gebunden sind, eine Gruppe 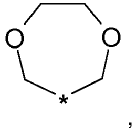
   - **R³**: H, -C(O)-O-C₁₋₄-Alkyl oder eine C₁₋₆-Alkylgruppe, die mit 1, 2, 3, 4 oder 5 Fluor-atomen substituiert sein kann,
   - **R^{a}**: H, F, -OCH₃ oder -OCF₃,
   - **R^{b}**: H, F, -OCH₃ oder -OCF₃, und
   - **R^{c}**: H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

Eine achte Ausführungsform der vorliegenden Erfindung besteht in den Verbindungen der allgemeinen Formel **Ia** 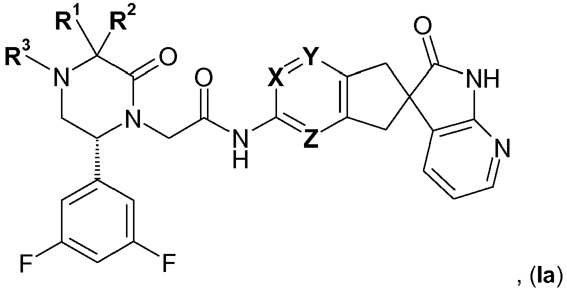 in denen **R¹, R², X, Y** und **Z** wie voranstehend unter der Ausführungsform 1, 2, 3, 4, 5, 6 oder 7 beschrieben definiert sind und
- **R³**: H oder CH₃ bedeutet,
deren individuelle Diastereomere, deren individuelle Enantiomere sowie deren Salze, insbesondere deren physiologisch verträgliche Salze mit anorganischen oder organischen Säuren oder Basen.

Eine neunte Ausführungsform der vorliegenden Erfindung besteht in den Verbindungen der allgemeinen Formel **Ib** 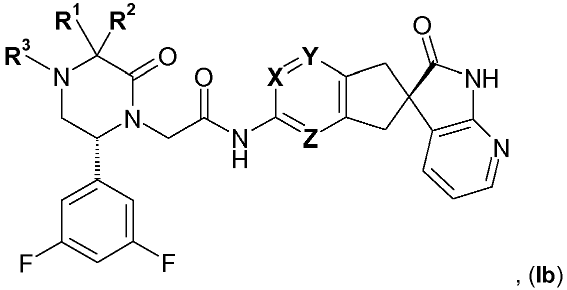 in denen **R¹**, **R²**, **X, Y** und **Z** wie voranstehend unter der Ausführungsform 1, 2, 3, 4, 5, 6 oder 7 beschrieben definiert sind und
- **R³**: H oder CH₃ bedeutet,
deren individuelle Diastereomere, deren individuelle Enantiomere sowie deren Salze, insbesondere deren physiologisch verträgliche Salze mit anorganischen oder organischen Säuren oder Basen.

Eine zehnte Ausführungsform der vorliegenden Erfindung besteht in den Verbindungen der allgemeinen Formel **Ic** 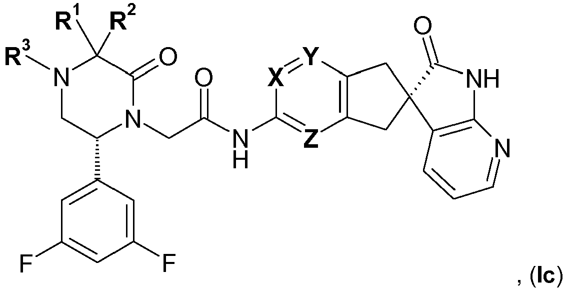 in denen **R¹**, **R²**, **X, Y** und **Z** wie voranstehend unter der Ausführungsform 1, 2, 3, 4, 5, 6 oder 7 beschrieben definiert sind und
- **R³**: H oder CH₃ bedeutet,
deren individuelle Diastereomere, deren individuelle Enantiomere sowie deren Salze, insbesondere deren physiologisch verträgliche Salze mit anorganischen oder organischen Säuren oder Basen.

Eine elfte Ausführungsform der vorliegenden Erfindung besteht in den Verbindungen der obigen allgemeinen Formel **I**, in der **R¹, R², R³, R^{a}, R^{b}** und **R^{c}** wie voranstehend unter der Ausführungsform 1, 2, 3, 4, 5, 6, oder 7 definiert sind,
- **X**: CH oder N,
- **Y**: CH und
- **Z**: CH bedeutet,
deren individuelle Diastereomere, deren individuelle Enantiomere sowie deren Salze, insbesondere deren physiologisch verträgliche Salze mit anorganischen oder organischen Säuren oder Basen.

Eine zwölfte Ausführungsform der vorliegenden Erfindung besteht in den Verbindungen der obigen allgemeinen Formel **Ia,** in der **R¹** und **R²** wie voranstehend unter der Ausführungsform 1, 2, 3, 4, 5, 6, oder 7 definiert sind,
- **R³**: H oder CH₃,
- **X**: CH oder N,
- **Y**: CH und
- **Z**: CH bedeutet,
deren individuelle Diastereomere, deren individuelle Enantiomere sowie deren Salze, insbesondere deren physiologisch verträgliche Salze mit anorganischen oder organischen Säuren oder Basen.

Eine dreizehnte Ausführungsform der vorliegenden Erfindung besteht in den Verbindungen der obigen allgemeinen Formel **Ib**, in der **R¹** und **R²** wie voranstehend unter der Ausführungsform 1, 2, 3, 4, 5, 6, oder 7 definiert sind,
- **R³**: H oder CH₃,
- **X**: CH oder N,
- **Y**: CH und
- **Z**: CH bedeutet,
deren individuelle Diastereomere, deren individuelle Enantiomere sowie deren Salze, insbesondere deren physiologisch verträgliche Salze mit anorganischen oder organischen Säuren oder Basen.

Eine vierzehnte Ausführungsform der vorliegenden Erfindung besteht in den Verbindungen der obigen allgemeinen Formel **Ic**, in der **R¹** und **R²** wie voranstehend unter der Ausführungsform 1, 2, 3, 4, 5, 6, oder 7 definiert sind,
- **R³**: H oder CH₃,
- **X**: CH oder N,
- **Y**: CH und
- **Z**: CH bedeutet,
deren individuelle Diastereomere, deren individuelle Enantiomere sowie deren Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise folgende Verbindungen genannt:

| **Nr.** | **Struktur** |
|---|---|
| (1) | 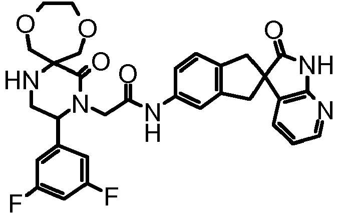 |
| (1a) | 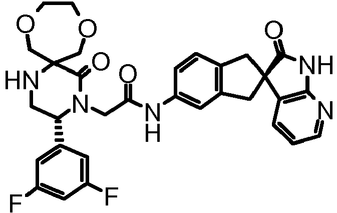 |
| (1b) | 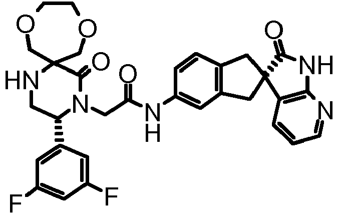 |
| (2) | 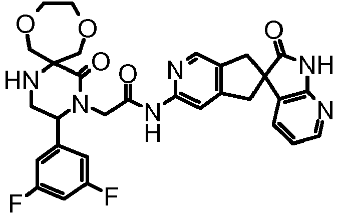 |
| (3) | 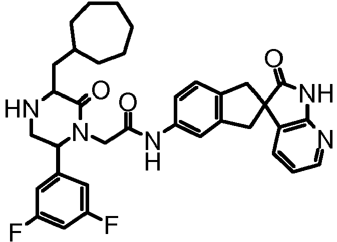 |
| (4) | 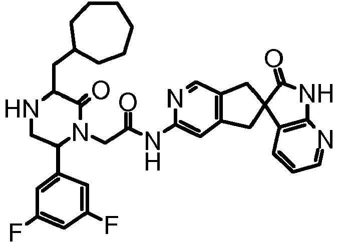 |
| (5) | 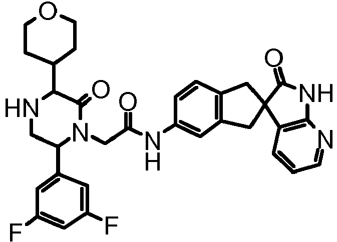 |
| (6) | 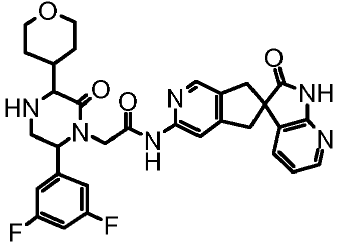 |
| (7) | 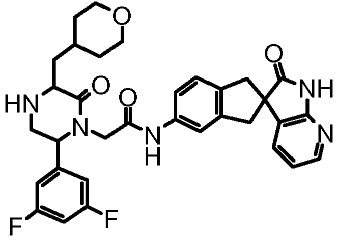 |
| (8) | 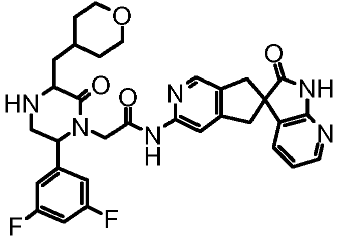 |
| (9) | 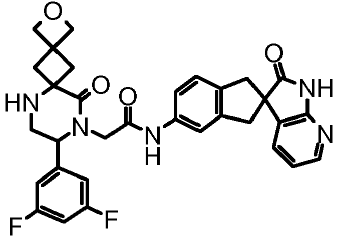 |
| (10) | 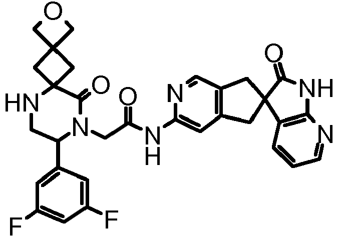 |
| (11) | 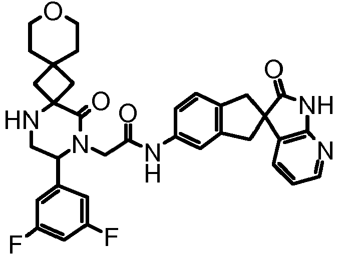 |
| (12) | 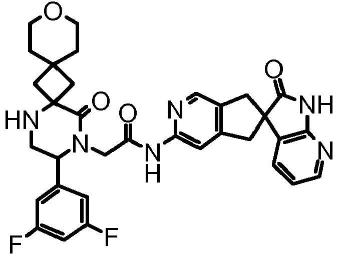 |
| (13) | 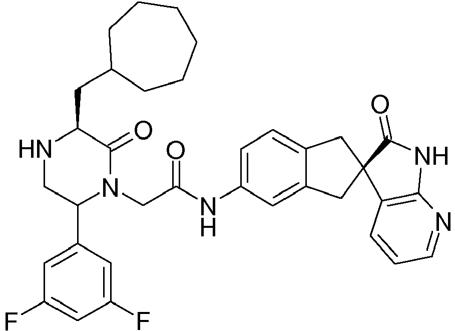 |
| (14) | 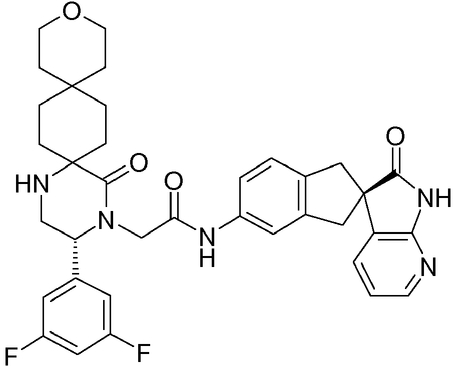 |
| (15) | 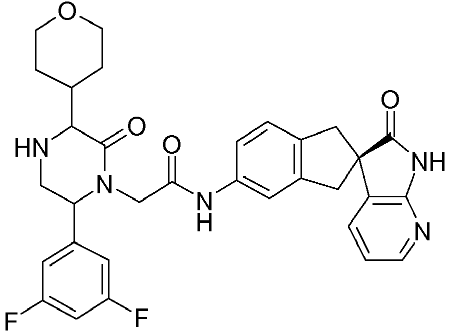 |
| (16) | 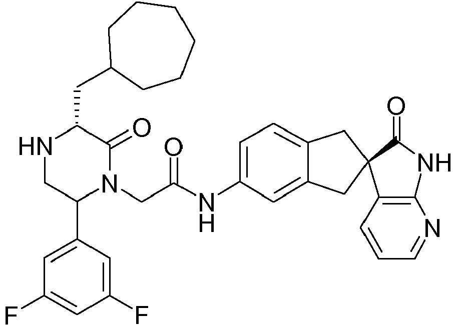 |
| (17) | 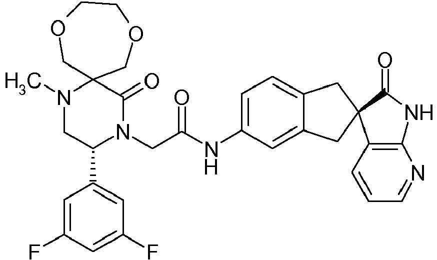 |
| (18) | 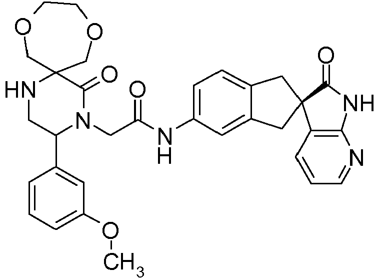 |
| (19) | 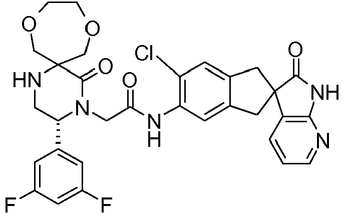 |
| (20) | 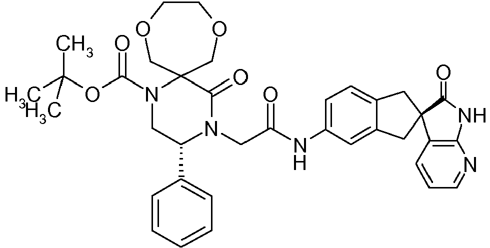 |
| (21) | 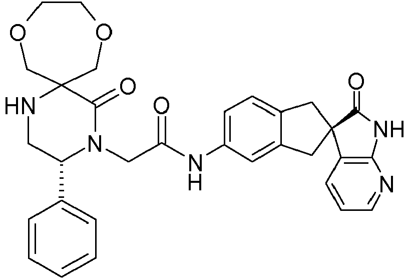 |
| (22) | 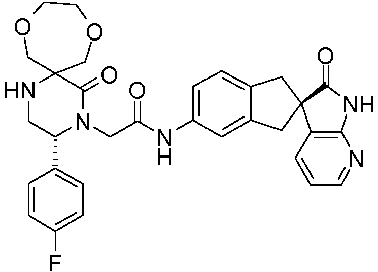 |
| (23) | 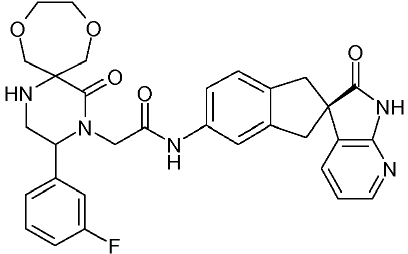 |
| (24) | 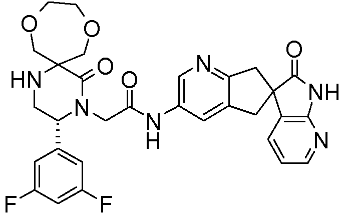 |

deren individuelle Diastereomere, deren individuelle Enantiomere und deren Salze, insbesondere deren physiologisch verträgliche Salze mit anorganischen oder organischen Säuren oder Basen.

Ein weiterer Gegenstand der vorliegenden Erfindung sind die Verbindungen der allgemeinen Formel **II** 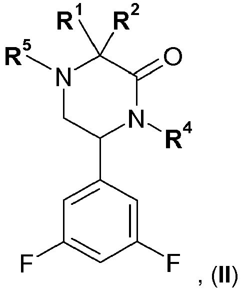 in der
- **R¹**: H,
- **R²**: eine Gruppe ausgewählt aus 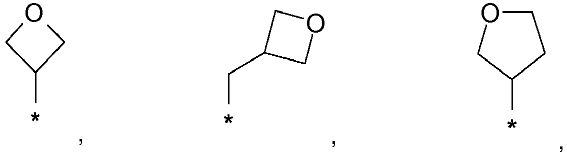 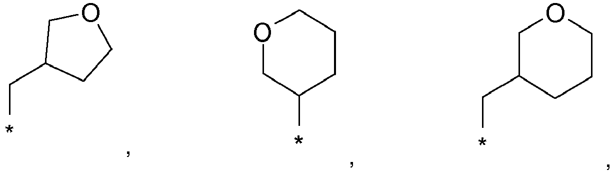 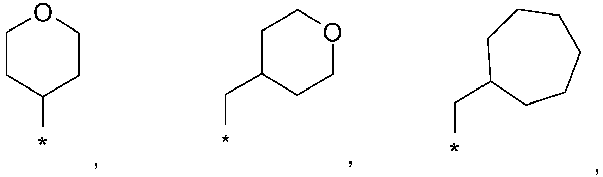 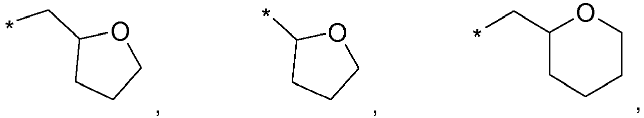 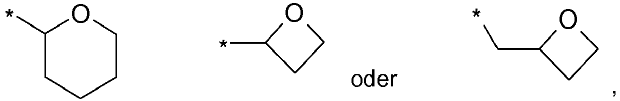
- **R⁴**: H oder CH₂-C(O)-O**R**^{4.1},
- **R^{4.1}**: H, C₁₋₆-Alkyl oder Benzyl,
- **R⁵**: H, Benzyl oder -C(O)-O**R^{5.1}**, bevorzugt H oder -C(O)-O-*tert*-Butyl, und
- **R^{5.1}**: C₁₋₆-Alkyl oder Benzyl bedeutet,
deren individuelle Diastereomere, deren individuelle Enantiomere und deren Salze, insbesondere deren physiologisch verträgliche Salze mit anorganischen oder organischen Säuren oder Basen.

Ein weiter bevorzugter Gegenstand der vorliegenden Erfindung sind die Verbindungen der allgemeinen Formel **IIa** 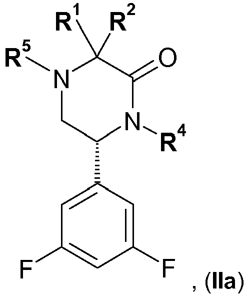 in der
- **R¹**: H,
- **R²**: eine Gruppe ausgewählt aus 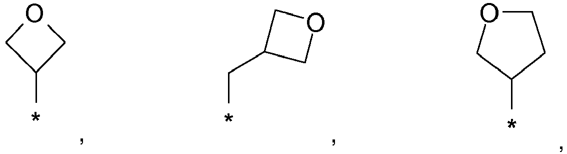 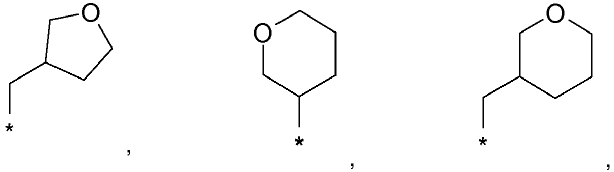 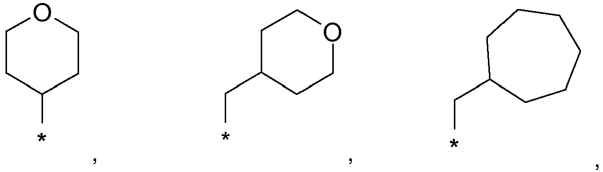 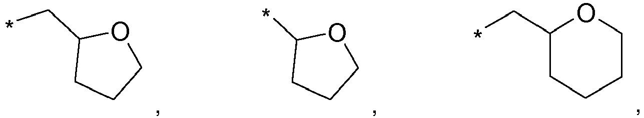 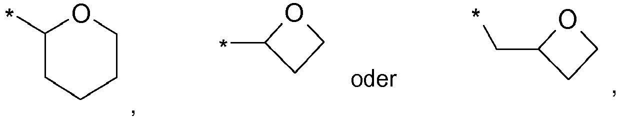
- **R⁴**: H oder-CH₂-C(O)-O**R^{4.1}**,
- **R^{4.1}**: H, C₁₋₆-Alkyl oder Benzyl,
- **R⁵**: H, Benzyl oder -C(O)-O**R^{5.1}**, bevorzugt H oder -C(O)-O-*tert*-Butyl, und
- **R^{5.1}**: C₁₋₆-Alkyl oder Benzyl bedeutet,
deren individuelle Diastereomere, deren individuelle Enantiomere sowie deren Salze, insbesondere deren physiologisch verträgliche Salze mit anorganischen oder organischen Säuren oder Basen.

Ein weiterer Gegenstand der vorliegenden Erfindung ist ein Verfahren zur Herstellung von Verbindungen der allgemeinen Formel **II** 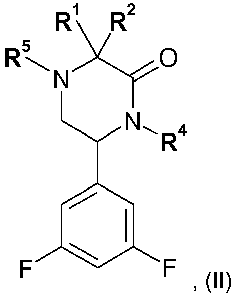 in der
- **R¹**: H,
- **R²**: eine Gruppe ausgewählt aus 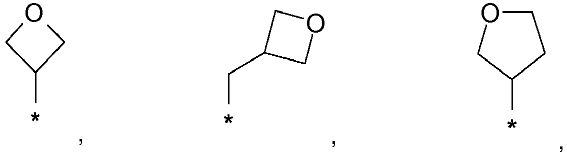 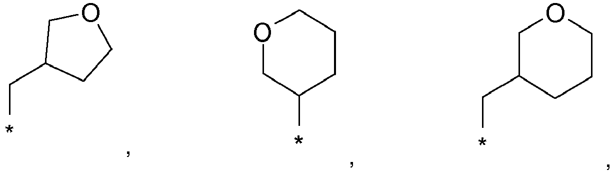 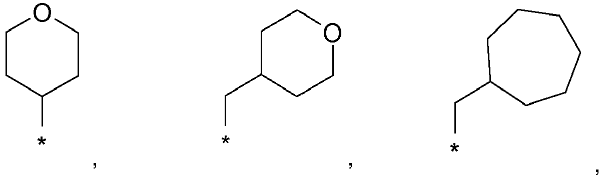 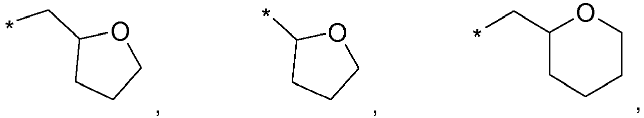 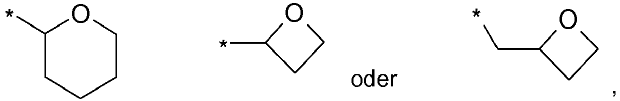
- **R⁴**: H oder -CH₂-C(O)-O**R^{4.1}**,
- R^{4.1}: H, C₁₋₆-Alkyl oder Benzyl,
- **R⁵**: H, Benzyl oder -C(O)-O**R^{5.1}**, bevorzugt H oder -C(O)-O-*tert*-Butyl, und
- **R^{5.1}**: C₁₋₆-Alkyl oder Benzyl bedeutet,
deren individuelle Diastereomere, deren individuelle Enantiomere und deren Salze, insbesondere deren physiologisch verträgliche Salze mit anorganischen oder organischen Säuren oder Basen,
umfassend die Schritte:
(a) Umsetzung einer Verbindung der allgemeinen Formel **III** 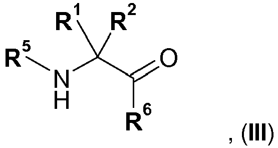 in der **R¹** und **R²** wie eingangs erwähnt definiert sind,
   - **R⁵**: Benzyl oder -C(O)-O**R^{5.1}**,
   - **R^{5.1}**: Benzyl und
   - **R⁶**: -O-C₁₋₆-Alkyl bedeutet,
   mit einer Verbindung der allgemeinen Formel **IV** 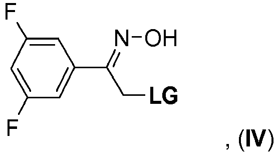 in der **LG** eine Abgangsgruppe darstellt, beispielsweise ein Brom- oder lodatom, eine Trifluormethansulfonyl-, Methansulfonyl- oder Toluolsulfonylgruppe, bevorzugt ein Bromatom;
(b) Überführen einer unter Schritt (a) erhaltenen Verbindung der allgemeinen Formel **V** 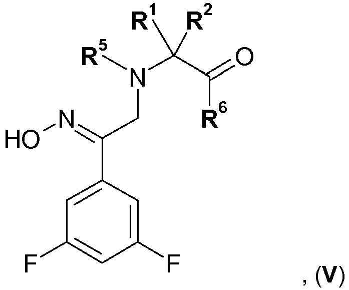 in der **R¹** und **R²** wie eingangs erwähnt definiert sind, **R⁵** Benzyl oder -C(O)-O-Benzyl und **R⁶** die Gruppe -O-C₁₋₆-Alkyl bedeutet, unter reduktiven Bedingungen, wie beispielsweise in einer Wasserstoffatmosphäre bei erhöhtem Druck und Temperatur und in Gegenwart eines Katalysators, beispielsweise in Gegenwart von Palladium auf Kohle, in eine Verbindung der allgemeinen Formel **VI** 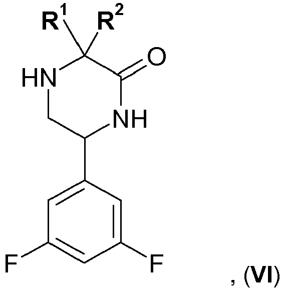 in der **R¹** und **R²** wie eingangs erwähnt definiert sind;
(c) Überführen einer unter Schritt (b) erhaltenen Verbindung der allgemeinen Formel **VI** in eine Verbindung der allgemeinen Formel VII 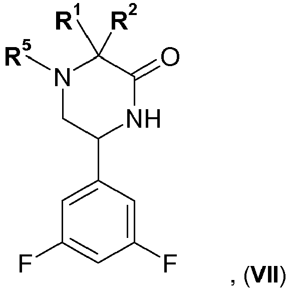 in der **R¹** und **R²** wie eingangs erwähnt definiert sind,
   - **R⁵**: Benzyl oder -C(O)-O**R^{5.1}** und
   - **R^{5.1}**: C₁₋₆-Alkyl oder Benzyl bedeutet;
(d) Umsetzung einer unter Schritt (c) erhaltenen Verbindung der allgemeinen Formel **VII** mit einer Verbindung der allgemeinen Formel **VIII** 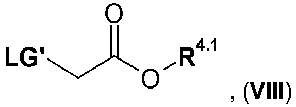 in der **LG'** eine Abgangsgruppe darstellt, beispielsweise ein Brom- oder lodatom, eine Trifluormethansulfonyl-, Methansulfonyl- oder Toluolsulfonylgruppe, bevorzugt ein Bromatom, und
   - **R^{4.1}**: H, C₁₋₆-Alkyl oder Benzyl bedeutet; und
(e) Isolieren einer unter Schritt (d) erhaltenen Verbindung der allgemeinen Formel **II,** in der **R¹**, **R²**, **R⁴** und **R⁵** wie eingangs erwähnt definiert sind.

Die unter Schritt (c) beschriebene Überführung kann nach allgemein bekannten Methoden und wie bespielsweise in T.W. Greene, P.G.M. Wuts, "Protective Groups in Organic Synthesis", Wiley, 1991 und 1999 beschrieben durchgeführt werden.

Ein weiterer Gegenstand der vorliegenden Anmeldung ist die Verwendung der Verbindungen der allgemeinen Formel **II,** in der **R¹**, **R²**, **R⁴** und **R⁵** wie eingangs erwähnt definiert sind, deren individuelle Diastereomere, deren individuelle Enantiomere und deren Salze, insbesondere deren physiologisch verträgliche Salze mit anorganischen oder organischen Säuren oder Basen zur Herstellung von Verbindungen der allgemeinen Formel **I**, welche CGRP-antagonistische Eigenschaften besitzen.

Ein weiterer Gegenstand der vorliegenden Anmeldung sind die Verbindungen der allgemeinen Formel **IV**, in der **LG** wie voranstehend erwähnt definiert ist, sowie deren Verwendung als Bausteine zur Herstellung von Verbindungen der allgemeinen Formel **II.**

Ein weiterer Gegenstand der vorliegenden Anmeldung sind die Verbindungen der allgemeinen Formel **IX** 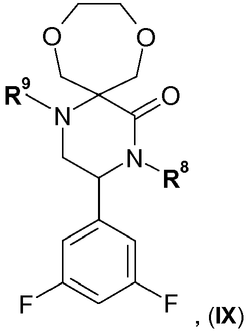 in der
- **R⁸**: H oder -CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen.

Ein weiter bevorzugter Gegenstand der vorliegenden Erfindung sind die Verbindungen der allgemeinen Formel **IXa** 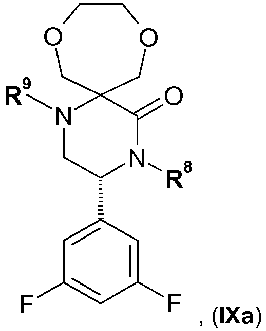 in der
- **R⁸**: H oder -CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere sowie deren Salze, insbesondere deren physiologisch verträgliche Salze mit anorganischen oder organischen Säuren oder Basen.

Ein weiterer Gegenstand der vorliegenden Anmeldung betrifft ein Verfahren zur Herstellung von Verbindungen der allgemeinen Formel IX, 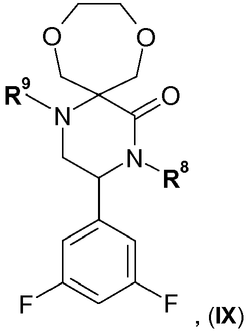 in der
- **R⁸**: H oder -CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen,
umfassend die Schritte:
(a) Umsetzung einer Verbindung der allgemeinen Formel X 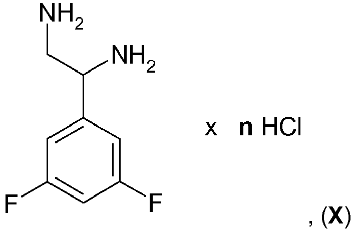 in der n eine der Ziffern 0, 1 oder 2 darstellt, mit [1,4]Dioxepan-6-on in Gegenwart eines Phasentransfer-Katalysators, wie beispielsweise Benzyltriethylammonium-chlorid, sowie in Gegenwart von Chloroform und Natronlauge;
(b) Isolieren einer unter Schritt (a) erhaltenen Verbindung der Formel XI 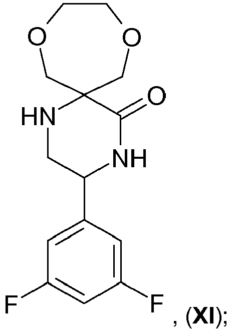
(c) Überführen einer unter Schritt (b) erhaltenen Verbindung der Formel **XI** in eine Verbindung der allgemeinen Formel IX 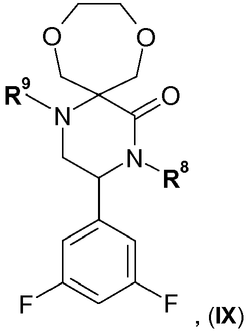 in der
   - **R⁸**: H und
   - **R⁹**: Benzyl, Benzyl-O-C(O)- oder C₁₋₆-Alkyl-O-C(O)- bedeutet;
(d) Umsetzung einer unter Schritt (c) erhaltenen Verbindung der allgemeinen Formel **IX** mit einer Verbindung der allgemeinen Formel **VIII** 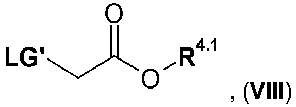 in der **LG'** eine Abgangsgruppe darstellt, beispielsweise ein Brom- oder lodatom, eine Trifluormethansulfonyl-, Methansulfonyl- oder Toluolsulfonylgruppe, bevorzugt ein Bromatom und
   - **R^{4.1}**: H, C₁₋₆-Alkyl oder Benzyl bedeutet; und
(e) Isolieren einer unter Schritt (d) erhaltenen Verbindung der allgemeinen Formel **IX** 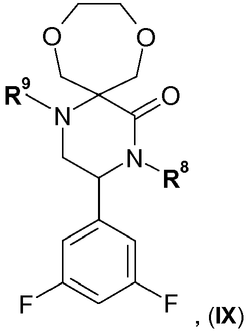 in der
   - **R⁸**: -CH₂-C(O)-O-**R^{8.1}**,
   - **R^{8.1}**: H, C₁₋₆-Alkyl oder Benzyl und
   - **R⁹**: Benzyl, Benzyl-O-C(O)- oder C₁₋₆-Alkyl-O-C(O)- bedeutet.

Die unter Schritt (c) beschriebene Überführung kann nach allgemein bekannten Methoden und wie bespielsweise in T.W. Greene, P.G.M. Wuts, "Protective Groups in Organic Synthesis", Wiley, 1991 und 1999 beschrieben durchgeführt werden.

Ein weiterer Gegenstand der vorliegenden Anmeldung ist die Verwendung der Verbindungen der allgemeinen Formel **IX**, in der **R⁸** und **R⁹** wie eingangs erwähnt definiert sind, deren individuelle Diastereomere, deren individuelle Enantiomere und deren Salze, insbesondere deren physiologisch verträgliche Salze mit anorganischen oder organischen Säuren oder Basen zur Herstellung von Verbindungen der allgemeinen Formel **I**, welche CGRP-antagonistische Eigenschaften besitzen.

Ein weiterer Gegenstand der vorliegenden Erfindung sind die Verbindungen der allgemeinen Formel **XII** 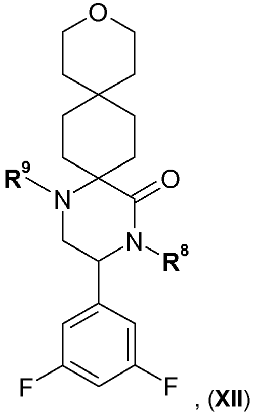 in der
- **R⁸**: H oder -CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen,

Ein weiter bevorzugter Gegenstand der vorliegenden Erfindung sind die Verbindungen der allgemeinen Formel **XIIa** 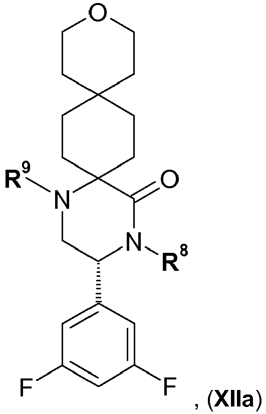 in der
- **R⁸**: H oder -CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere sowie deren Salze, insbesondere deren physiologisch verträgliche Salze mit anorganischen oder organischen Säuren oder Basen.

Ein weiterer Gegenstand der vorliegenden Anmeldung betrifft ein Verfahren zur Herstellung von Verbindungen der allgemeinen Formel **XII,** 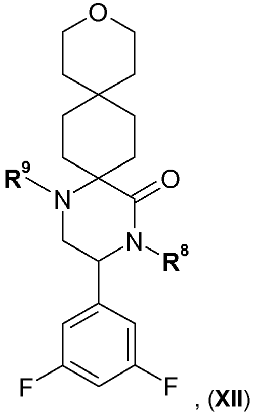 in der
- **R⁸**: H oder CH₂-C(O)-O-**R^{8.1}**,
- **R^{8.1}**: H, C₁₋₆-Alkyl oder Benzyl und
- **R⁹**: H, Benzyl, Benzyl-O-C(O)- oder C₁₋₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen,
umfassend die Schritte:
(a) Umsetzung einer Verbindung der allgemeinen Formel **X** 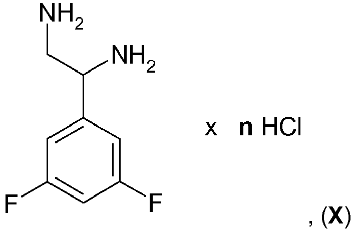 in der n eine der Ziffern 0, 1 oder 2 darstellt, mit 3-Oxaspiro[5.5]undecan-9-on in Gegenwart eines Phasentransfer-Katalysators, wie beispielsweise Benzyltriethylammoniumchlorid, sowie in Gegenwart von Chloroform und Natronlauge;
(b) Isolieren einer unter Schritt (a) erhaltenen Verbindung der Formel **XIII** 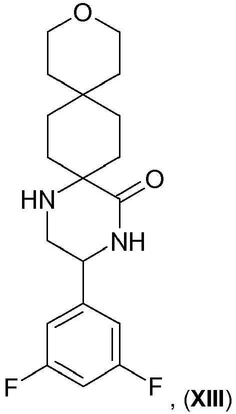
(c) Überführen einer unter Schritt (b) erhaltenen Verbindung der Formel **XIII** in eine Verbindung der allgemeinen Formel **XII** 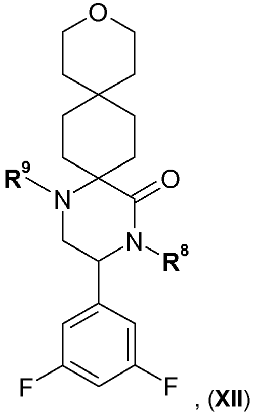 in der
   - **R⁸**: H und
   - **R⁹**: Benzyl, Benzyl-O-C(O)- oder C₁₋₆-Alkyl-O-C(O)- bedeutet;
(d) Umsetzung einer unter Schritt (c) erhaltenen Verbindung der allgemeinen Formel **XII** mit einer Verbindung der allgemeinen Formel **VIII** 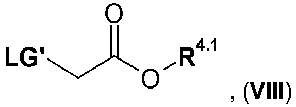 in der LG' eine Abgangsgruppe darstellt, beispielsweise ein Brom- oder lodatom, eine Trifluormethansulfonyl-, Methansulfonyl- oder Toluolsulfonylgruppe, bevorzugt ein Bromatom, und
   - **R^{4.1}**: H, C₁₋₆-Alkyl oder Benzyl bedeutet; und
(e) Isolieren einer unter Schritt (d) erhaltenen Verbindung der allgemeinen Formel **XII** 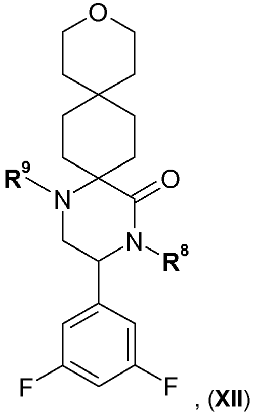 in der
   - **R⁸**: -CH₂-C(O)-O-**R^{8.1}**,
   - **R^{8.1}**: H, C₁₋₆-Alkyl oder Benzyl und
   - **R⁹**: Benzyl, Benzyl-O-C(O)- oder C₁₋₆-Alkyl-O-C(O)- bedeutet.

Die unter Schritt (c) beschriebene Überführung kann nach allgemein bekannten Methoden und wie bespielsweise in T.W. Greene, P.G.M. Wuts, "Protective Groups in Organic Synthesis", Wiley, 1991 und 1999 beschrieben durchgeführt werden.

Ein weiterer Gegenstand der vorliegenden Anmeldung ist die Verwendung der Verbindungen der allgemeinen Formel **XII,** in der **R⁸** und **R⁹** wie eingangs erwähnt definiert sind, deren individuelle Diastereomere, deren individuelle Enantiomere und deren Salze, insbesondere deren physiologisch verträgliche Salze mit anorganischen oder organischen Säuren oder Basen zur Herstellung von Verbindungen der allgemeinen Formel **I**, welche CGRP-antagonistische Eigenschaften besitzen.

Sind in Verbindungen der allgemeinen Formel (**II**), in der **R¹**, **R²**, **R⁴** und **R⁵** wie eingangs erwähnt definiert sind, in Verbindungen der allgemeinen Formel (**IX**), in der **R⁸** und **R⁹** wie eingangs erwähnt definiert sind, und in Verbindungen der allgemeinen Formel (**XII**), in der **R⁸** und **R⁹** wie eingangs erwähnt definiert sind, Esterfunktionalitäten enthalten, so können diese nach allgemein bekannten Verfahren wie sie auch in T.W. Greene, P.G.M. Wuts, "Protective Groups in Organic Synthesis", Wiley, 1991 und 1999 beschrieben sind, in die entsprechende Säure überführt werden.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.
Die Verbindungen, die von dieser Erfindung umfasst sind, sind jene, die auch chemisch stabil sind.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

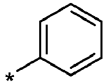

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Ebenfalls mit vom Gegenstand dieser Erfindung umfasst sind die erfindungsgemäßen Verbindungen, einschließlich deren Salze, in denen ein Kohlenstoffatom, durch Silizium ausgetauscht ist.

Ebenfalls mit vom Gegenstand dieser Erfindung umfasst sind die erfindungsgemäßen Verbindungen, die als Tautomere vorliegen.

Unter dem Begriff "C₁₋₄-Alkyl" (auch soweit sie Bestandteil anderer Reste sind) werden verzweigte und unverzweigte Alkylgruppen mit 1, 2, 3 oder 4 Kohlenstoffatomen und unter dem Begriff "C₁₋₆-Alkyl" werden verzweigte und unverzweigte Alkylgruppen mit 1, 2, 3, 4, 5 oder 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, Pentyl, Neopentyl oder *n*-Hexyl. Gegebenenfalls werden für die vorstehend genannten Gruppen auch die Abkürzungen Me, Et, n-Pr, *i*-Pr, n-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁-Alkylen" (auch soweit sie Bestandteil anderer Reste sind) wird eine Methylengruppe mit einem Kohlenstoffatom verstanden.
Die Definition für C₀-Alkylen bedeutet eine Bindung.

Unter dem Begriff "C₄₋₆-Cycloalkyl" (auch soweit sie Bestandteil anderer Reste sind) werden cyclische Alkylgruppen mit 4, 5 oder 6 Kohlenstoffatomen verstanden. Beispielsweise werden hierfür genannt: Cyclobutyl, Cyclopentyl oder Cyclohexyl.

Verbindungen der allgemeinen Formel **I** können saure und/oder basische Gruppen besitzen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkalialkoholaten oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren.

Sogenannte Prodrugs von Verbindungen der allgemeinen Formel **I** sind ebenfalls Gegenstand dieser Erfindung. Als Prodrug wird jedes Molekül bezeichnet, das nach Applikation an Säugetieren das aktive Prinzip der allgemeinen Formel **I** in-vivo freisetzt. Das Prodrug kann per se keine oder nur geringe pharmakologische Aktivität haben, es setzt allerdings nach Verabreichung in-vivo das aktive Prinzip der allgemeinen Formel **I** frei und dieses weist die beschriebene Aktivität auf. Prodrugs für Verbindungen der allgemeinen Formel **I** können hergestellt werden, indem geeignete funktionelle Gruppen in der Verbindung der allgemeinen Formel **I** so modifiziert werden, wie es einem Fachmann in diesem Gebiet bekannt ist. (H. Bundgaard (Editor), Design of Prodrugs. (1986), Elsevier)

Diese Erfindung umfasst auch jene Metaboliten, die sich aus den Verbindungen der allgemeinen Formel **I** ableiten. Unter Metaboliten versteht man in diesem Zusammenhang solche Verbindungen, die nach Applikation in-vivo aus der Verbindung der allgemeinen Formel **I** entstehen. Als Beispiel für Metaboliten werden genannt:
- Sekundäre Amine der Verbindung der allgemeinen Formel **I** können in die entsprechenden primären Amine überführt werden. (-NR₁R₂ -> -NHR₁ oder -NHR₂)
- Stickstoff-Atome der Verbindung der allgemeinen Formel **I** können in die entsprechenden Stickstoff-Oxide überführt werden. (=N- -> =N⁺-(O⁻)-)

### HERSTELLVERFAHREN

Gegenstand der Erfindung ist weiterhin ein Verfahren zur Herstellung der Verbindungen der allgemeinen Formel **I** 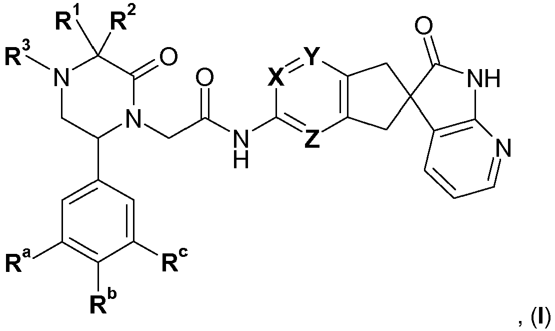 in **der X, Y, Z, R¹, R²**, **R³**, **R^{a}**, **R^{b}** und **R^{c}** wie voranstehend erwähnt definiert sind.

Einige Methoden zur Herstellung der erfindungsgemäßen Verbindungen der allgemeinen Formel **I** sowie deren Vorstufen sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung verständlich zu machen. Die Beispiele sollen der Anschaulichkeit der Erfindung dienen und sollen die Erfindung in keinster Weise einschränken.

Die erfindungsgemäßen Verbindungen sowie deren Intermediate können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.
Beispielsweise kommt
- als Schutzrest für eine Carboxylgruppe die Trimethylsilyl-, Methyl-,Ethyl-, *tert-*Butyl-, Benzyl- oder Tetrahydropyranylgruppe, und
- als Schutzrest für eine Amidgruppe die N-Methoxymethyl- (MOM), N-Benzyl-oxymethyl- (BOM), N-(Trimethylsilyl)ethoxymethyl- (SEM), N-*tert*-Butyldimethylsiloxymethyl-, N-*tert*-Butyldimethylsilyl- (TBDMS), N-Triisopropylsilyl- (TIPS), N-Benzyl-, N-4-Methoxybenzyl- (PMB), N-Triphenylmethyl- (Tr), N-*tert*-Butoxycarbonyl- (Boc), N-Benzyloxycarbonyl- (Cbz), N-Trimethylsilylethylsulfonyl- (SES);
- als Schutzrest für eine Amino-, Alkylamino- oder Iminogruppe die Acetyl-, Trifluoracetyl-, Benzoyl-, Ethoxycarbonyl-, *tert*-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht. Weitere Schutzgruppen und deren Abspaltung sind in T.W. Greene, P.G.M. Wuts, "Protective Groups in Organic Synthesis", Wiley, 1991 und 1999 beschrieben.

Eine allgemeine Synthese für Piperazinone der allgemeinen Formel (1-5), in der **R¹** und **R²** wie voranstehend erwähnt definiert sind, ist in Schema 1a dargestellt.

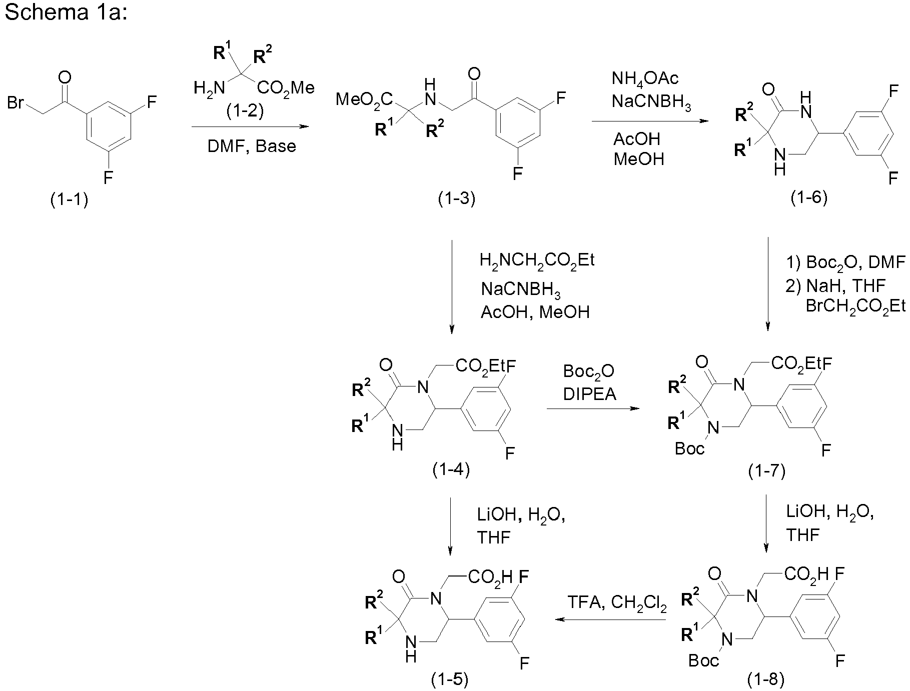

Die Umsetzung von 2-Brom-1-(3,5-difluor-phenyl)-ethanon (1-1) mit einem α-Aminoester der allgemeinen Formel (1-2), in der **R¹** und **R²** wie voranstehend erwähnt definiert sind, liefert das Aminoketon der allgemeinen Formel (1-3), in der **R¹** und **R²** wie voranstehend erwähnt definiert sind. Für diese Umsetzung können verschiedene Basen verwendet werden, beispielsweise NaHCO₃, K₂CO₃ und Na₃PO₄. Eine reduktive Aminierung des Aminoketons (1-3) mit Glycinethylester liefert unter geeigneten Reaktionsbedingungen das Piperazinon der allgemeinen Formel (1-4), in der **R¹** und **R²** wie voranstehend erwähnt definiert sind. Ester der allgemeinen Formel (1-4), in der **R¹** und **R²** wie voranstehend erwähnt definiert sind, können durch basische oder saure Hydrolyse (J. March, Advanced Organic Chemistry (New York: J. Wiley and Sons, 1985) oder durch Umsetzen mit Alkalimetallsalzen (bevorzugt Lil oder NaCN) in einem inerten Lösungsmittel in die Säure der allgemeinen Formel (1-5) überführt werden, in der **R¹** und **R²** wie voranstehend erwähnt definiert sind. Inerte Lösungsmittel können Dialkylformamide (besonders bevorzugt ist N,N-Dimethylformamid), Dialkylacetamide (besonders bevorzugt ist N,N-Dimethylacetamid), cyclische Amide (besonders bevorzugt ist N-Methylpyrrolidon) sein. Besonders bevorzugt ist eine alkalische Verseifung mit Alkalimetallhydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

Alternativ kann das Aminoketon der allgemeinen Formel (1-3) in einer reduktiven Aminierung mit Ammoniumacetat in das entsprechende Diamin überführt werden, das unter geeigneten Versuchsbedingungen gleich zu dem Piperazinon der allgemeinen Formel (1-6), in der **R¹** und **R²** wie voranstehend erwähnt definiert sind, cyclisiert. Das Piperazinon der allgemeinen Formel (1-6) kann unter geeigneten Reaktionsbedingungen zunächst in das entsprechende *tert*-Butylcarbamat und anschließend mit Bromessigsäureethylester zu dem Ester der allgemeinen Formel (1-7) umgesetzt werden, in der **R¹** und **R²** wie voranstehend erwähnt definiert sind. Alternativ lassen sich Verbindungen der allgemeinen Formel (1-7), in der **R¹** und **R²** wie voranstehend erwähnt definiert sind, aus Verbindungen der allgemeinen Formel (1-4), in der **R¹** und **R²** wie voranstehend erwähnt definiert sind, mit Di-*tert*-butyldicarbonat und einer Base, wie beispielsweise Diisopropylethylamin, in einem geeigneten Lösungsmittel herstellen. Die Verseifung des Esters (1-7) zu der entsprechenden Säure der allgemeinen Formel (1-8), in der **R¹** und **R²** wie voranstehend erwähnt definiert sind, kann wie oben beschrieben erfolgen. Besonders bevorzug ist die Verwendung von Alkalimetallhydroxiden wie Lithiumhydroxid in einer Mischung aus Wasser und Tetrahydrofuran.

Die Boc-Schutzgruppe der Säure mit der allgemeinen Formel (1-8) kann unter geeigneten Bedingungen wie sie z.B in T.W. Greene, P.G.M. Wuts, "Protective Groups in Organic Synthesis", Wiley, 1991 und 1999 beschrieben sind, abgespalten werden. Bevorzugt ist die Verwendung von Trifluoressigsäure in Dichlormethan bei Raumtemperatur und Normaldruck. Nach der Entschützung erhält man die Säure der allgemeinen Formel (1-5), in der **R¹** und **R²** wie voranstehend erwähnt definiert sind.

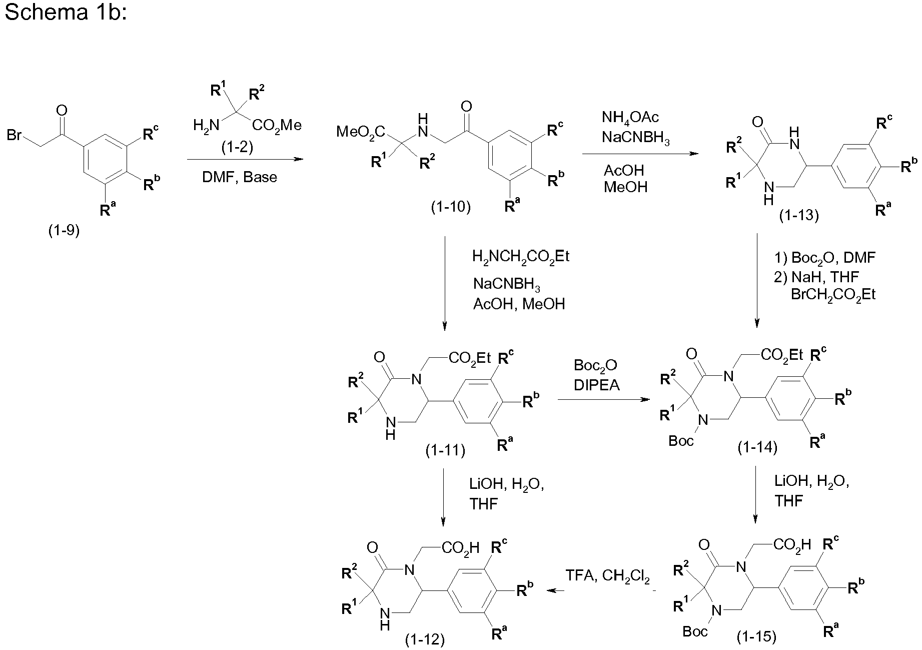

Wie in Schema 1 b gezeigt, lassen sich in analoger Weise zu Schema 1 a Verbindungen der allgemeinen Formel (1-12) synthetisieren, in der **R¹**, **R²**, **R^{a}**, **R^{b}** und **R^{c}** wie voranstehend erwähnt definiert sind:
Die Umsetzung von 2-Brom-1-(3,5-difluor-phenyl)-ethanon (1-9) mit einem α-Aminoester der allgemeinen Formel (1-2), in der **R¹** und **R²** wie voranstehend erwähnt definiert sind, liefert das Aminoketon der allgemeinen Formel (1-10), in der **R¹**, **R²**, **R^{a}**, **R^{b}** und **R^{c}** wie voranstehend erwähnt definiert sind. Für diese Umsetzung können verschiedene Basen verwendet werden, beispielsweise NaHCO₃, K₂CO₃ und Na₃PO₄. Eine reduktive Aminierung des Aminoketons (1-10) mit Glycinethylester liefert unter geeigneten Reaktionsbedingungen das Piperazinon der allgemeinen Formel (1-11), in der **R¹**, **R²**, **R^{a}**, **R^{b}** und **R^{c}** wie voranstehend erwähnt definiert sind. Ester der allgemeinen Formel (1-11), in der **R¹**, **R²**, **R^{a}**, **R^{b}** und **R^{c}** wie voranstehend erwähnt definiert sind, können durch basische oder saure Hydrolyse (J. March, Advanced Organic Chemistry (New York: J. Wiley and Sons, 1985) oder durch Umsetzen mit Alkalimetallsalzen (bevorzugt Lil oder NaCN) in einem inerten Lösungsmittel in die Säure der allgemeinen Formel (1-12) überführt werden, in der **R¹**, **R²**, **R^{a}**, **R^{b}** und **R^{c}** wie voranstehend erwähnt definiert sind. Inerte Lösungsmittel können Dialkylformamide (besonders bevorzugt ist N,N-Dimethylformamid), Dialkylacetamide (besonders bevorzugt ist N,N-Dimethylacetamid), cyclische Amide (besonders bevorzugt ist N-Methylpyrrolidon) sein. Besonders bevorzugt ist eine alkalische Verseifung mit Alkalimetallhydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

Alternativ kann das Aminoketon der allgemeinen Formel (1-10) in einer reduktiven Aminierung mit Ammoniumacetat in das entsprechende Diamin überführt werden, das unter geeigneten Versuchsbedingungen gleich zu dem Piperazinon der allgemeinen Formel (1-13), in der **R¹**, **R²**, **R^{a}**, **R^{b}** und **R^{c}** wie voranstehend erwähnt definiert sind, cyclisiert. Das Piperazinon der allgemeinen Formel (1-13) kann unter geeigneten Reaktionsbedingungen zunächst in das entsprechende *tert*-Butylcarbamat und anschließend mit Bromessigsäureethylester zu dem Ester der allgemeinen Formel (1-14) umgesetzt werden, in der **R¹**, **R²**, **R^{a}**, **R^{b}** und **R^{c}** wie voranstehend erwähnt definiert sind. Alternativ lassen sich Verbindungen der allgemeinen Formel (1-14), in der **R¹**, **R²**, **R^{a}**, **R^{b}** und **R^{c}** wie voranstehend erwähnt definiert sind, aus Verbindungen der allgemeinen Formel (1-11), in der **R¹**, **R²**, **R^{a}**, **R^{b}** und **R^{c}** wie voranstehend erwähnt definiert sind, mit Di-*tert*-butyldicarbonat und einer Base, wie beispielsweise Diisopropylethylamin, in einem geeigneten Lösungsmittel herstellen. Die Verseifung des Esters (1-14) zu der entsprechenden Säure der allgemeinen Formel (1-15), in der **R¹**, **R²**, **R^{a}**, **R^{b}** und **R^{c}** wie voranstehend erwähnt definiert sind, kann wie oben beschrieben erfolgen. Besonders bevorzug ist die Verwendung von Alkalimetallhydroxiden wie Lithiumhydroxid in einer Mischung aus Wasser und Tetrahydrofuran.

Die Boc-Schutzgruppe der Säure mit der allgemeinen Formel (1-15) kann unter geeigneten Bedingungen wie sie z.B in T.W. Greene, P.G.M. Wuts, "Protective Groups in Organic Synthesis", Wiley, 1991 und 1999 beschrieben sind, abgespalten werden. Bevorzugt ist die Verwendung von Trifluoressigsäure in Dichlormethan bei Raumtemperatur und Normaldruck. Nach der Entschützung erhält man die Säure der allgemeinen Formel (1-12), in der **R¹**, **R²**, **R^{a}**, **R^{b}** und **R^{c}** wie voranstehend erwähnt definiert sind.

Verbindungen der allgemeinen Formel (2-5), in der X, **Y, Z, R¹** und **R²** wie voranstehend erwähnt definiert sind, lassen sich wie in Schema 2a gezeigt herstellen.

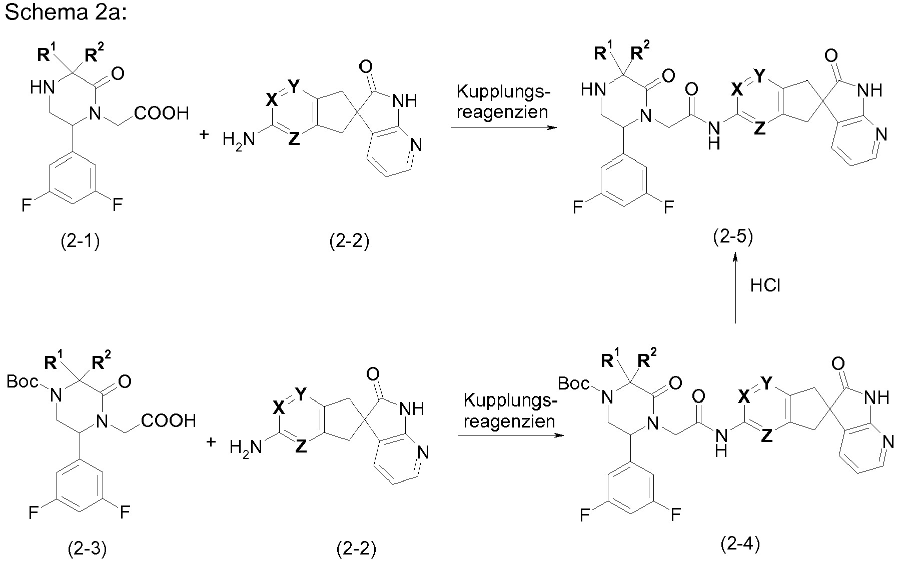

Carbonsäuren der allgemeinen Formel (2-1), in der **R¹** und **R²** wie voranstehend erwähnt definiert sind, können unter Zuhilfenahme von Standard-Peptid-Kupplungsreagenzien und einer Base in einem inerten Lösungsmittel mit Aminen der allgemeinen Formel (2-2), in der **X, Y und Z** wie voranstehend erwähnt definiert sind, zu Amiden der allgemeinen Formel (2-5), in der X, **Y, Z, R¹** und **R²** wie voranstehend erwähnt definiert sind, umgesetzt werden (siehe z. B. Houben-Weyl, Methoden der Organischen Chemie, Bd. 15/2). Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagenzien können beispielsweise 1 *H*-Benzotriazol-1-yl-oxy-tripyrrolidino-phosphonium-hexafluorophosphat (PyBOP), Dicyclohexylcarbodiimid (DCC), Diisopropylcarbodiimid (DIC), Ethyl-(3-dimethylaminopropyl)-carbodiimid (EDC), O-(1*H*-Benzotriazol-1-yl)-N,N-N,N-tetramethyl-uroniumhexafluorphosphat (HBTU) oder-tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von EDC. Ferner kann noch 1-Hydroxybenztriazol (HOBt) der Reaktionsmischung zugesetzt werden. Die Aktivierung der Carboxylgruppe kann auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Besonders bevorzugt ist die Verwendung von Diisopropylethylamin als Base und Dimethylformamid als Lösungsmittel. In analoger Weise können Verbindungen der allgemeinen Formel **(2-5),** in der **X, Y, Z, R¹** und **R²** wie voranstehend erwähnt definiert sind, aus den entsprechenden Carbonsäuren der allgemeinen Formel (2-3), in der **R¹** und **R²** wie voranstehend erwähnt definiert sind, und Aminen der allgemeinen Formel (2-2), in **der X, Y** und **Z** wie voranstehend erwähnt definiert sind, synthetisiert werden. Hierfür müssen die entsprechenden Amide der allgemeinen Formel (2-4), in **der X, Y, Z, R¹** und **R²** wie voranstehend erwähnt definiert sind, entsprechend entschützt werden. Die Entfernung der Boc-Schutzgruppe erfolgt nach literaturbekannten Methoden, wie beispielsweise mit HCl in einem geeigneten Lösungsmittel.

In einigen Fällen kann das Endprodukt weiter derivatisiert werden, z.B. durch Manipulation der Substituenten. Diese Manipulationen können unter anderem diejenigen sein, die dem Fachmann allgemein bekannt sind wie Oxidation, Reduktion, Alkylierung, Acylierung und Hydrolyse, müssen allerdings nicht auf diese beschränkt sein.

In analoger Weise zu Schema 2a lassen sich Verbindungen der allgemeinen Formel **(2-8),** in **der X, Y, Z, R¹, R²**, **R³**, **R^{a}**, **R^{b}** und **R^{c}** wie voranstehend erwähnt definiert sind, wie in Schema 2b gezeigt herstellen.

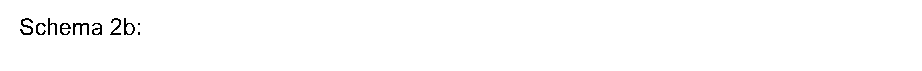

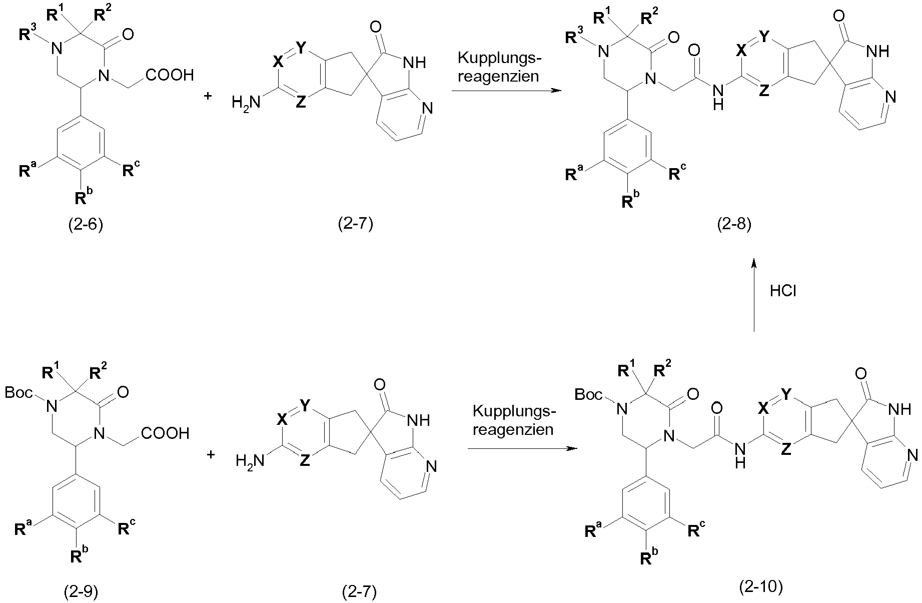

Carbonsäuren der allgemeinen Formel (2-6), in der **R¹**, **R²**, **R³**, **R^{a}**, **R^{b}** und **R^{c}** wie voranstehend erwähnt definiert sind, können unter Zuhilfenahme von Standard-Peptid-Kupplungsreagenzien und einer Base in einem inerten Lösungsmittel mit Aminen der allgemeinen Formel (2-7), in der **X, Y** und **Z** wie voranstehend erwähnt definiert sind, zu Amiden der allgemeinen Formel **(2-8),** in der **X**, **Y, Z, R¹**, **R²**, **R³**, **R^{a}**, **R^{b}** und **R^{c}** wie voranstehend erwähnt definiert sind, umgesetzt werden (siehe z. B. Houben-Weyl, Methoden der Organischen Chemie, Bd. 15/2). Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagenzien können beispielsweise 1*H*-Benzotriazol-1-yl-oxytripyrrolidino-phosphonium-hexafluorophosphat (PyBOP), Dicyclohexylcarbodiimid (DCC), Diisopropylcarbodiimid (DIC), Ethyl-(3-dimethylamino-propyl)-carbodiimid (EDC), O-(1*H-*Benzotriazol-1-yl)-N,N-N,N-tetramethyl-uroniumhexafluorphosphat (HBTU) oder -tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von EDC. Ferner kann noch 1-Hydroxybenztriazol (HOBt) der Reaktionsmischung zugesetzt werden. Die Aktivierung der Carboxylgruppe kann auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Besonders bevorzugt ist die Verwendung von Diisopropylethylamin als Base und Dimethylformamid als Lösungsmittel. In analoger Weise können Verbindungen der allgemeinen Formel **(2-8),** in der **X, Y, Z**, **R¹**, **R²**, **R^{a}**, **R^{b}** und **R^{c}** wie voranstehend erwähnt definiert sind und **R³** ein Wasserstoff-Atom darstellt, aus den entsprechenden Carbonsäuren der allgemeinen Formel (2-9), in der **R¹**, **R²**, **R^{a}**, **R^{b}** und **R^{c}** wie voranstehend erwähnt definiert sind, und Aminen der allgemeinen Formel (2-7), in der **X, Y** und **Z** wie voranstehend erwähnt definiert sind, synthetisiert werden. Hierfür müssen die entsprechenden Amide der allgemeinen Formel (2-10), in der **X, Y,** Z, **R¹**, **R²**, **R^{a}**, **R^{b}** und **R^{c}** wie voranstehend erwähnt definiert sind, entsprechend entschützt werden. Die Entfernung der Boc-Schutzgruppe erfolgt nach literaturbekannten Methoden, wie beispielsweise mit HCl in einem geeigneten Lösungsmittel.

Die Synthese der als Intermediate verwendeten α-Aminoester erfolgt nach Methoden, wie sie dem Fachmann bekannt sind oder in der Literatur beschrieben sind. Exemplarisch ist die Synthese von α-Aminoestern in Schema 3 und Schema 4 beschrieben. Die dort beschriebenen Verfahren dienen nur der Anschaulichkeit und sind nicht auf diese beschränkt.

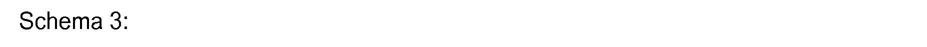

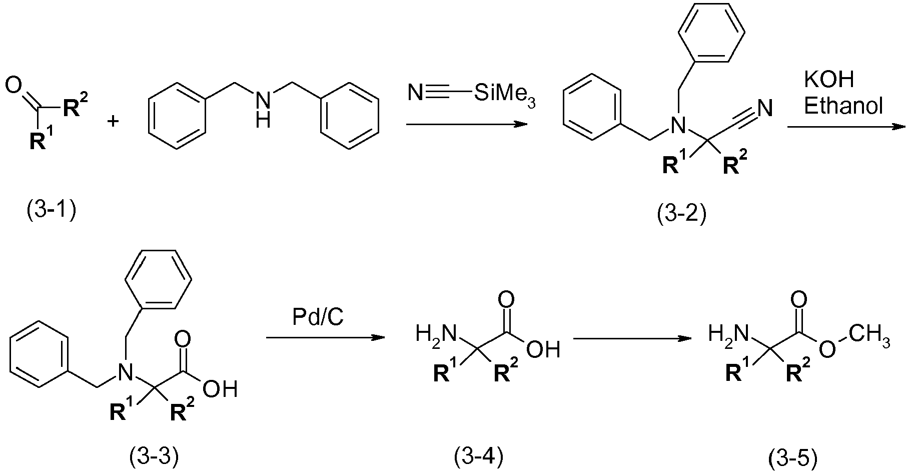

Ketone der allgemeinen Formel (3-1), in der **R¹** und **R²** wie voranstehend erwähnt definiert sind, können mit Dibenzylamin und Trimethylsilylcyanid in einem geeigneten Lösungsmittel wie Essigsäure zu den entsprechenden Nitrilen der allgemeinen Formel (3-2), in der **R¹** und **R²** wie voranstehend erwähnt definiert sind, umgesetzt werden. Nitrile der allgemeinen Formel (3-2) lassen sich mit Natriumhydroxid in einem geeigneten Lösungsmittel wie beispielsweise in Ethanol zu der entsprechenden Säure der allgemeinen Formel (3-3), in der **R¹** und **R²** wie voranstehend erwähnt definiert sind, umsetzen. Die Abspaltung der Benzylschutzgruppen erfolgt beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur, und bei einem Wasserstoffdruck von 1 bis 7 bar, vorzugsweise jedoch von 1 bis 5 bar. Nach der Abspaltung der Benzylschutzgruppe erhält man eine Aminosäure der allgemeinen Formel (3-4), in der **R¹** und **R²** wie voranstehend erwähnt definiert sind. Die Aminosäure der allgemeinen Formel (3-4) lässt sich unter allgemein bekannten Methoden und wie bespielsweise in T.W. Greene, P.G.M. Wuts, "Protective Groups in Organic Synthesis", Wiley, 1991 und 1999 beschrieben in den entsprechenden α-Aminoester der allgemeinen Formel (3-5), in der **R¹** und **R²** wie voranstehend erwähnt definiert sind, überführen.

Wie in Schema 4 gezeigt, lassen sich α-Aminoester der allgemeinen Formel (4-4), in der **R¹** wie voranstehend erwähnt definiert und **R²** bevorzugt ein Wasserstoffatom darstellt, auch nach der Schöllkopf-Hartwig-Methode synthetisieren.

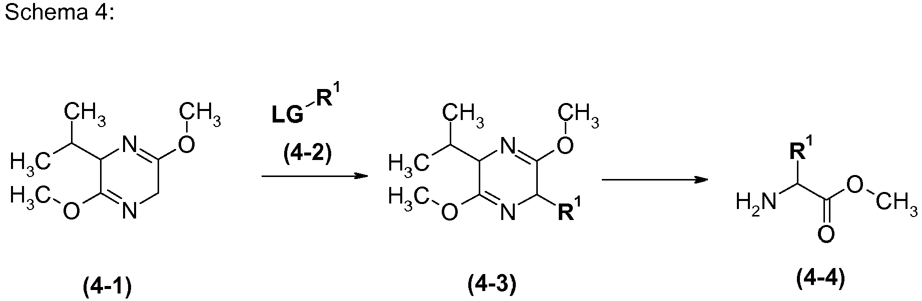

Ein Bislactimether der allgemeinen Formel (4-1) kann mit einer Verbindung der allgemeinen Formel (4-2), in der **R¹** wie voranstehend erwähnt definiert ist, umgesetzt werden. Als Austrittsgruppe **LG** können Bromide und lodide, Trifluormethansulfonate, Methansulfonate und Toluolsulfonate fungieren, müssen aber nicht auf diese beschränkt sein. Ganz besonders bevorzugt sind Iodide als Austrittsgruppe **LG.** Der Bislactimether der allgemeinen Formel (4-3), in der **R¹** wie voranstehend erwähnt definiert ist, kann unter geeigneten Reaktionsbedingungen in geeigneten Lösungsmitteln mit Säure, bevorzugt ist hierbei wässrige Salzsäure in Acetonitril in den α-Aminoester der allgemeinen Formel (4-4) überführt werden.

Die Synthese der Verbindung (5-6) ist in der internationalen Publikation WO 2008/020902 (Intermediat 41) beschrieben und hier in Schema 5 gezeigt:

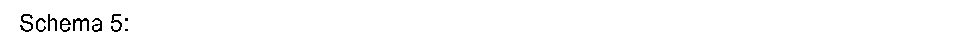

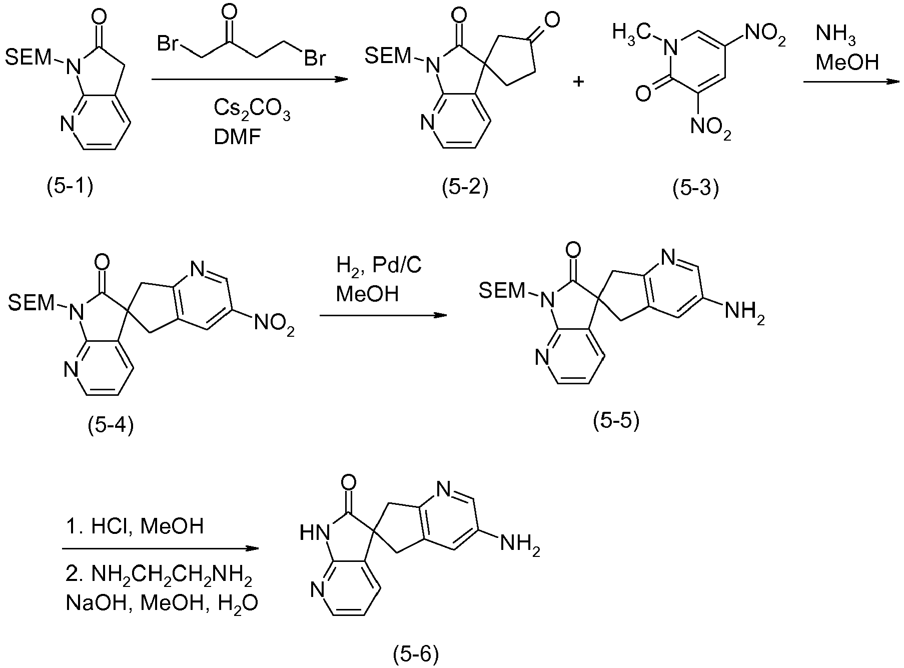

Die Synthese der Verbindung (6-5) ist in der internationalen Publikation WO 2008/020902 (Intermediat 42) beschrieben und hier in Schema 6 gezeigt:

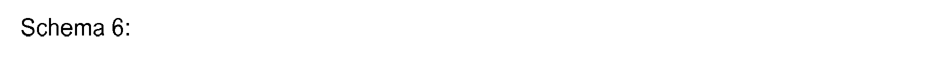

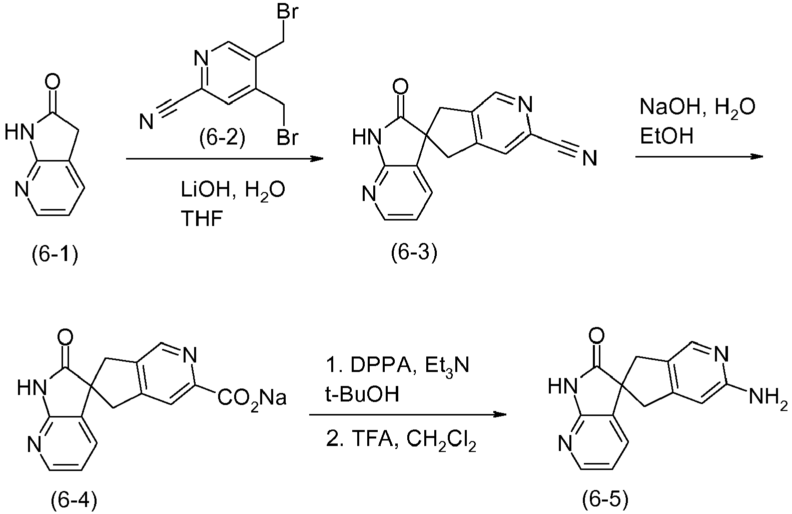

Die Synthese der Verbindung (7-5) ist in der internationalen Publikation WO 2008/020902 (Intermediat 43) beschrieben und hier in Schema 7 gezeigt:

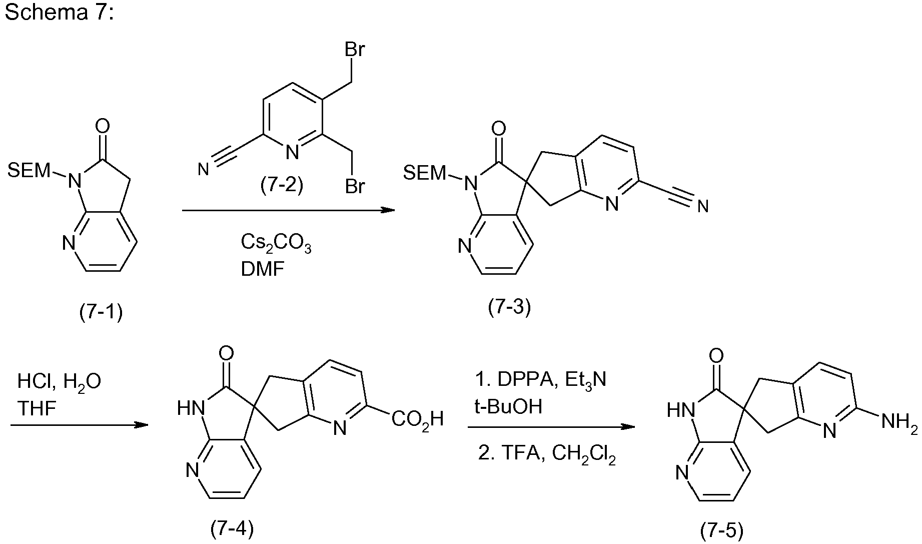

Verbindungen der allgemeinen Formel (8-3), in der **m**¹ eine der Ziffern 1 oder 2, **m**² eine der Ziffern 1 oder 2, **n**³ eine der Ziffern 0 oder 1 und **n²** eine der Ziffern 1 oder 2 bedeutet, lassen sich wie in Schema 8 gezeigt herstellen. Die Synthese erfolgt analog zu US 2005/0261501.

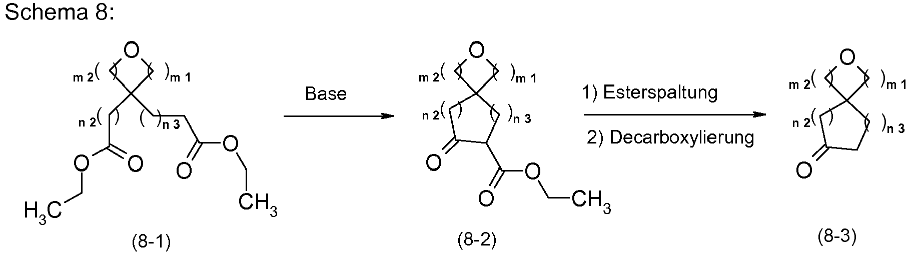

Eine Verbindung der allgemeinen Formel (8-1), in der **m**¹ eine der Ziffern 1 oder 2, **m²** eine der Ziffern 1 oder 2, **n³** eine der Ziffern 0 oder 1 und **n²** eine der Ziffern 1 oder 2 bedeutet, kann mit einer Base, wie beispielsweise Natriumhydrid oder Natriumethanolat, und in einem geeigneten Lösungsmittel, wie beispielsweise Tetrahydrofuran, deprotoniert und anschließend zu einer Verbindung der allgemeinen Formel (8-2), in der **m**¹ eine der Ziffern 1 oder 2, **m**² eine der Ziffern 1 oder 2, **n³** eine der Ziffern 0 oder 1 und **n²** eine der Ziffern 1 oder 2 bedeutet, cyclisiert werden. Die Estergruppe kann zunächst unter geeigneten Bedingungen, beispielsweise mit HCl in Ethanol, verseift und anschließend decarboxyliert werden, um die Verbindung der allgemeinen Formel (8-3), in der **m¹** eine der Ziffern 1 oder 2, **m²** eine der Ziffern 1 oder 2, **n³** eine der Ziffern 0 oder 1 und **n²** eine der Ziffern 1 oder 2 bedeutet, zu erhalten.

Verbindungen der allgemeinen Formel (9-3), in der **m¹** eine der Ziffern 1 oder 2 und **m²** eine der Ziffern 1 oder 2 bedeutet, lassen sich wie in Schema 9 gezeigt herstellen. Die Synthese erfolgt analog zu US 2005/0261501.

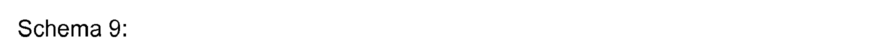

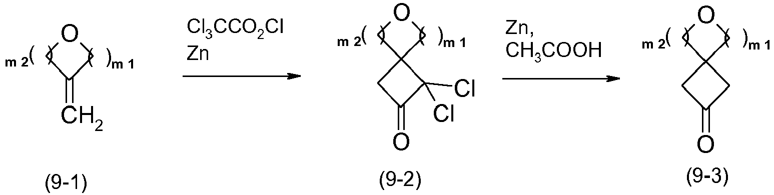

Verbindungen der allgemeinen Formel (9-1), in der **m¹** eine der Ziffern 1 oder 2 und **m²** eine der Ziffern 1 oder 2 bedeutet, lassen sich mit Zink und Trichloressigsäurechlorid in einem geeigneten Lösungsmittel, wie beispielweise in Diethylether, umsetzen. Gegebenenfalls wird die Reaktion unter Ultraschall durchgeführt. Verbindungen der allgemeinen Formel (9-2), in der **m¹** eine der Ziffern 1 oder 2 und **m²** eine der Ziffern 1 oder 2 bedeutet, lassen sich gegebenenfalls in einem geeigneten Lösungsmittel, wie beispielsweise Wasser mit Essigsäure und Zinkpulver, in Verbindungen der allgemeinen Formel (9-3), in der **m¹** eine der Ziffern 1 oder 2 und **m²** eine der Ziffern 1 oder 2 bedeutet, überführen.

Verbindungen der allgemeinen Formel (10-3), in der **m¹** eine der Ziffern 1 oder 2 und **m²** eine der Ziffern 1 oder 2 bedeutet, lassen sich wie in Schema 10 gezeigt herstellen. Die Synthese erfolgt analog zu US 2005/0261501.

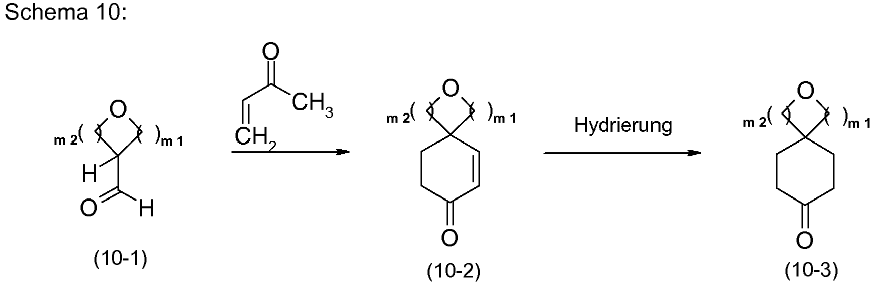

Verbindungen der allgemeinen Formel (10-1), in der der **m¹** eine der Ziffern 1 oder 2 und **m²** eine der Ziffern 1 oder 2 bedeutet, werden in einem geeigneten Lösungsmittel mit Methylvinylketon in einer Michael-Reaktion und einer anschließenden intramolekularen Aldol-Reaktion zu dem Cyclohexenon-Derivat mit der allgemeinen Formel (10-2), in der der **m¹** eine der Ziffern 1 oder 2 und **m²** eine der Ziffern 1 oder 2 bedeutet, umgesetzt. Die Reaktionen können basenkatalysiert, beispielsweise mit ethanolischer Kaliumhydroxid Lösung, bzw. säurekatalysiert, beispielsweise mit Schwefelsäure, sein. Die Cyclohexenone der allgemeinen Formel (10-2), in der der **m¹** eine der Ziffern 1 oder 2 und **m²** eine der Ziffern 1 oder 2 bedeutet, können zu den entsprechenden Cyclohexanon-Derivaten der allgemeinen Formel (10-3), in der der **m¹** eine der Ziffern 1 oder 2 und **m²** eine der Ziffern 1 oder 2 bedeutet, hydriert werden. Die Hydrierung kann beispielsweise in einer Wasserstoffatmosphäre mit einem Katalysator, wie beispielsweise Palladium auf Kohle, und in einem geeigneten Lösungsmittel durchgeführt werden.

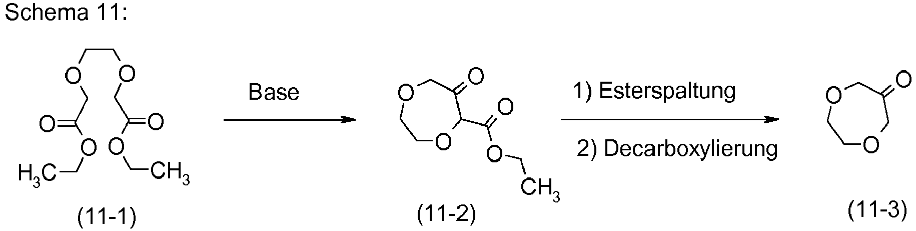

Eine Verbindung der Formel (11-1) kann mit einer Base, wie beispielsweise Natrium-*tert-*Butanolat, und in einem geeigneten Lösungsmittel, wie beispielsweise Tetrahydrofuran, deprotoniert und anschließend zu einer Verbindung der Formel (11-2) cyclisiert werden. Die Estergruppe kann zunächst unter geeigneten Bedingungen, beispielsweise mit HCl in Ethanol, verseift und anschließend decarboxyliert werden, um die Verbindung der Formel (11-3) zu erhalten.

Eine allgemeine Synthese für Piperazinone der allgemeinen Formel (12-7), in der **R¹** und **R²** wie voranstehend erwähnt definiert sind, ist in Schema 12a dargestellt.

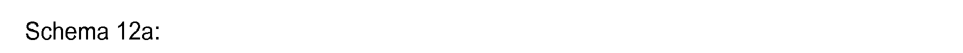

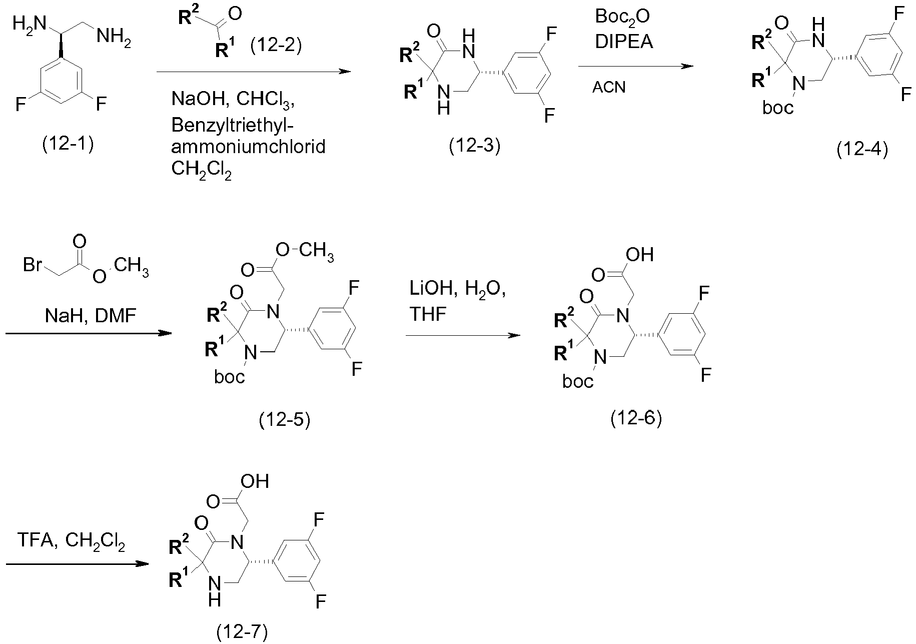

Die Umsetzung von 1-(3,5-Difluor-phenyl)-ethan-1,2-diamin (12-1) mit einem Keton der allgemeinen Formel (12-2), in der **R¹** und **R²** wie voranstehend erwähnt definiert sind, in einem geeigneten Lösungsmittel wie beispielsweise Dichlormethan und in Gegenwart von Chloroform, Benzyltriethylammoniumchlorid und einer Base wie beispielsweise Natronlauge liefert das Piperazinon der allgemeinen Formel (12-3), in der **R¹** und **R²** wie voranstehend erwähnt definiert sind. Das Piperazinon der allgemeinen Formel (12-3) kann unter geeigneten Reaktionsbedingungen zunächst in das entsprechende *tert*-Butylcarbamat der allgemeinen Formel (12-4), in der **R¹** und **R²** wie voranstehend erwähnt definiert sind, und anschließend mit Bromessigsäuremethylester zu dem Ester der allgemeinen Formel (12-5) umgesetzt werden, in der **R¹** und **R²** wie voranstehend erwähnt definiert sind. Die Verseifung des Esters (12-5) zu der entsprechenden Säure der allgemeinen Formel (12-6), in der **R¹** und **R²** wie voranstehend erwähnt definiert sind, kann durch basische oder saure Hydrolyse (J. March, Advanced Organic Chemistry (New York: J. Wiley and Sons, 1985) in einem inerten Lösungsmittel erfolgen. Besonders bevorzug ist die Verwendung von Alkalimetallhydroxiden wie Lithiumhydroxid in einer Mischung aus Wasser und Tetrahydrofuran.

Die Boc-Schutzgruppe der Säure mit der allgemeinen Formel (12-6) kann unter geeigneten Bedingungen wie sie z.B in T.W. Greene, P.G.M. Wuts, "Protective Groups in Organic Synthesis", Wiley, 1991 und 1999 beschrieben sind, abgespalten werden. Bevorzugt ist die Verwendung von Trifluoressigsäure in Dichlormethan bei Raumtemperatur und Normaldruck. Nach der Entschützung erhält man die Säure der allgemeinen Formel (12-7), in der **R¹** und **R²** wie voranstehend erwähnt definiert sind.

Wie in Schema 12b gezeigt, lassen sich in analoger Weise zu Schema 12a Piperazinone der allgemeinen Formel (12-13), in der **R¹**, **R², R^{a}, R^{b}** und **R^{c}** wie voranstehend erwähnt definiert sind, herstellen.

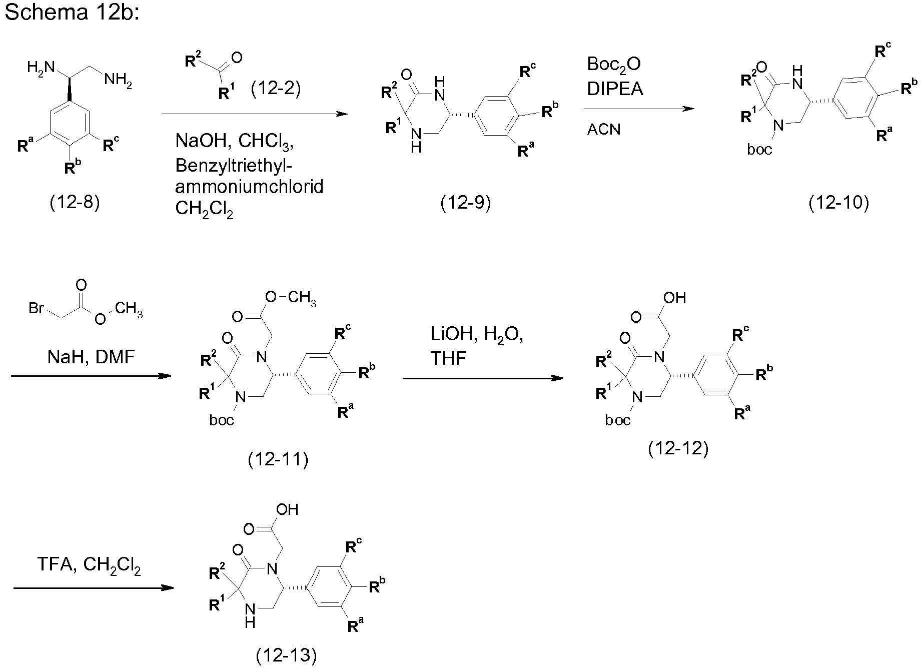

Die Umsetzung einer Verbindung der allgemeinen Formel (12-8), in der **R^{a}, R^{b}** und **R^{c}** wie voranstehend erwähnt definiert sind, mit einem Keton der allgemeinen Formel (12-2), in der **R¹** und **R²** wie voranstehend erwähnt definiert sind, in einem geeigneten Lösungsmittel wie beispielsweise Dichlormethan und in Gegenwart von Chloroform, Benzyltriethylammoniumchlorid und einer Base, wie beispielsweise Natronlauge, liefert ein Piperazinon der allgemeinen Formel (12-9), in der **R¹, R², R^{a}, R^{b}** und **R^{c}** wie voranstehend erwähnt definiert sind. Das Piperazinon der allgemeinen Formel (12-9) kann unter geeigneten Reaktionsbedingungen zunächst in ein entsprechendes *tert*-Butylcarbamat der allgemeinen Formel (12-10), in der **R¹**, **R², R^{a}, R^{b}** und **R^{c}** wie voranstehend erwähnt definiert sind, und anschließend mit Bromessigsäuremethylester zu dem Ester der allgemeinen Formel (12-11) umgesetzt werden, in der **R¹**, **R², R^{a}, R^{b}** und **R^{c}** wie voranstehend erwähnt definiert sind. Die Verseifung des Esters (12-11) zu einer entsprechenden Säure der allgemeinen Formel (12-12), in der **R¹**, **R², R^{a}, R^{b}** und **R^{c}** wie voranstehend erwähnt definiert sind, kann durch basische oder saure Hydrolyse (J. March, Advanced Organic Chemistry (New York: J. Wiley and Sons, 1985) in einem inerten Lösungsmittel erfolgen. Besonders bevorzug ist die Verwendung von Alkalimetallhydroxiden wie Lithiumhydroxid in einer Mischung aus Wasser und Tetrahydrofuran.
Die Boc-Schutzgruppe einer Säure mit der allgemeinen Formel (12-12), in der **R¹**, **R², R^{a}, R^{b}** und **R^{c}** wie voranstehend erwähnt definiert sind, kann unter geeigneten Bedingungen wie sie z.B in T.W. Greene, P.G.M. Wuts, "Protective Groups in Organic Synthesis", Wiley, 1991 und 1999 beschrieben sind, abgespalten werden. Bevorzugt ist die Verwendung von Trifluoressigsäure in Dichlormethan bei Raumtemperatur und Normaldruck. Nach der Entschützung erhält man die Säure der allgemeinen Formel (12-13), in der **R¹**, **R², R^{a}, R^{b}** und **R^{c}** wie voranstehend erwähnt definiert sind.

Eine allgemeine Synthese für Piperazinone der allgemeinen Formel (13-8), in der **R¹** und **R²** wie voranstehend erwähnt definiert sind, ist in Schema 13a dargestellt.

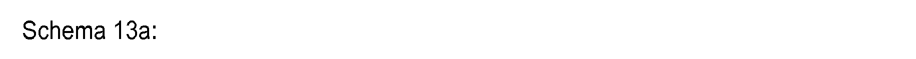

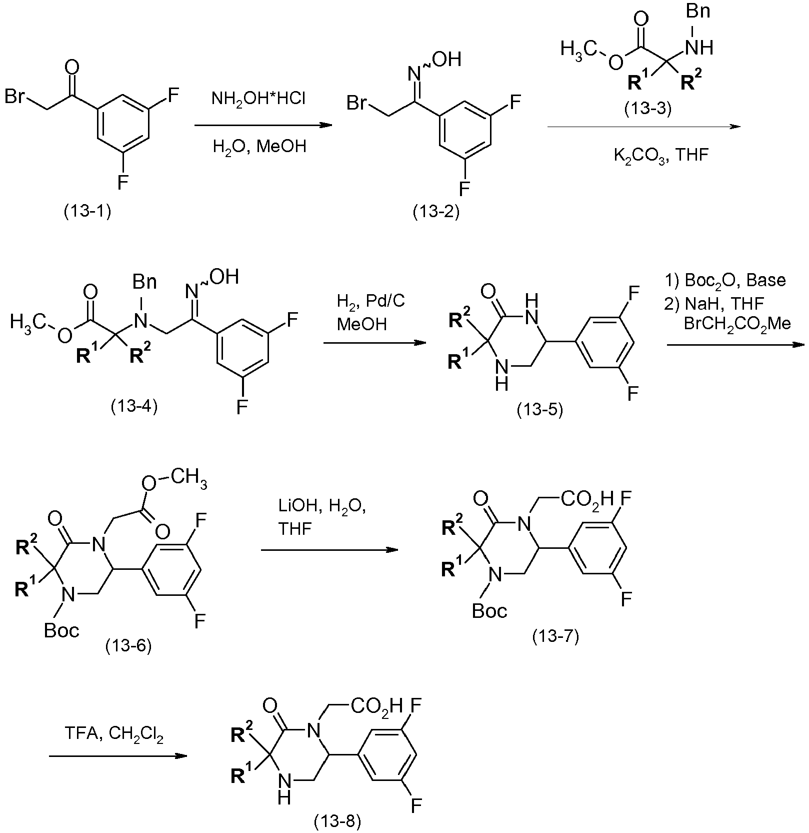

Die Umsetzung von 2-Brom-1-(3,5-difluor-phenyl)-ethanon (13-1) mit Hydroxylamin Hydrochlorid liefert das Oxim der allgemeinen Formel (13-2). Die Umsetzung von 2-Brom-1-(3,5-difluor-phenyl)-ethanonoxim mit einem α-Aminoester der allgemeinen Formel (13-3), in der **R¹** und **R²** wie voranstehend erwähnt definiert sind, liefert das Oxim der allgemeinen Formel (13-4), in der **R¹** und **R**² wie voranstehend erwähnt definiert sind. Für diese Umsetzung können verschiedene Basen in inerten Lösungsmitteln verwendet werden, beispielsweise NaHCO₃, K₂CO₃ und Na₃PO₄. Als Lösungsmittel eignet sich beispielsweise THF. Unter geeigneten Reaktionsbedingungen liefert die Hydrierung des Oxims der allgemeinen Formel (13-4), in der **R¹** und **R²** wie voranstehend erwähnt definiert sind, in Gegenwart eines Katalysators das Piperazinon der allgemeinen Formel (13-5), in der **R¹** und **R²** wie voranstehend erwähnt definiert sind. Bevorzugt sind Hydrierungen mit Palladium auf Kohle als Katalysator bei erhöhtem Wasserstoff-Druck und erhöhten Temperaturen. Die Einführung einer geeigneten Schutzgruppe an das Piperazinon der allgemeinen Formel (13-5), in der **R¹** und **R²** wie voranstehend erwähnt definiert sind, kann analog zu beschriebenen Methoden durchgeführt werden (T.W. Greene, P.G.M. Wuts, "Protective Groups in Organic Synthesis", Wiley, 1991 und 1999). Besonders bevorzugt ist die Einführung einer Boc-Schutzgruppe mittels Boc-Anhydrid in einem geeigneten Lösungsmittel in Gegenwart einer Base. Das so erhaltene Piperazinon kann unter geeigneten Reaktionsbedingungen mit einem Bromessigsäureester-Derivat, ganz besonders bevorzugt ist Bromessigsäuremethylester zu einem Ester der allgemeinen Formel (13-6) umgesetzt werden, in der **R¹** und **R²** wie voranstehend erwähnt definiert sind. Die Verseifung des Esters der allgemeinen Formel (13-6), in der **R¹** und **R²** wie voranstehend erwähnt definiert sind, zu der entsprechenden Säure der allgemeinen Formel (13-7), in der **R¹** und **R²** wie voranstehend erwähnt definiert sind, kann wie in T.W. Greene, P.G.M. Wuts, "Protective Groups in Organic Synthesis", Wiley, 1991 und 1999 beschrieben, erfolgen. Besonders bevorzugt ist die Verwendung von Alkalimetall-Hydroxiden wie Lithiumhydroxid in einer Mischung aus Wasser und Tetrahydrofuran.
Die Entschützung des Piperazinons der allgemeinen Formel (13-7), in der **R¹** und **R²** wie voranstehend erwähnt definiert sind, kann unter geeigneten Bedingungen wie sie z.B in T.W. Greene, P.G.M. Wuts, "Protective Groups in Organic Synthesis", Wiley, 1991 und 1999 beschrieben sind, durchgeführt werden. Die Abspaltung der Boc-Schutzgruppe kann beispielsweise mit Trifluoressigsäure in Dichlormethan oder methanolischer Salzsäure erfolgen. Nach der Abspaltung der Schutzgruppe erhält man die Säure der allgemeinen Formel (13-8), in der **R¹** und **R²** wie voranstehend erwähnt definiert sind.

Eine allgemeine Synthese für Piperazinone der allgemeinen Formel (13-16), in der **R¹**, **R², R^{a}, R^{b}** und **R^{c}** wie voranstehend erwähnt definiert sind, ist in Schema 13b dargestellt.

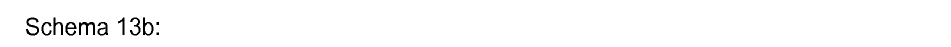

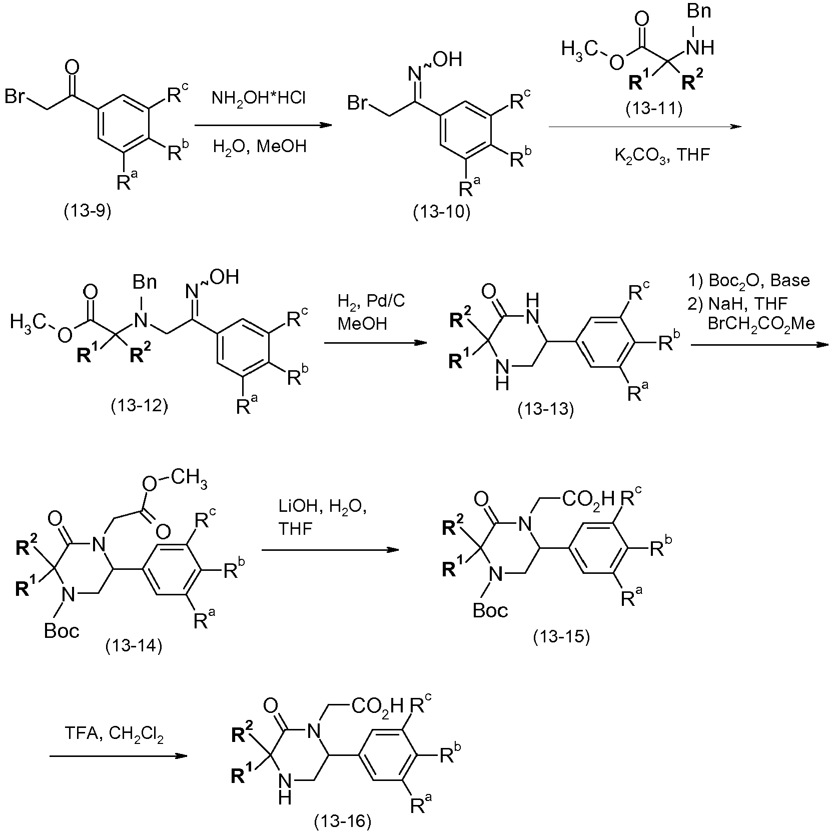

Die Umsetzung eines Bromketons der allgemeinen Formel (13-9), in der **R^{a}, R^{b}** und **R^{c}** wie voranstehend erwähnt definiert sind, mit Hydroxylamin Hydrochlorid liefert das Oxim der allgemeinen Formel (13-10), in der **R^{a}, R^{b}** und **R^{c}** wie voranstehend erwähnt definiert sind. Die Umsetzung des Oxims der allgemeinen Formel (13-10), in der **R^{a}, R^{b}** und **R^{c}** wie voranstehend erwähnt definiert sind, mit einem α-Aminoester der allgemeinen Formel (13-11), in der **R¹** und **R**² wie voranstehend erwähnt definiert sind, liefert das Oxim der allgemeinen Formel (13-12), in der **R¹**, **R², R^{a}, R^{b}** und **R^{c}** wie voranstehend erwähnt definiert sind. Für diese Umsetzung können verschiedene Basen in inerten Lösungsmitteln verwendet werden, beispielsweise NaHCO₃, K₂CO₃ und Na₃PO₄. Als Lösungsmittel eignet sich beispielsweise THF. Unter geeigneten Reaktionsbedingungen liefert die Hydrierung des Oxims der allgemeinen Formel (13-12), in der **R¹**, **R², R^{a}, R^{b}** und **R^{c}** wie voranstehend erwähnt definiert sind, in Gegenwart eines Katalysators das Piperazinon der allgemeinen Formel (13-13), in der **R¹**, **R², R^{a}, R^{b}** und **R^{c}** wie voranstehend erwähnt definiert sind. Bevorzugt sind Hydrierungen mit Palladium auf Kohle als Katalysator bei erhöhtem Wasserstoff-Druck und erhöhten Temperaturen. Die Einführung einer geeigneten Schutzgruppe an das Piperazinon der allgemeinen Formel (13-13), in der **R¹**, **R², R^{a}, R^{b}** und **R^{c}** wie voranstehend erwähnt definiert sind, kann analog zu beschriebenen Methoden durchgeführt werden (T.W. Greene, P.G.M. Wuts, "Protective Groups in Organic Synthesis", Wiley, 1991 und 1999). Besonders bevorzugt ist die Einführung einer Boc-Schutzgruppe mittels Boc-Anhydrid in einem geeigneten Lösungsmittel in Gegenwart einer Base. Das so erhaltene Piperazinon kann unter geeigneten Reaktionsbedingungen mit einem Bromessigsäureester-Derivat, ganz besonders bevorzugt ist Bromessigsäuremethylester zu einem Ester der allgemeinen Formel (13-14) umgesetzt werden, in der **R¹**, **R², R^{a}, R^{b}** und **R^{c}** wie voranstehend erwähnt definiert sind. Die Verseifung des Esters der allgemeinen Formel (13-14), in der **R¹**, **R², R^{a}, R^{b}** und **R^{c}** wie voranstehend erwähnt definiert sind, zu der entsprechenden Säure der allgemeinen Formel (13-15), in der **R¹**, **R², R^{a}, R^{b}** und **R^{c}** wie voranstehend erwähnt definiert sind, kann wie in T.W. Greene, P.G.M. Wuts, "Protective Groups in Organic Synthesis", Wiley, 1991 und 1999 beschrieben, erfolgen. Besonders bevorzugt ist die Verwendung von Alkalimetall-Hydroxiden wie Lithiumhydroxid in einer Mischung aus Wasser und Tetrahydrofuran.
Die Entschützung des Piperazinons der allgemeinen Formel (13-15), in der **R¹**, **R², R^{a}, R^{b}** und **R^{c}** wie voranstehend erwähnt definiert sind, kann unter geeigneten Bedingungen wie sie z.B in T.W. Greene, P.G.M. Wuts, "Protective Groups in Organic Synthesis", Wiley, 1991 und 1999 beschrieben sind, durchgeführt werden. Die Abspaltung der Boc-Schutzgruppe kann beispielsweise mit Trifluoressigsäure in Dichlormethan oder methanolischer Salzsäure erfolgen. Nach der Abspaltung der Schutzgruppe erhält man die Säure der allgemeinen Formel (13-16), in der **R¹**, **R², R^{a}, R^{b}** und **R^{c}** wie voranstehend erwähnt definiert sind.

Verbindungen der allgemeinen Formel (14-5), in der **R¹**, **R², R^{a}, R^{b}** und **R^{c}** wie voranstehend erwähnt definiert sind, lassen sich wie in Schema 14 gezeigt herstellen.

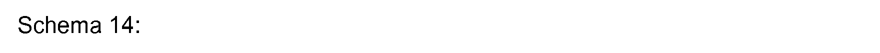

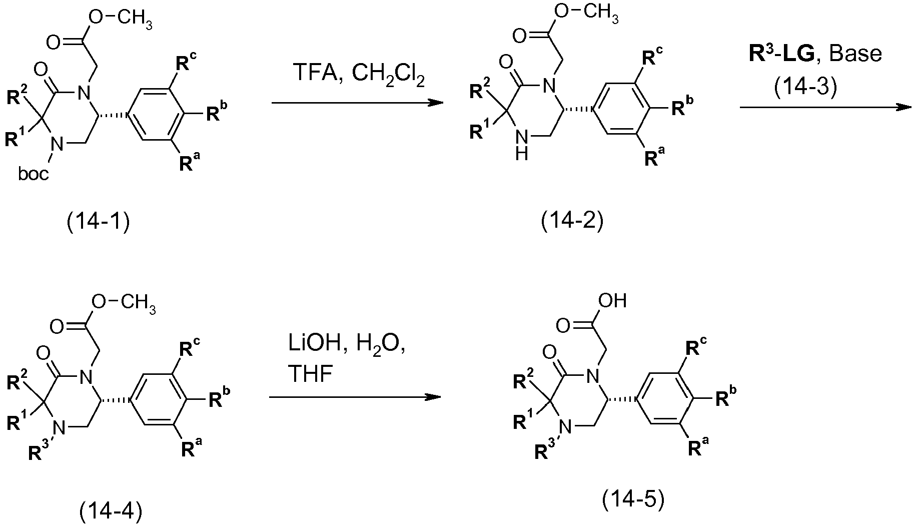

Die Boc-Schutzgruppe der Verbindung mit der allgemeinen Formel (14-1), in der **R¹**, **R², R^{a}, R^{b}** und **R^{c}** wie voranstehend erwähnt definiert sind, kann unter geeigneten Bedingungen wie sie z.B in T.W. Greene, P.G.M. Wuts, "Protective Groups in Organic Synthesis", Wiley, 1991 und 1999 beschrieben sind, abgespalten werden.
Verbindungen der allgemeinen Formel (14-2), in der **R¹**, **R², R^{a}, R^{b}** und **R^{c}** wie voranstehend erwähnt definiert sind, können mit Verbindungen der allgemeinen Formel (14-3), in der **R³** wie voranstehend erwähnt definiert sind und **LG** eine Austrittsgruppe darstellt, in einem geeigneten Lösungsmittel und gegebenenfalls mit Hilfe einer Base umgesetzt werden. Als Austrittsgruppe **LG** können Bromide und lodide, Trifluormethansulfonate, Methansulfonate und Toluolsulfonate fungieren, müssen aber nicht auf diese beschränkt sein. Ganz besonders bevorzugt sind lodide als Austrittsgruppe **LG.** Ganz besonders bevorzugt ist Methyl als **R³.** Als Lösungsmittel eignet sich Acetonitril und als Base Kaliumcarbonat. Die Verseifung des Esters der allgemeinen Formel (14-4), in der **R¹**, **R², R³, R^{a}, R^{b}** und **R^{c}** wie voranstehend erwähnt definiert sind, zu der entsprechenden Säure der allgemeinen Formel (14-5), in der **R¹**, **R², R³, R^{a}, R^{b}** und **R^{c}** wie voranstehend erwähnt definiert sind, kann durch basische oder saure Hydrolyse (J. March, Advanced Organic Chemistry (New York: J. Wiley and Sons, 1985) in einem inerten Lösungsmittel erfolgen. Besonders bevorzug ist die Verwendung von Alkalimetallhydroxiden wie Lithiumhydroxid in einer Mischung aus Wasser und Tetrahydrofuran.

Die erfindungsgemäßen neuen Verbindungen der allgemeinen Formel I und deren Intermediate können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel I fallenden Racematen sowie deren Inter-mediate gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (R)-(+)-I-Phenyl-ethylamin, (S)-(-)-I-Phenylethylamin oder (S)-Brucin, entstehen.

Nach einem üblichen Verfahren zur Isomerentrennung wird das Racemat einer Verbindung der allgemeinen Formel **I** sowie deren Intermediate mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten *(R)-*bzw. (*S*)-konfigurierten Reaktionskomponente durchführt.

Die absolute Stereochemie kann durch Röntgenkristallographie von kristallinen Produkten oder Intermediaten bestimmt werden. Gegebenenfalls werden diese Verbindungen mit geeigneten Reagenzien umgesetzt, die ein asymmetrisches Zentrum mit bekannter Konfiguration haben.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC Membranen (∼ 20 µg Proteinmenge) werden für 180 Minuten bei Raumtemperatur mit 50 pM ¹²⁵I-Iodotyrosyl-Calcitonin-Gene-Related Peptide und ansteigenden Konzentrationen der Testsubstanzen in einem Gesamtvolumen von 250 µl inkubiert (Assay Puffer: 10 mM Tris, 50 mM NaCl, 5 mM MgCl₂, 1 mM EDTA, pH=7.4). Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM BIBN4096BS während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test Kᵢ-Werte ≤ 50 µM.

### B. CGRP-Antagonismus in SK-N-MC-Zellen

SK-N-MC Zellen (-1000 Zellen pro well) werden in Anwesenheit von steigenden Konzentrationen von CGRP und verschiedenen Konzentrationen der Testsubstanz für 30 Minuten inkubiert.

Die cAMP-Gehalte der Proben werden mittels AlphaScreen cAMP assay kit (Perkin Elmer) bestimmt und die pA₂-Werte antagonistisch wirkender Substanzen graphisch ermittelt.

Um zu zeigen, dass die Verbindungen der allgemeinen Formel **I** mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden.

| **Nr.** | **CGRP Bindung Kᵢ [nM]** |
|---|---|
| (1a) | 0.03 |
| (1b) | 1.77 |
| (2) | 1.02 |
| (3) | 0.72 |
| (13) | 0.72 (Diastereomer 1) |
| | 0.71 (Diastereomer 2) |
| (14) | 0.07 |
| (15a) | 0.10 |
| (15b) | 0.39 |
| (16a) | 5.40 |
| (16b) | 0.13 |
| (17) | 0.03 |
| (18) | 0.84 |
| (19) | 1.41 |
| (20) | 0.96 |
| (21) | 0.44 |
| (22) | 0.11 |
| (23) | 0.05 |
| (24) | 0.07 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Ein weiterer Gegenstand der vorliegenden Erfindung betrifft daher die Verwendung der Verbindungen der voranstehend genannten allgemeinen Formel **I** zur Behandlung der voranstehend genannten Krankheiten (Indikationen).

Ein weiterer Gegenstand der vorliegenden Erfindung betrifft die Verwendung der Verbindungen der voranstehend genannten allgemeinen Formel **I** zur Herstellung eines Arzneimittels zur Behandlung der voranstehend genannten Krankheiten (Indikationen).

Unter einer "Behandlung" soll erfindungsgemäß eine akute, präventive, palliative oder curative Behandlung verstanden sein.

Ein weiterer Gegenstand der vorliegenden Erfindung betrifft ferner die Verwendung der voranstehend genannten allgemeinen Formel **I** zur vorbeugenden Behandlung der voranstehend genannten Krankheiten (Indikationen).

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Ein weiterer Gegenstand der vorliegenden Erfindung betrifft ein Verfahren zur Behandlung einer der voranstehend genannten Krankheiten (Indikationen) an einem Lebewesen, vorzugsweise am Menschen, der einer solchen Behandlung bedarf, umfassend die Verabreichung einer therapeutisch wirksamen Menge einer Verbindung der allgemeinen Formel **I**, gegebenenfalls zusammen mit einem oder mehreren Hilfsstoffen und/oder Verdünnungsmitteln.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetyl-salicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing lonenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosol-formulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der allgemeinen Formel gemäß den obigen bevorzugten Ausführungsformen.

Besonders bevorzugt ist es, wenn die Verbindungen der allgemeinen Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss-oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der allgemeinen Formel **I** inhalativ verabreicht werden, besonders bevorzugt ist es, wenn die Verabreichung ein oder zweimal täglich erfolgt. Hierzu müssen die Verbindungen der allgemeinen Formel **I** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Die nachfolgenden Verbindungen können analog der voranstehend beschriebenen allgemeinen Herstellverfahren (Schema 1 bis 14) synthetisiert werden:
Beispiel 1 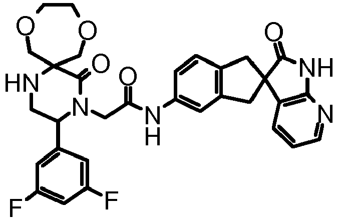
Beispiel 2 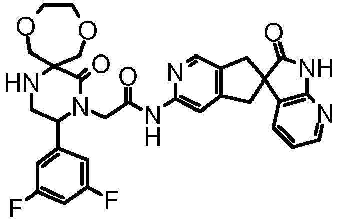
Beispiel 3 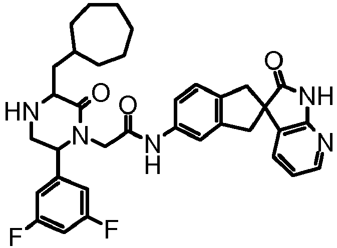
Beispiel 4 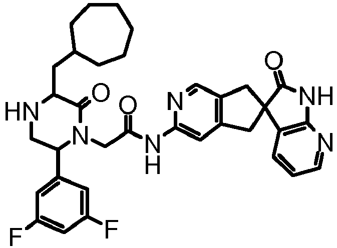
Beispiel 5 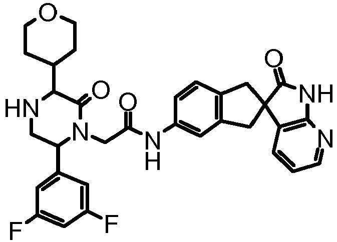
Beispiel 6 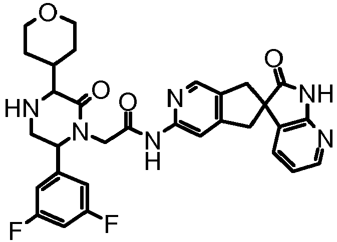
Beispiel 7 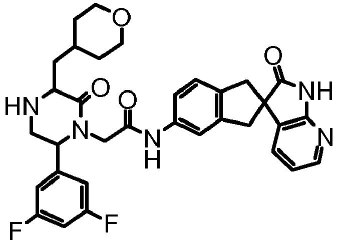
Beispiel 8 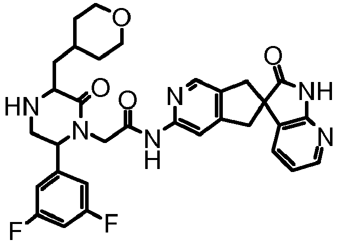
Beispiel 9 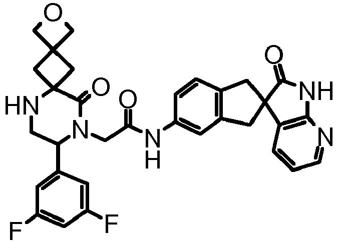
Beispiel 10 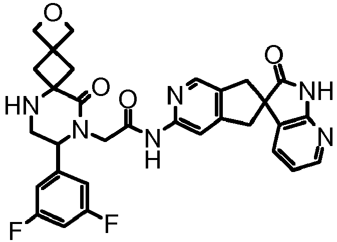
Beispiel 11 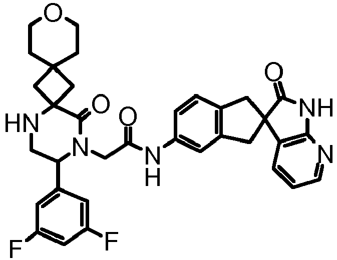
Beispiel 12 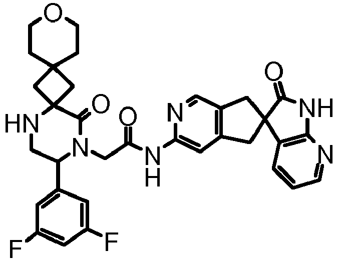
Beispiel 13 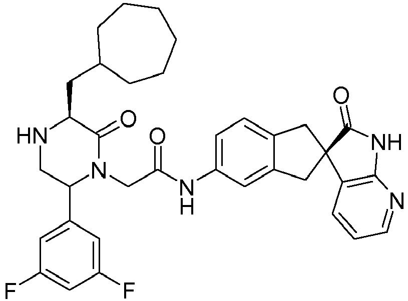
Beispiel 14 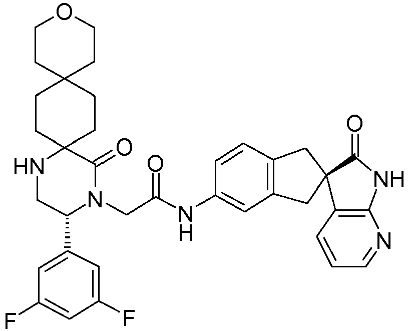
Beispiel 15 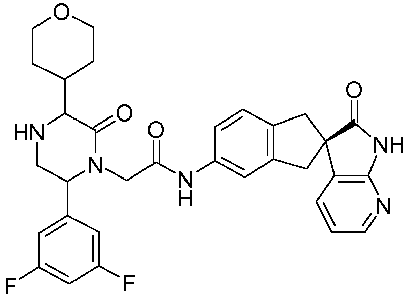
Beispiel 16 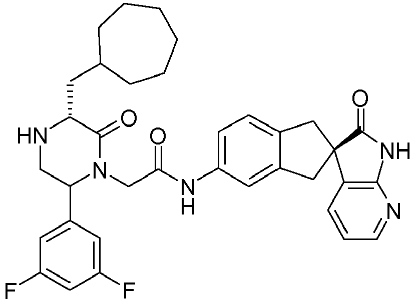
Beispiel 17 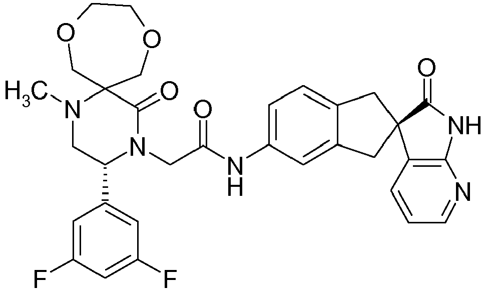
Beispiel 18 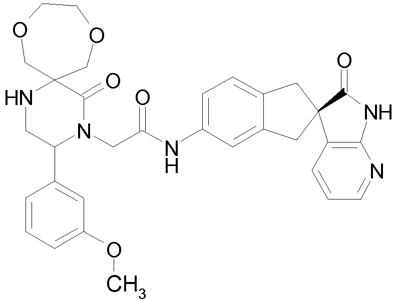
Beispiel 19 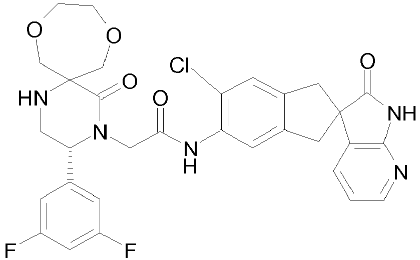
Beispiel 20 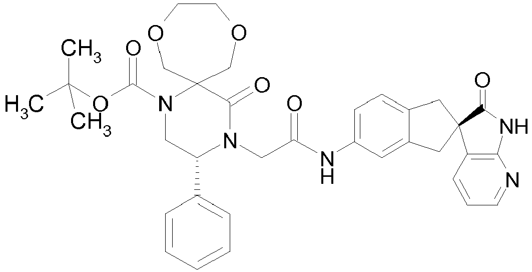
Beispiel 21 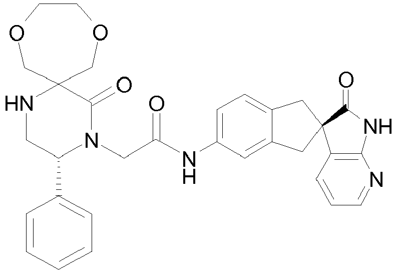
Beispiel 22 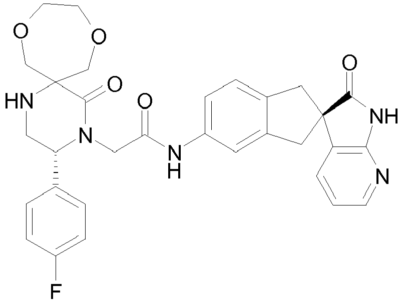
Beispiel 23 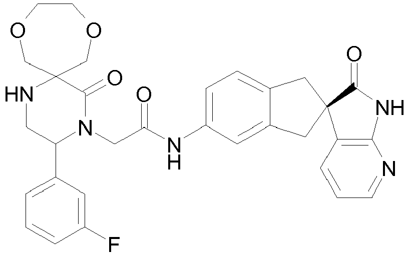
Beispiel 24

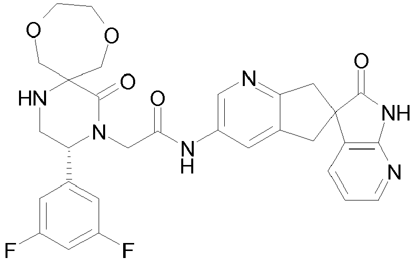

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.

Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.

Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX*™*, 35 bis 70 µm) verwendet.

Die angegebenen HPLC-Daten werden unter nachstehend angeführten Parametern und unter Verwendung der aufgeführten Säulen erhoben:

### Verwendete Säulen:

**(Säulentemperatur: 30°C; Injektionsvolumen: 5 µL; Detektion bei 254 nm)**

| | |
|---|---|
| S1 | Zorbax-Säule (Agilent Technologies), SB (Stable Bond) C18; |
| | 1.8 µm; 3.0 x 30 mm |
| S2 | Sunfire C18 (Waters);C18; |
| | 2.5 µm; 3.0 x 30 mm |
| S3 | Zorbax-Säule (Agilent Technologies), SB (Stable Bond) C18; |
| | 3.5 µm; 4.6 x 75 mm |
| S4 | Waters XBridge; C18; |
| | 2.5 µM; 3.0 x 30 mm |

### Verwendete Lösungsmittel:

### Saure Bedingungen:

Lösungsmittel A: Wasser (mit 0.1% Ameisensäure)
Lösungsmittel B: Acetonitril (mit 0.1% Ameisensäure)
(Die prozentualen Angaben beziehen sich auf das Gesamtvolumen.)

**Gradienten:**

| Gradient | Zeit [min] | %A | %B |
|---|---|---|---|
| **G1** (1.6 ml/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |
| **G2** (1.6 ml/min) | 0.00 | 95 | 5 |
| | 4.50 | 10 | 90 |
| | 5.00 | 10 | 90 |
| | 5.50 | 95 | 5 |

### Saure Bedingungen:

Lösungsmittel A: Wasser (mit 0.2% Ameisensäure)
Lösungsmittel B: Methanol
(Die prozentualen Angaben beziehen sich auf das Gesamtvolumen.)

**Gradienten:**

| Gradient | Zeit [min] | %A | %B |
|---|---|---|---|
| **G3** (1.6 ml/min) | 0.00 | 95 | 5 |
| | 4.50 | 10 | 90 |
| | 6.50 | 10 | 90 |
| | 7.00 | 95 | 5 |
| **G4** (1.6 ml/min) | 0.00 | 95 | 5 |
| | 2.00 | 10 | 90 |
| | 7.00 | 10 | 90 |
| | 7.50 | 95 | 5 |

### Basische Bedingungen:

Lösungsmittel A: Wasser (mit 0.2% Ammoniak)
Lösungsmittel B: Methanol + 3% Wasser
(Die prozentualen Angaben beziehen sich auf das Gesamtvolumen.)

**Gradienten:**

| Gradient | Zeit [min] | %A | %B |
|---|---|---|---|
| **G5** (1.6 ml/min) | 0.00 | 95 | 5 |
| | 0.20 | 95 | 5 |
| | 2.80 | 5 | 95 |
| | 3.00 | 5 | 95 |
| | 3.10 | 0 | 100 |
| | 3.80 | 0 | 100 |

### Methoden:

Die jeweilige verwendete HPLC-Methode ergibt sich aus Kombination der zuvor beschriebenen Säulen und Gradienten:

| | Säule | Gradient |
|---|---|---|
| Methode A | S1 | G1 |
| Methode B | S2 | G1 |
| Methode C | S3 | G2 |
| Methode D | S3 | G3 |
| Methode E | S3 | G4 |
| Methode F | S4 | G5 |

Bei präparativen HPLC-Reinigungen werden in der Regel die gleichen Gradienten verwendet, die bei der Erhebung der analytischen HPLC-Daten benutzt wurden. Die Sammlung der Produkte erfolgt massengesteuert, die das Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- AcOH: Essigsäure
- BINAP: 2,2'-Bis(diphenylphosphino)-1,1'-Binaphthyl
- Boc: *tert*-Butyloxycarbonyl
- Cyc: Cyclohexan
- CDI: 1,1'-Carbonyldimidazol
- DCM: Dichlormethan
- DIPE: Diisopropylether
- DIPEA: Diisopropylethylamin
- DMF: *N,N*-Dimethylformamid
- DMSO: Dimethylsulfoxid
- dppf: 1,1'-Bis(diphenyl-phosphino)ferrocen
- d. Th.: der Theorie
- EI: Elektronenstoß-Ionisation (bei MS)
- ESI: Elektrospray-lonisation (bei MS)
- EtOAc: Essigsäureethylester
- EtOH: Ethanol
- E-Wasser: Entionisiertes Wasser
- FM: Fließmittel
- HATU: O-(7-Azabenzotriazol-1-yl)-N,N,N,N-Tetramethylamethyluronium
- HCI: Chlorwasserstoff
- HCOOH: Ameisensäure
- HOBT: 1-Hydroxybenzotriazol
- HPLC: High Performance Liquid Chromatography
- HPLC-MS: HPLC gekoppelte Massenspektrometrie
- i.vac.: in vacuo (im Vakuum)
- konz.: konzentriert
- MeOH: Methanol
- MS: Massenspektrometrie
- MW: Molekulargewicht [g/mol]
- NaOH: Natriumhydroxid
- NH₄OH: Ammoniumhydroxid (wässrige Ammoniak-Lösung, 30%)
- NMP: *N*-Methyl-2-pyrrolidin
- Pd₂dba₃: Bis(dibenzylidenaceton)-palladium(0)
- PE: Petrolether
- R_{f}: Retentionsindex (bei DC)
- RP: reversed phase
- RT: Raumtemperatur
- Rₜ: Retentionszeit (bei HPLC)
- TEA: Triethylamin
- TFA: Trifluoressigsäure
- THF: Tetrahydrofuran
- ULTS: Umluft-Trockenschrank

### Herstellung der Ausgangsverbindungen:

### Intermediat 1:

### 6-Oxo-[1,4]dioxepan-5-carbonsäure-ethylester

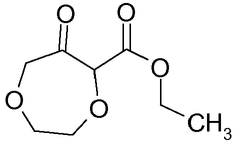

Der Ansatz wurde unter einer Stickstoff Schutzgasatmosphäre durchgeführt. 7.93 g (80.0 mmol) Natrium-*tert*-butylat wurden in 150 ml THF vorgelegt und auf Siedetemperatur erhitzt. Bei dieser Temperatur wurden 8.50 g (36.3 mmol) (2-Ethoxycarbonylmethoxy-ethoxy)-essigsäureethylester (Herstellung analog Canadian Journal of Chemistry; 74; 8; 1996; 1437-1446) in 100 ml THF innerhalb von 3 h zugetropft. Nach Beendigung der Zugabe wurde das Gemisch 1 h unter Rückfluss gekocht und anschließend über Nacht bei RT gerührt. Anschließend wurde der Rektionsansatz mit Eisessig auf pH5 eingestellt und einrotiert. Der Rückstand wurde mit Wasser versetzt und mit Essigsäureethylester extrahiert. Die organische Phasen wurden vereinigt, getrocknet und das Lösungsmittel unter vermindertem Druck und gelindem Erwärmen entfernt.

| | |
|---|---|
| Ausbeute: | 2.4 g (35% d. Th.) |
| ESI-MS: | m/z = 189 (M+H)⁺ |
| R_{f} : | 0.5 (Kieselgel, Petrolether/Essigsäureethylester = 2:1) |

### Intermediat 2:

### [1,4]Dioxepan-6-on

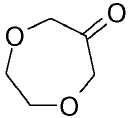

11.5.g (61.1 mmol) 6-Oxo-[1,4]dioxepan-5-carbonsäure-ethylester wurden mit 100 ml einer wässrigen 10%-igen Salzsäure Lösung 2.5 h unter Rückfluss gekocht. Nach dem Abkühlen wurde das Reaktionsgemisch mit Kaliumcarbonat gesättigt und anschließend mit Diethylether extrahiert. Die organischen Phasen wurden vereinigt und bis zur Trockne einrotiert.

| | |
|---|---|
| Ausbeute: | 4.50g (63% d. Th.) |
| EI-MS: | m/z = 116 (M*)⁺ |
| Rₜ (HPLC): | 1.27 min (Methode C) |

### Intermediat 3:

### (R)-3-(3,5-Difluor-phenyl)-8,11-dioxa-1,4-diaza-spiro[5.6]dodecan-5-on

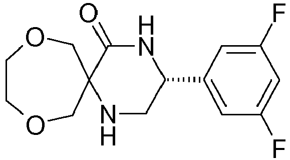

3.13 g (15.0 mmol) (R)-1-(3,5-Difluorphenyl)ethan-1,2-diamin-Hydrochlorid wurden mit 0.17 g (0.73 mmol) Benzyltriethylammoniumchlorid in 30 ml Dichlormethan vorgelegt und mit Eis/Aceton gekühlt. Anschließend wurden 5.21 ml (102.2 mmol) einer 50%igen Natronlauge zugegeben. Bei 0°C bis 2°C wurden 1.70 g (14.6 mmol) [1,4]Dioxepan-6-on zusammen mit 1.83 ml (22.7 mmol) Chloroform und 20 ml Dichlormethan innerhalb von 1.5 h zugetropft. Über Nacht ließ man den Ansatz auf RT erwärmen. Anschließend wurde auf 0°C gekühlt und mit einer 6N wässrigen Salzsäure Lösung der Reaktionsansatz auf pH1 gestellt. Die Phasen wurden getrennt. Die wässrige Phase wurde mit einer 4N Natronlauge alkalisch gestellt und mit Essigsäureethylester extrahiert. Die vereinigten organischen Phasen wurden getrocknet und bis zur Trockne einrotiert. Die Aufreinigung erfolgte mittels flash-Chromatographie.

| | |
|---|---|
| Ausbeute: | 1.10 g (25% d. Th.) |
| ESI-MS: | m/z = 299 (M+H)⁺ |
| Rₜ (HPLC): | 0.95 min (Methode A) |

Die enantiomere (S)-Verbindung kann analog der voranstehend beschriebenen Synthese unter Verwendung der entsprechenden (*S*)-konfigurierten Reaktionskomponente erhalten werden.

### Intermediat 4:

### (R)-tert-Butyl 3-(3,5-difluorphenyl)-5-oxo-8,11-dioxa-1,4-diazaspiro[5.6]dodecan-1-carboxylat

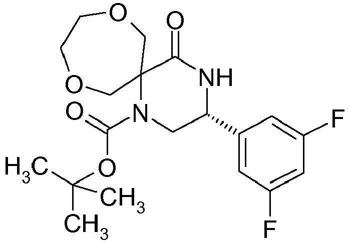

Zu 1.10 g (3.69 mmol) (*R*)-3-(3,5-Difluor-phenyl)-8,11-dioxa-1,4-diaza-spiro[5.6]dodecan-5-on und 0.64 ml (3.70 mmol) DIPEA in 30 ml Acetonitril wurde bei RT 3.93 g (18.0 mmol) Di-*tert*-butyl-dicarbonat zugegeben. Nach 7 h Kochen unter Rückfluss wurden nochmals 2.00 g (9.16 mmol) Di-*tert*-butyl-dicarbonat zugegeben und über Nacht am Rückfluss gekocht. Anschließend wurde das Lösungsmittel abrotiert und der Rückstand mittel flash-Chromatographie gereinigt.

| | |
|---|---|
| Ausbeute: | 0.52 g (35% d. Th.) |
| ESI-MS: | m/z = 399 (M+H)⁺ |
| Rₜ (HPLC): | 1.33 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 5:

### (R)-3-(3,5-Difluor-phenyl)-4-methoxycarbonylmethyl-5-oxo-8,11-dioxa-1,4-diaza-spiro-[5.6]dodecan-1-carbonsäure-tert-butylester

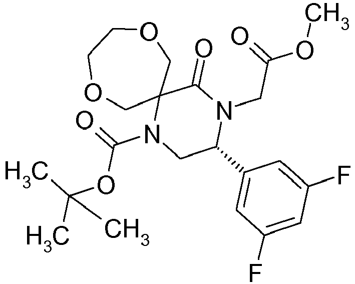

Der Ansatz wurde unter einer Stickstoff-Schutzgasatmosphäre durchgeführt. Zu 0.52 g (1.31 mmol) 5-(*R*)-*tert*-Butyl 3-(3,5-difluorphenyl)-5-oxo-8,11-dioxa-1,4-diazaspiro-[5.6]-dodecan-1-carboxylat in 20 ml DMF wurden bei 0°C 62.8 mg (1.44 mmol) Natriumhydrid (55% in Paraffinöl) zugegeben und für 20 min bei 0°C gerührt. Anschließend wurden 0.14 ml (1.44 mmol) Bromessigsäure-methylester in 5 ml DMF langsam zugetropft und für 30 min bei 0°C und 5 h bei RT gerührt. Der Ansatz wurde mit Wasser versetzt und mit Essigsäureethylester extrahiert. Die vereinigten organischen Phasen wurden getrocknet und bis zur Trockne einrotiert.

| | |
|---|---|
| Ausbeute: | 0.49 g (80% d. Th.) |
| ESI-MS: | m/z = 471 (M+H)⁺ |
| Rₜ (HPLC): | 1.43 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 6:

### (R)-4-Carboxymethyl-3-(3,5-difluor-phenyl)-5-oxo-8,11-dioxa-1,4-diaza-spiro[5.6]dodecan-1-carbonsäure-tert-butylester

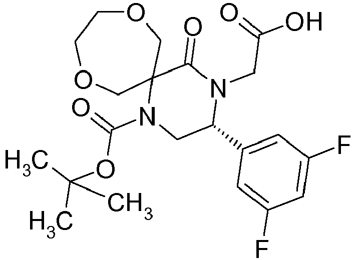

0.235 g (0.50 mmol) (*R*)-3-(3,5-Difluor-phenyl)-4-methoxycarbonylmethyl-5-oxo-8,11-dioxa-1,4-diaza-spiro[5.6]dodecan-1-carbonsäure-*tert*-butylester, 5 ml THF, 1 ml Wasser und 13.2 mg (0.55 mmol) Lithiumhydroxid wurden 2 h bei RT gerührt. Anschließend wurde mit einer 0.1 N Salzsäure auf pH7 eingestellt. Der Reaktionsansatz wurde bis zur Trockne einrotiert.

| | |
|---|---|
| Ausbeute: | 228 mg (quantitativ) |
| ESI-MS: | m/z = 455 (M-H)⁻ |
| Rₜ (HPLC): | 1.30 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 7:

### (S)-2-Amino-3-cycloheptyl-propionsäure-methylester

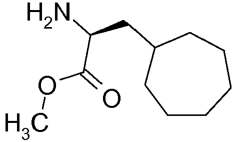

Bei -78°C wurden zu 2.90 ml (16.2 mmol) (*R*)-2-Isopropyl-3,6-dimethoxy-2,5-dihydro-pyrazin in 55 ml THF 7.80ml (19.5 mmol) n-Butyllithium (2.5 M in Hexan) zugetropft und 2 h bei -50°C nachgerührt. Anschließend wurden bei -70°C 4.25 g (17.8 mmol) (Iodmethyl)cycloheptan in 15 ml THF zugetropft, anschließend für 30 min bei -78°C, 3 h bei 0°C und über Nacht bei RT nachgerührt. Nach Zugabe von Wasser und Methanol wurde für 20 min bei RT gerührt. Es wurde Essigsäureethylester zugegeben und mit gesättigter, wässriger Kochsalzlösung extrahiert. Die organische Phase wurde getrocknet und bis zur Trockne einrotiert. Der Rückstand wurde mittels flash-Chromatographie aufgereinigt. Die das Produkt enthaltenden Fraktionen wurden vereinigt und einrotiert. Der erhaltene Rückstand wurde mit Acetonitril, Wasser und 1 M Salzsäure versetzt und über Nacht bei RT gerührt. Das Acetonitril wurde abgedampft und die wässrige Phase mit Kaliumhydrogencarbonat neutralisiert. Es wurde mit Essigsäureethylester extrahiert. Die vereinigten organischen Phasen wurden getrocknet und bis zur Trockne einrotiert.

| | |
|---|---|
| Ausbeute: | 1.04 g (32% d. Th.) |
| ESI-MS: | m/z = 200 (M+H)⁺ |
| Rₜ (HPLC): | 0.96 min (Methode A) |

Die enantiomere (R)-Verbindung kann analog der voranstehend beschriebenen Synthese unter Verwendung der entsprechenden (R)-konfigurierten Reaktionskomponente erhalten werden.

### Intermediat 8:

### (S)-2-Benzylamino-3-cycloheptyl-propionsäure-methylester

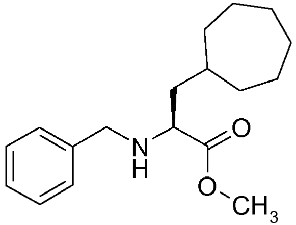

1.04 g (5.22 mmol) (*S*)-2-Amino-3-cycloheptyl-propionsäure-methylester wurden mit 0.62 ml (5.22 mmol) Benzylbromid, 1.44 g (10.4 mmol) Kaliumcarbonat und 20 ml DMF bei 100°C für 3.5 h gerührt. Anschließend wurde Wasser zugegeben und mit Essigsäureethylester extrahiert. Die organische Phase wurde eingedampft und der Rückstand mittels flash-Chromatographie gereinigt.

| | |
|---|---|
| Ausbeute: | 824 mg (55% d. Th.) |
| ESI-MS: | m/z = 290 (M+H)⁺ |
| Rₜ (HPLC): | 1.25 min (Methode A) |

Die enantiomere (R)-Verbindung kann analog der voranstehend beschriebenen Synthese unter Verwendung der entsprechenden (R)-konfigurierten Ausgangskomponente erhalten werden.

### Intermediat 9:

### (S)-2-(Benzyl-{2-(3,5-difluor-phenyl)-2-[hydroxyimino]-ethyl}-amino)-3-cycloheptyl-propionsäure-methylester

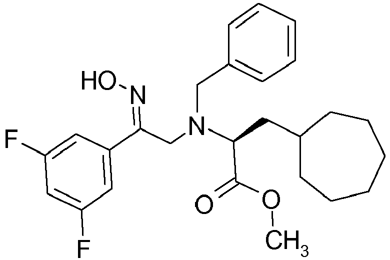

820 mg (2.83 mmol) (S)-2-Benzylamino-3-cycloheptyl-propionsäure-methylester wurden zusammen mit 0.71 g (2.83 mmol) 2-Brom-1-(3,5-difluor-phenyl)-ethanon-oxim, 0.51 g (3.68 mmol) Kaliumcarbonat und 10 ml THF über Nacht bei RT gerührt. Nach Zugabe von Wasser und Essigsäureethylester wurden die organische Phasen vereinigt, mit Wasser gewaschen, getrocknet und bis zur Trockne einrotiert. Der Rückstand wurde mittels RP-HPLC gereinigt. Die produktenthaltenden Fraktionen wurden vereinigt, das organische Lösungsmittel entfernt und der wässrige Rückstand mit gesättigter, wässriger Natriumhydrogencarbonat-Lösung alkalisch gestellt. Es wurde mit Essigsäureethylester extrahiert, die organische Phase getrocknet und einrotiert.

| | |
|---|---|
| Ausbeute: | 600 mg (46% d. Th.) |
| ESI-MS: | m/z = 459 (M+H)⁺ |
| Rₜ (HPLC): | 5.70 min /5.93 min (Methode D) |

Die enantiomere (R)-Verbindung kann analog der voranstehend beschriebenen Synthese unter Verwendung der entsprechenden (R)-konfigurierten Ausgangskomponente erhalten werden.

### Intermediat 10:

### (S)-3-Cycloheptylmethyl-6-(3,5-difluor-phenyl)-piperazin-2-on

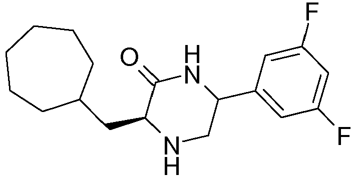

Unter einer Wasserstoffatmosphäre wurden 0.590 g (1.29 mmol) (S)-2-(Benzyl-{2-(3,5-difluor-phenyl)-2-[hydroxyimino]-ethyl}-amino)-3-cycloheptyl-propionsäure-methylester und 0.10 g Palladium auf Kohle (10%) in 10 ml Methanol für 3 Tage bei 50°C und 3.45 bar Wasserstoffdruck hydriert. Nach dem Entfernen des Katalysators wurde das Filtrat einrotiert. Der Rückstand wurde mit Petrolether und Diisopropylether verrührt und abfiltriert.

| | |
|---|---|
| Ausbeute: | 210 mg (51% d. Th.) |
| ESI-MS: | m/z = 323 (M+H)⁺ |
| Rₜ (HPLC): | 0.92 min / 1.00 min (Methode A) |

### Intermediat 11:

### (2S)-tert-Butyl-2-(cycloheptylmethyl)-5-(3,5-difluorphenyl)-3-oxopiperazin-1-carboxylat

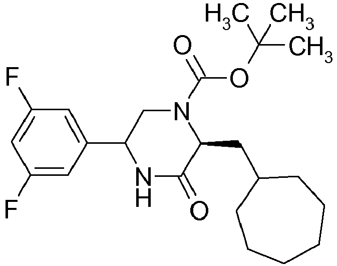

120 mg (0.37 mmol) (3*S*)-3-(Cycloheptylmethyl)-6-(3,5-difluorphenyl)piperazin-2-on, 90 mg (0.41 mmol) Di-*tert*-Butyldicarbonat und 0.06 ml (0.37 mmol) DIPEA wurden in 4 ml Acetonitril über Nacht bei RT gerührt. Das Reaktionsgemisch wurde eingeengt und der Rückstand zwischen Wasser und Essigsäureethylester verteilt. Die wässrige Phase wurde mit Essigsäureethylester extrahiert. Die organische Phase wurde getrocknet und eingeengt.

| | |
|---|---|
| Ausbeute: | 90 mg (57% d. Th.) |
| ESI-MS: | m/z = 421 (M-H)⁻ |
| Rₜ (HPLC): | 2.26/2.39 min (Methode F) |

### Intermediat 12:

### (2S)-tert-Butyl-2-(cycloheptylmethyl)-5-(3,5-difluorphenyl)-4-(2-methoxy-2-oxoethyl)-3-oxopiperazin-1-carboxylat

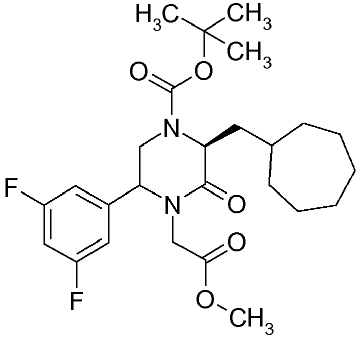

Unter einer Stickstoffatmosphäre wurden bei 0°C zu 90 mg (0.21 mmol) (2*S*)-*tert*-Butyl-2-(cycloheptylmethyl)-5-(3,5-difluorphenyl)-3-oxopiperazin-1-carboxylat in 3 ml DMF 10 mg (0.23 mmol) Natriumhydrid zugegeben. Nach 30 min bei 0°C wurden 0.02 ml (0.26 mmol) Bromessigsäuremethylester zugegeben und anschließend das Reaktionsgemisch über Nacht auf RT erwärmt. Nach dem Einengen wurde der Rückstand mit Wasser versetzt und zweimal mit Essigsäureethylester extrahiert. Die organische Phase wurde getrocknet und eingeengt.

| | |
|---|---|
| Ausbeute: | 105 mg (quantitativ) |
| ESI-MS: | m/z = 395 (M-Boc+H)⁺ |
| Rₜ (HPLC): | Diastereomere: 2.26 / 2.39 min (Methode E) |

### Intermediat 13:

### 2-((3S)-4-(tert-butoxycarbonyl)-3-(cycloheptylmethyl)-6-(3,5-difluorphenyl)-2-oxopiperazin-1-yl)ethansäure

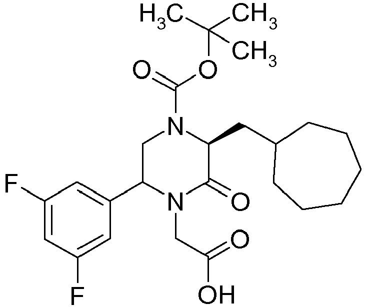

Zu 105 mg (0.21 mmol) (2*S*)-*tert*-Butyl-2-(cycloheptylmethyl)-5-(3,5-difluorphenyl)-4-(2-methoxy-2-oxoethyl)-3-oxopiperazin-1-carboxylat in 2 ml THF wurden 10 mg (0.42 mmol) Lithiumhydroxid in 800 µl Wasser zugegeben. Nach 2 h wurde Tetrahydrofuran abdestilliert und die wässrige Lösung mit Salzsäure neutralisiert. Die wässrige Phase wurde mit Essigsäureethylester extrahiert. Die Essigsäureethylester-Phase wurde getrocknet und eingeengt.

| | |
|---|---|
| Ausbeute: | 100 mg (98% d. Th.) |
| ESI-MS: | m/z = 381 (M-Boc+H)⁺ |
| Rₜ (HPLC): | 1.36 / 1.51 min (Methode E) |

### Intermediat 14:

### 3-Oxaspiro[5.5]undec-7-en-9-on

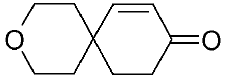

5.00 g (43.8 mmol) Tetrahydropyran-4-carboxaldehyd und 3.56 ml (43.9 mmol) Methylvinylketon wurden in 70 ml Toluol mit 0.07 ml konz. Schwefelsäure am Wasserabscheider gekocht. Nach 3 h wurden nochmals 3.56 ml Methylvinylketon zugegeben und weitere 3 h gekocht. Nach dem Erkalten wurde das Reaktionsgemisch in Essigsäureethylester aufgenommen, mit Wasser gewaschen, getrocknet und einrotiert.

| | |
|---|---|
| Ausbeute: | 1.30 g (18% d. Th.) |
| ESI-MS: | m/z = 167 (M+H)⁺ |
| Rₜ (HPLC): | 1.07 min (Methode A) |

### Intermediat 15:

### 3-Oxaspiro[5.5]undecan-9-on

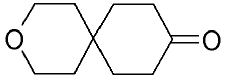

Unter einer Wasserstoff-Atmosphäre wurden 1.30 g (7.82 mmol) 3-Oxaspiro[5.5]undec-7-en-9-on mit 0.2 g Pd/C (10%ig) in 15 ml Essigsäureethylester bei 50°C und 3 bar Wasserstoffdruck drei Tage hydriert. Das Reaktionsgemisch wurde abgesaugt und eingeengt.

| | |
|---|---|
| Ausbeute: | 1.20 g (91% d. Th.) |
| ESI-MS: | m/z = 168 M*+ |
| Rₜ (HPLC): | 0.69 min (Methode A) |

### Intermediat 16:

### (3R)-(3,5-Difluorphenyl)-12-oxa-1,4-diaza-dispiro[5.2.5.2]hexadecan-5-on

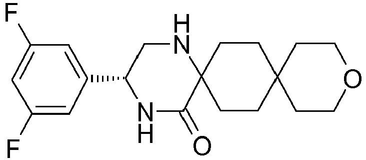

1.53 g (7.32 mmol) (1*R*)-1-(3,5-difluorphenyl)-ethan-1,2-diamin-Hydrochlorid und 82 mg (0.36 mmol) Benzyltriethylammoniumchlorid wurden in 50 ml Dichlormethan vorgelegt. Unter Eis/Aceton-Kühlung wurden 2.55 ml (50.0 mmol) einer 50%igen, wässrigen NaOH zugegeben. Bei 0-2°C wurden 1.20 g (7.13 mmol) 3-Oxaspiro[5.5]undecan-9-on und 0.89 ml (11.0 mmol) Chloroform in 20 ml Dichlormethan innerhalb von 1.5 h zugegeben. Der Reaktionsansatz wurde über Nacht auf RT erwärmt. Bei 0°C wurden 2.14 ml (12.9 mmol) einer 6M Salzsäure zugegeben. Die wässrige Phase wurde mit einer 6M Salzsäure auf pH1 eingestellt. Die organische Phase wurde abgetrennt und verworfen. Die Wasserphase wurde alkalisch gestellt und zweimal mit Dichlormethan extrahiert. Die vereinigten organischen Phasen wurden getrocknet und eingeengt. Der Rückstand wurde mittels flash-Chromatographie aufgereinigt.

| | |
|---|---|
| Ausbeute: | 220 mg (9% d. Th.) |
| ESI-MS: | m/z = 351 (M+H)⁺ |
| Rₜ (HPLC): | 0.78 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 17:

### (3R)-(3,5-Difluorphenyl)-5-oxo-12-oxa-1,4-diaza-dispiro[5.2.5.2]hexadecan-1-carbonsäure-tert-butylester

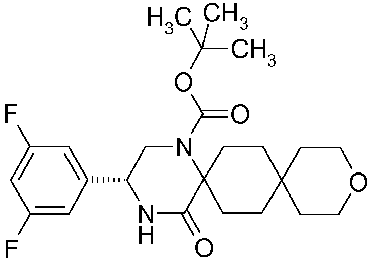

Zu 220 mg (0.63 mmol) (3*R*)-(3,5-Difluorphenyl)-12-oxa-1,4-diaza-dispiro-[5.2.5.2]-hexadecan-5-on und 0.10 ml (0.60 mmol) DIPEA in 10 ml ACN wurden bei RT 687 mg (3.15 mmol) Di-*tert*-butyldicarbonat zugegeben. Nach 4 h bei 60°C wurden nochmals 687 mg Di-*tert*-butyldicarbonat zugegeben. Nach weiteren 3 h bei 60°C wurden 687mg Di*-tert-*butyldicarbonat zugegeben und über Nacht bei 60°C gerührt. Der Reaktionsansatz wurde eingeengt. Der Rückstand wurde mit Diisopropylether/Petrolether kristallisiert. Der Feststoff wurde abgesaugt, mit Diisopropylether/Petrolether gewaschen und getrocknet.

| | |
|---|---|
| Ausbeute: | 83 mg (29% d. Th.) |
| ESI-MS: | m/z = 451 (M+H)⁺ |
| Rₜ (HPLC): | 1.65 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 18:

### (3R)-(3,5-Difluorphenyl)-4-methoxycarbonylmethyl-5-oxo-12-oxa-1,4-diaza-dispiro-[5.2.5.2]-hexadecan-1-carbonsäure-tert-butylester

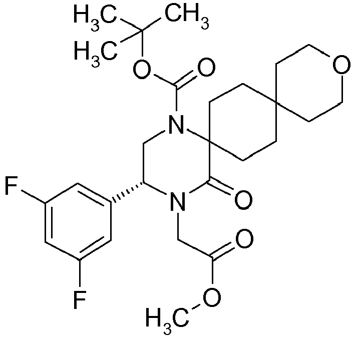

Unter einer Stickstoff-Atmosphäre wurden bei 0°C zu 80 mg (0.18 mmol) (3*R*)-(3,5-Di-fluorphenyl)-5-oxo-12-oxa-1,4-diaza-dispiro-[5.2.5.2]-hexadecan-1-carbonsäure-*tert-*butylester in 15 ml DMF 9 mg (0.20 mmol) Natriumhydrid zugegeben. Nach 20 min bei 0°C wurden 20 µl (0.20 mmol) Bromessigsäuremethylester zugegeben. Der Reaktionsansatz wurde 30 min bei 0°C und 5 h bei RT nachgerührt. Der Ansatz wurde auf Wasser gegossen und zweimal mit Essigsäureethylester extrahiert. Die vereinigten organischen Phasen wurden mit Wasser gewaschen, getrocknet und eingeengt.

| | |
|---|---|
| Ausbeute: | 83 mg (89% d. Th.) |
| ESI-MS: | m/z = 523 (M+H)⁺ |
| Rₜ (HPLC): | 1.75 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 19:

### 4-Carboxymethyl-(3R)-(3,5-difluorphenyl)-5-oxo-12-oxa-1,4-diaza-dispiro-[5.2.5.2]-hexadecan-1-carbonsäure-tert-butylester

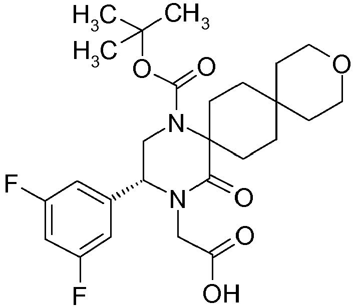

Zu 80 mg (0.15 mmol) (3R)-(3,5-Difluorphenyl)-4-methoxycarbonylmethyl-5-oxo-12-oxa-1,4-diaza-dispiro[5.2.5.2]hexadecan-1-carbonsäure-*tert*-butylester in 5 ml THF und 1 ml Wasser wurden 4 mg (0.18 mmol) Lithiumhydroxyid zugegeben und bei RT 2 h gerührt. Der Ansatz wurde mit einer 0.1 M Salzsäure auf pH 7 eingestellt, eingedampft und getrocknet.

| | |
|---|---|
| Ausbeute: | 78 mg (quantitativ) |
| ESI-MS: | m/z = 507 (M-H)⁻ |
| Rₜ (HPLC): | 1.56 min (Methode A) |

Die enantiomere (S)-Verbindung kann analog der voranstehend beschriebenen Synthese unter Verwendung der entsprechenden (*S*)-konfigurierten Ausgangskomponente erhalten werden.

### Intermediat 20:

### (S)-5-Amino-1,3-dihydrospiro[inden-2,3'-pyrrolo[2,3-b]pyridin]-2'(1'H)-on

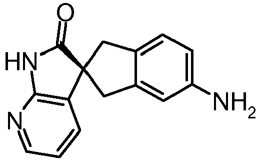

Diese Verbindung und deren Vorstufen wurden analog zur WO 2007/061677 synthetisiert.

### Intermediat 21:

### (R)-5-Amino-1,3-dihydrospiro[inden-2,3'-pyrrolo[2,3-b]pyridin]-2'(1'H)-on

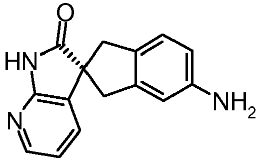

Diese Verbindung und deren Vorstufen wurden analog zur WO 2007/061677 synthetisiert.

### Intermediat 22:

### 2-Brom-1-(3,5-difluor-phenyl)-ethanonoxim

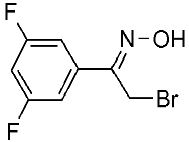

34.5 g (146.8 mmol) 3,5-Difluorphenacylbromid, 30.6 g (440.4 mmol) Hydroxylamin-hydrochlorid, 44.0 ml Wasser und 320.0 mL Methanol wurden über Nacht bei RT nachgerührt. Der Reaktionsansatz wurde am Rotationsverdampfer einrotiert und mit 150 mL Wasser versetzt und zweimal mit Essigsäureethylester extrahiert. Die vereinigten organischen Phasen wurden mit Wasser gewaschen, getrocknet und bis zur Trockne einrotiert.

| | |
|---|---|
| Ausbeute: | 32.7 g (89 % d. Th.) |

### Intermediat 23:

### 3-(3-Methoxyphenyl)-8,11-dioxa-1,4-diazaspiro[5.6]dodecan-5-on

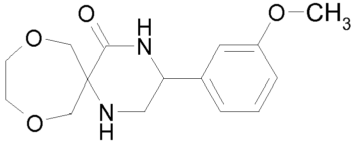

32 mg (0.14 mmol) Benzyltriethylammoniumchlorid und 0.50 g 1-(3-Methoxyphenyl)ethan-1,2-diamin (hergestellt analog zu DE2240256) in 5 ml DCM wurden auf 0°C gekühlt und mit 1.04 ml (19 mmol) 50%iger wässriger Natriumhydroxidlösung versetzt. Bei dieser Temperatur wurden 0.44 g 80%iges (3 mmol) [1,4]Dioxepan-6-on und 0.37 ml (4.6 mmol) Trichlormethan in 4 ml DCM zugetropft. Den Reaktionsansatz ließ man über Nacht auf RT erwärmen. Die Reaktion wurde mit einer 4N Salzsäure Lösung sauer gestellt und die Phasen getrennt. Die wässrige Phase wurde mit einer 4N Natriumhydroxid-Lösung basisch gestellt und mit EtOAc extrahiert. Die organische Phase wurde getrocknet und eingeengt. Der Rückstand wurde mittels flash-Chromatographie aufgereinigt.

| | |
|---|---|
| Ausbeute: | 390 mg (45% d. Th.) |
| ESI-MS: | m/z = 293 (M-H)⁻ |
| Rₜ (HPLC): | 0.63 min (Methode A) |

### Intermediat 24:

### 3-(3-Methoxyphenyl)-5-oxo-8,11-dioxa-1,4-diazaspiro[5.6]dodecan-1-carbonsäure-tert-butylester

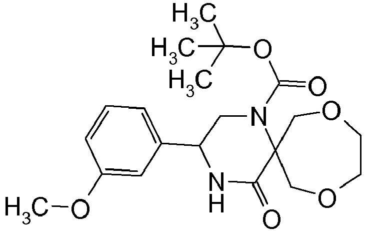

Zu 0.38 g (1.3 mmol) 3-(3-Methoxyphenyl)-8,11-dioxa-1,4-diazaspiro[5.6]dodecan-5-on in 10 ml THF wurden 0.44 ml (2.6 mmol) DIPEA und 0.40 g (1.8 mmol) Di-*tert*-butyldicarbonat zugegeben. Über Nacht wurde die Reaktion unter Rückfluss gekocht. Der Reaktionsansatz wurde eingeengt. Der Rückstand wurde mit Diethylether verrieben. Der Feststoff wurde abgesaugt, mit Diethylether gewaschen und getrocknet.

| | |
|---|---|
| Ausbeute: | 345 mg (69% d. Th.) |
| ESI-MS: | m/z = 393 (M+H)⁺ |

### Intermediat 25:

### 3-(3-Methoxyphenyl)-5-oxo-8,11-dioxa-1,4-diazaspiro[5.6]dodecan-1-carbonsäure-tert-butylester

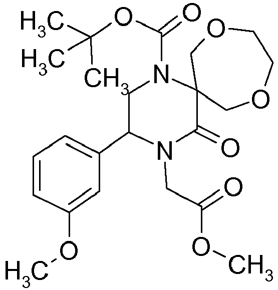

Zu 0.33 g (0.83 mmol) 3-(3-Methoxyphenyl)-5-oxo-8,11-dioxa-1,4-diazaspiro[5.6]dodecan-1-carbonsäure-*tert*-butylester in 5 ml DMF wurden 30 mg (0.91 mmol) Natriumhydrid zugegeben. Nach 5 min bei RT wurden 89 µl (0.91 mmol) Bromessigsäuremethylester zugegeben und anschließend das Reaktionsgemisch 1h bei RT gerührt. Der Reaktionsansatz wurde auf Eiswasser gegossen und mit Essigsäureethylester extrahiert. Die organische Phase wurde mit Wasser gewaschen, getrocknet und eingeengt.

| | |
|---|---|
| Ausbeute: | 380 mg (quantitativ) |
| ESI-MS: | m/z = 465 (M+H)⁺ |

### Intermediat 26:

### 2-(1-(tert-Butoxycarbonyl)-3-(3-methoxyphenyl)-5-oxo-8,11-dioxa-1,4-diazaspiro[5.6]-dodecan-4-yl)ethansäure

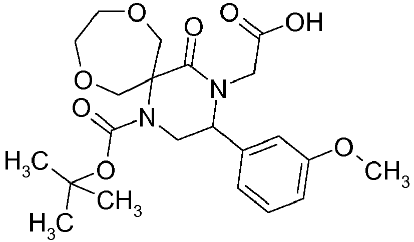

Zu 0.38 g (0.82 mmol) (3-(3-Methoxyphenyl)-5-oxo-8,11-dioxa-1,4-diazaspiro[5.6]dodecan-1-carbonsäure-*tert*-butylester in 5 ml MeOH wurden 2.5 ml (2.5 mmol) einer 1 N wässrige Natronlauge zugegeben. Nach 1 h wurde MeOH abdestilliert und die wässrige Lösung mit 1 N Salzsäure sauer gestellt. Die wässrige Phase wurde mit Essigsäureethylester extrahiert. Die Essigsäureethylester-Phase wurde getrocknet und eingeengt.

| | |
|---|---|
| Ausbeute: | 360 mg (98% d. Th.) |
| ESI-MS: | m/z = 451 (M+H)⁺ |
| Rₜ (HPLC): | 1.24 min (Methode A) |

### Intermediat 27:

### tert-Butyl 3-(3-methoxyphenyl)-5-oxo-4-(2-oxo-2-((R)-2'-oxo-1,1',2',3-tetrahydrospiro-[inden-2,3'-pyrrolo[2,3-b]pyridin]-5-ylamino)ethyl)-8,11-dioxa-1,4-diazaspiro[5.6]dodecan-1-carboxylat

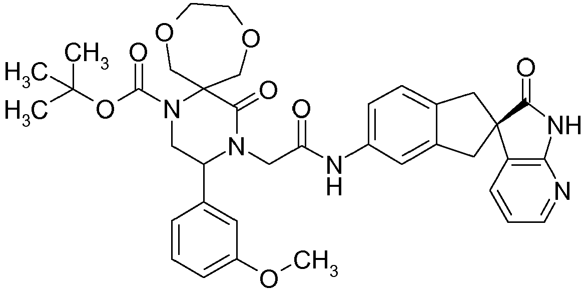

0.44 g (0.81 mmol) 80%ige 2-(1-(*tert*-Butoxycarbonyl)-3-(3-methoxyphenyl)-5-oxo-8,11-dioxa-1,4-diazaspiro[5.6]dodecan-4-yl)ethansäure in 10 ml DMF wurden mit 0.75 g (2.0 mmol) HATU und 0.34 ml (2.4 mmol) TEA versetzt und 10 min bei RT gerührt. Anschließend wurden 0.25 g (0.98 mmol) (*S*)-5-Amino-1,3-dihydrospiro[inden-2,3'-pyrrolo[2,3-b]pyridin]-2'(1'H)-on zugegeben und über Nacht bei RT gerührt. Das Reaktionsgemisch wurde auf Wasser gegossen. Der ausgefallene Niederschlag wurde abgesaugt, mit Wasser gewaschen und getrocknet.

| | |
|---|---|
| Ausbeute: | 610 mg (99% d. Th.) |
| ESI-MS: | m/z = 682 (M-H)⁻ |
| Rₜ (HPLC): | 1.39 min (Methode A) |

### Intermediat 28:

### 5-Amino-6-chlor-1,3-dihydrospiro[inden-2,3'-pyrrolo[2,3-b]pyridin]-2'(1'H)-on

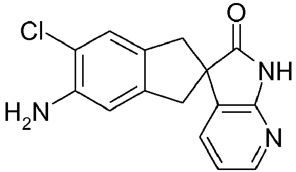

0.50 g (2.0 mmol) 5-Amino-1,3-dihydrospiro[inden-2,3'-pyrrolo[2,3-b]pyridin]-2'(1'H)-on in 20 ml DCM wurden mit 0.27 g (2 mmol) N-Chlorsuccinimid versetzt und 5h bei RT gerührt. Der Anatz wurde eingeengt und der Rückstand mittels HPLC gereinigt.

| | |
|---|---|
| Ausbeute: | 0.14 g (25% d. Th.) |
| ESI-MS: | m/z = 286 (M+H)⁺ |
| Rₜ (HPLC): | 1.10 min (Methode A) |

### Intermediat 29:

### (3R)-tert-Butyl 4-(2-(5-chlor-2'-oxo-1,1',2',3-tetrahydrospiro[inden-2,3'-pyrrolo[2,3-b]-pyridin]-6-ylamino)-2-oxoethyl)-3-(3,5-difluorphenyl)-5-oxo-8,11-dioxa-1,4-diazaspiro-[5.6]dodecan-1-carboxylat

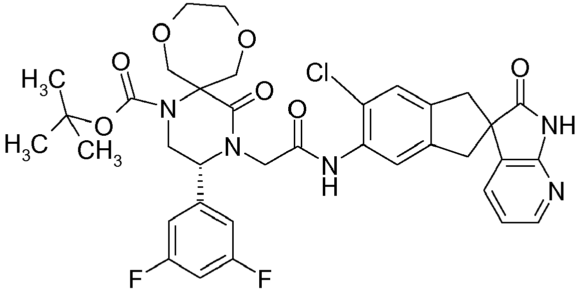

0.11 g (0.24 mmol) (*R*)-2-(1-(*tert*-Butoxycarbonyl)-3-(3,5-difluorphenyl)-5-oxo-8,11-dioxa-1,4-diazaspiro[5.6]dodecan-4-yl)ethansäue in 4 ml DMF wurden mit 0.11 g (0.3 mmol) HATU und 0.07 ml (0.5 mmol) TEA versetzt und 5 min bei RT gerührt. Anschließend wurden 70 mg (0.24 mmol) 5-Amino-6-chlor-1,3-dihydrospiro[inden-2,3'-pyrrolo[2,3-b]-pyridin]-2'(1'H)-on zugegeben und über Nacht bei RT gerührt. Das Reaktionsgemisch wurde mittels HPLC gereinigt.

| | |
|---|---|
| Ausbeute: | 40 mg (23% d. Th.) |
| ESI-MS: | m/z = 725 (M+H)⁺ |
| Rₜ (HPLC): | 1.5 min (Methode A) |

### Intermediat 30:

### (R)-3-Phenyl-8,11-dioxa-1,4-diazaspiro[5.6]dodecan-5-on

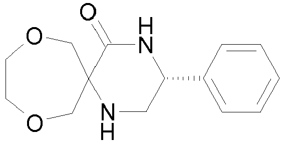

0.63 g (3.0 mmol) (1*S*)1-Phenylethan Dihydrochlorid wurden mit 32 mg (0.14 mmol) Benzyltriethylammoniumchlorid in 6 ml Dichlormethan vorgelegt und mit Eis/Aceton gekühlt. Anschließend wurden 1.0 ml (19 mmol) einer 50%igen Natronlauge zugegeben. Bei 0°C wurden 0.44 g (3 mmol) 80%iges [1,4]Dioxepan-6-on zusammen mit 0.37 ml (4.6 mmol) Chloroform und 5 ml Dichlormethan zugetropft. Über Nacht ließ man den Ansatz auf RT erwärmen. Anschließend wurde der Reaktionsansatz mit einer wässrigen 4N Salzsäurelösung sauer gestellt. Die Phasen wurden getrennt. Die wässrige Phase wurde mit einer 4N Natronlauge alkalisch gestellt und mit Essigsäureethylester extrahiert. Die vereinigten organischen Phasen wurden getrocknet und bis zur Trockne einrotiert. Die Aufreinigung erfolgte mittels flash-Chromatographie.

| | |
|---|---|
| Ausbeute: | 250 mg (32% d. Th.) |
| ESI-MS: | m/z = 263 (M+H)⁺ |
| Rₜ (HPLC): | 0.44 min (Methode A) |

### Intermediat 31:

### (R)-tert-Butyl 5-oxo-3-phenyl-8,11-dioxa-1,4-diazaspiro[5.6]dodecan-1-carboxylat

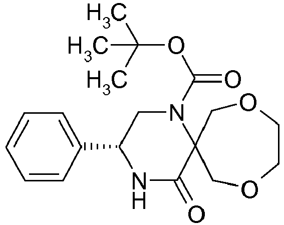

Zu 0.22 g (0.84 mmol) (3*R*)-3-Phenyl-8,11-dioxa-1,4-diazaspiro[5.6]dodecan-5-on in 10 ml THF wurden 0.31 ml (1.8 mmol) DIPEA und 0.28 g (1.2 mmol) Di-*tert*-butyldicarbonat zugegeben. Über Nacht wurde die Reaktion unter Rückfluss gekocht. Der Reaktionsansatz wurde eingeengt. Der Rückstand wurde mit Diethylether verrieben. Der Feststoff wurde abgesaugt, mit Diethylether gewaschen und getrocknet.

| | |
|---|---|
| Ausbeute: | 190 mg (63% d. Th.) |
| ESI-MS: | m/z = 363 (M+H)⁺ |
| Rₜ (HPLC): | 1.26 min (Methode A) |

### Intermediat 32:

### (R)-tert-Butyl 4-(2-methoxy-2-oxoethyl)-5-oxo-3-phenyl-8,11-dioxa-1,4-diazaspiro[5.6]dodecan-1-carboxylat

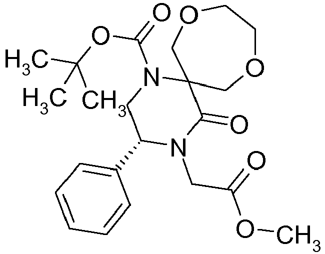

Zu 0.18 g (0.5 mmol) (3*R*)-*tert*-Butyl 5-oxo-3-phenyl-8,11-dioxa-1,4-diazaspiro[5.6]-dodecan-1-carboxylat_in 3 ml DMF wurden 24 mg (0.55 mmol) Natriumhydrid zugegeben. Nach 5 min bei RT wurden 54 µl (0.91 mmol) Bromessigsäuremethylester zugegeben und anschließend das Reaktionsgemisch 1h bei RT gerührt. Die Reaktion wurde auf Eiswasser gegossen und mit Essigsäureethylester extrahiert. Die organische Phase wurde mit Wasser gewaschen, getrocknet und eingeengt.

| | |
|---|---|
| Ausbeute: | 215 mg (quantitativ) |
| ESI-MS: | m/z = 435 (M+H)⁺ |
| Rₜ (HPLC): | 1.33 min (Methode A) |

### Intermediat 33:

### (R)-2-(1-(tert-Butoxycarbonyl)-5-oxo-3-phenyl-8,11-dioxa-1,4-diazaspiro[5.6]dodecan-4-yl)ethansäure

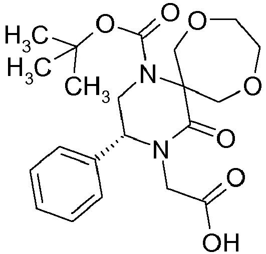

Zu 0.22 g (0.5 mmol) (*R*)*-tert-*Butyl 4-(2-methoxy-2-oxoethyl)-5-oxo-3-phenyl-8,11-dioxa-1,4-diazaspiro[5.6]dodecan-1-carboxylat_in 10 ml MeOH wurden 24 mg (1.0 mmol) Lithiumhydroxid in 1 ml Wasser zugegeben. Über Nacht wurde der Reaktionsansatz bei RT gerührt und anschließend das MeOH abdestilliert. Der Rückstand wurde mit Wasser versetzt und mit einer wässrigen 4N Salzsäure sauer gestellt. Die wässrige Phase wurde mit Essigsäureethylester extrahiert. Die Essigsäureethylester-Phase wurde getrocknet und eingeengt.

| | |
|---|---|
| Ausbeute: | 210 mg (quantitativ) |
| ESI-MS: | m/z = 421 (M+H)⁺ |
| Rₜ (HPLC): | 1.23 min (Methode A) |

### Intermediat 34:

### 3-(4-Fluorphenyl)-8,11-dioxa-1,4-diazaspiro[5.6]dodecan-5-on

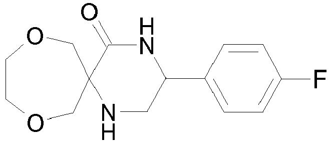

0.68 g (3.0 mmol) 1-(4-Fluorphenyl)ethan-1,2-diamin Dihydrochlorid wurden mit 32 mg (0.14 mmol) Benzyltriethylammoniumchlorid in 6 ml Dichlormethan vorgelegt und mit Eis/Aceton gekühlt. Anschließend wurden 1.0 ml (19 mmol) einer 50%igen Natronlauge zugegeben. Bei 0°C wurden 0.44 g (3.0 mmol) 80%iges [1,4]Dioxepan-6-on zusammen mit 0.37 ml (4.6 mmol) Chloroform und 5 ml Dichlormethan zugetropft. Über Nacht ließ man den Reaktionsansatz auf RT erwärmen. Anschließend wurde der Reaktionsansatz mit einer wässrigen 4N Salzsäure Lösung sauer gestellt. Die Phasen wurden getrennt. Die wässrige Phase wurde mit einer 4N Natronlauge alkalisch gestellt und mit Essigsäureethylester extrahiert. Die vereinigten organischen Phasen wurden getrocknet und bis zur Trockne einrotiert. Die Aufreinigung erfolgte mittels flash-Chromatographie.

| | |
|---|---|
| Ausbeute: | 260 mg (31% d. Th.) |
| ESI-MS: | m/z = 281 (M+H)⁺ |
| Rₜ (HPLC): | 0.56 min (Methode A) |

### Intermediat 35:

### tert-Butyl-3-(4-fluorphenyl)-5-oxo-8,11-dioxa-1,4-diazaspiro[5.6]dodecan-1-carboxylat

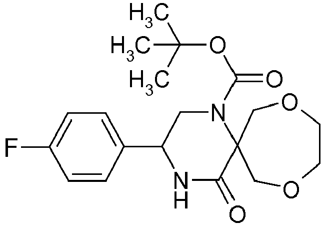

Zu 0.25 g (0.89 mmol) 3-(4-Fluorphenyl)-8,11-dioxa-1,4-diazaspiro[5.6]dodecan-5-on in 10 ml THF wurden 0.31 ml (1.8 mmol) DIPEA und 0.28 g (1.2 mmol) Di-*tert*-butyldicarbonat zugegeben. Die Reaktion wurde 2h bei RT und über Nacht unter Rückfluss gekocht. Der Reaktionsansatz wurde eingeengt. Der Rückstand wurde mit Diethylether verrieben. Der Feststoff wurde abgesaugt, mit Diethylether gewaschen und getrocknet.

| | |
|---|---|
| Ausbeute: | 240 mg (71 % d. Th.) |
| ESI-MS: | m/z = 381 (M+H)⁺ |
| Rₜ (HPLC): | 1.28 min (Methode A) |

### Intermediat 36:

### tert-Butyl-3-(4-fluorphenyl)-4-(2-methoxy-2-oxoethyl)-5-oxo-8,11-dioxa-1,4-diazaspiro[5.6]-dodecan-1-carboxylat

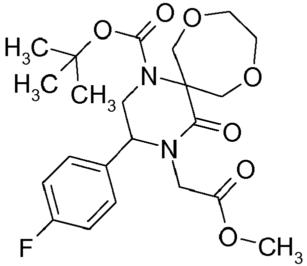

Zu 0.23 g (0.6 mmol) *tert*-Butyl-3-(4-fluorphenyl)-5-oxo-8,11-dioxa-1,4-diazaspiro[5.6]dodecan-1-carboxylat_in 5 ml DMF wurden 28 mg (0.66 mmol) Natriumhydrid zugegeben. Nach 5 min bei RT wurden 64 µl (0.91 mmol) Bromessigsäuremethylester zugetropft und anschließend wurde das Reaktionsgemisch 1h bei RT gerührt. Die Reaktion wurde auf Eiswasser gegossen und mit Essigsäureethylester extrahiert. Die organische Phase wurde mit Wasser gewaschen, getrocknet und eingeengt.

| | |
|---|---|
| Ausbeute: | 290 mg (quantitativ) |
| ESI-MS: | m/z = 453 (M+H)⁺ |
| Rₜ (HPLC): | 1.39 min (Methode A) |

### Intermediat 37:

### 2-(1-(tert-Butoxycarbonyl)-3-(4-fluorphenyl)-5-oxo-8,11-dioxa-1,4-diazaspiro[5.6]dodecan-4-yl)ethansäure

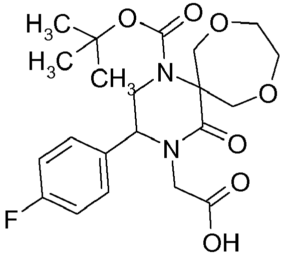

Zu 0.29 mg (0.61 mmol) *tert*-Butyl 3-(4-fluorphenyl)-4-(2-methoxy-2-oxoethyl)-5-oxo-8,11-dioxa-1,4-diazaspiro[5.6]dodecan-1-carboxylat in 5 ml MeOH wurden 2.0 ml (2.0 mmol) einer 1 N wässrigen Natronlauge zugegeben. Nach 1 h bei RT wurde MeOH abdestilliert und die wässrige Lösung mit 1 N Salzsäure sauer gestellt. Die wässrige Phase wurde mit Essigsäureethylester extrahiert. Die Essigsäureethylester-Phase wurde getrocknet und eingeengt.

| | |
|---|---|
| Ausbeute: | 265 mg (quantitativ) |
| ESI-MS: | m/z = 439 (M+H)⁺ |
| Rₜ (HPLC): | 1.25 min (Methode A) |

### Intermediat 38:

### 2-(3-(4-Fluorphenyl)-5-oxo-8,11-dioxa-1,4-diazaspiro[5.6]dodecan-4-yl)-N-((R)-2'-oxo-1,1',2',3-tetrahvdrospiro[inden-2,3'-pyrrolo[2,3-b]pyridin]-5-yl)acetamid

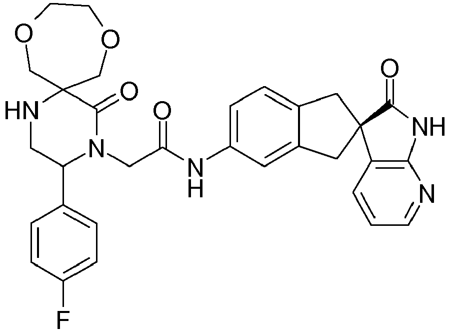

0.26 g (0.6 mmol) 2-(1-(*tert*-Butoxycarbonyl)-3-(4-fluorphenyl)-5-oxo-8,11-dioxa-1,4-diazaspiro[5.6]dodecan-4-yl)ethansäure in 10 ml DMF wurden mit 0.46 g (1.2 mmol) HATU und 0.20 ml (1.4 mmol) TEA versetzt und 10 min bei RT gerührt. Anschließend wurden 0.17 g (0.66 mmol) (*S*)-5-Amino-1,3-dihydrospiro[inden-2,3'-pyrrolo[2,3-b]pyridin]-2'(1'H)-on zugegeben und über Nacht bei RT gerührt. Das Reaktionsgemisch wurde auf Wasser gegossen. Der ausgefallene Niederschlag wurde abgesaugt, mit Wasser gewaschen und getrocknet.

| | |
|---|---|
| Ausbeute: | 370 mg (92% d. Th.) |
| ESI-MS: | m/z = 672 (M-H)⁻ |
| Rₜ (HPLC): | 1.40 min (Methode A) |

### Intermediat 39:

### 2-(3-(4-Fluorphenyl)-5-oxo-8,11-dioxa-1,4-diazaspiro[5.6]dodecan-4-yl)-N-((R)-2'-oxo-1,1',2',3-tetrahydrospiro[inden-2,3'-pyrrolo[2,3-b]pyridin]-5-yl)acetamid

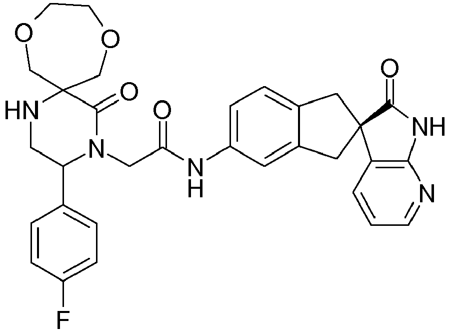

0.36 g (0.53 mmol) 2-(3-(4-Fluorphenyl)-5-oxo-8,11-dioxa-1,4-diazaspiro[5.6]dodecan-4-yl)-N-((*R*)-2'-oxo-1,1',2',3-tetrahydrospiro[inden-2,3'-pyrrolo[2,3-b]pyridin]-5-yl)acetamid in 5 ml Methanol wurden mit 10 ml einer 1.25 M methanolischen Salzsäure versetzt und 0.5 h bei RT und 3 h bei 50°C gerührt. Der Reaktionsansatz wurde einrotiert, mit Wasser versetzt und mit einer gesättigten NaHCO₃-Lösung neutralisiert. Der ausgefallene Niederschlag wurde abgesaugt, mit Wasser gewaschen und getrocknet.

| | |
|---|---|
| Ausbeute: | 220 mg (73% d. Th.) |
| ESI-MS: | m/z =572 (M+H)⁺ |
| Rₜ (HPLC): | 1.02 min (Methode A) |

### Intermediat 40:

### 3-(3-Fluorphenyl)-8,11-dioxa-1,4-diazaspiro[5.6]dodecan-5-on

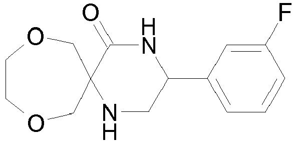

0.47 g (2.1 mmol) 1-(3-Fluorphenyl)ethan-1,2-diamin Dihydrochlorid wurden mit 23 mg (0.10 mmol) Benzyltriethylammoniumchlorid in 50 ml Dichlormethan vorgelegt und mit Eis/Aceton gekühlt. Anschließend wurden 0.71 ml (14 mmol) einer 50%igen Natronlauge zugegeben. Bei 0°C wurden 0.23 g (2 mmol) [1,4]Dioxepan-6-on zusammen mit 0.24 ml (4.6 mmol) Chloroform und 20 ml Dichlormethan zugetropft. Über Nacht ließ man den Ansatz auf RT erwärmen. Anschließend wurde der Reaktionsansatz auf 0°C abgekühlt und mit einer 6N wässrigen Salzsäure Lösung auf pH=1 eingestellt. Die Phasen wurden getrennt. Die wässrige Phase wurde mit einer 4N Natronlauge alkalisch gestellt und mit DCM extrahiert. Die vereinigten organischen Phasen wurden getrocknet und bis zur Trockne einrotiert.

| | |
|---|---|
| Ausbeute: | 250 mg (44% d. Th.) |
| ESI-MS: | m/z = 281 (M+H)⁺ |
| Rₜ (HPLC): | 0.62 min (Methode A) |

### Intermediat 41:

### tert-Butyl 3-(3-fluorphenyl)-5-oxo-8,11-dioxa-1,4-diazaspiro[5.6]dodecan-1-carboxylat

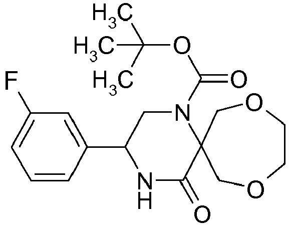

Zu 0.25 g (0.84 mmol) 3-(3-Fluorphenyl)-8,11-dioxa-1,4-diazaspiro[5.6]dodecan-5-on in 30 ml THF wurden 0.33 ml (1.9 mmol) DIPEA und 0.30 g (1.4 mmol) Di-*tert*-butyldicarbonat zugegeben. Über Nacht wurde derReaktionsansatz unter Rückfluss gekocht. Anschließend wurden weitere 0.30 g Di-*tert*-butyldicarbonat zugegeben und 12h unter Rückfluss gekocht. Anschließend wurden nochmals 0.30 g Di-*tert*-butyldicarbonat zugegeben und über Nacht unter Rückfluss gekocht. Der Reaktionsansatz wurde eingeengt. Der Rückstand wurde mit Diisopropylether verrieben. Der Feststoff wurde abgesaugt, mit Diisopropylether gewaschen und getrocknet.

| | |
|---|---|
| Ausbeute: | 210 mg (62% d. Th.) |
| ESI-MS: | m/z = 381 (M+H)⁺ |
| Rₜ (HPLC): | 1.274 min (Methode A) |

### Intermediat 42:

### tert-Butyl 3-(3-fluorphenyl)-4-(2-methoxy-2-oxoethyl)-5-oxo-8,11-dioxa-1,4-diazaspiro[5.6]-dodecan-1-carboxylat

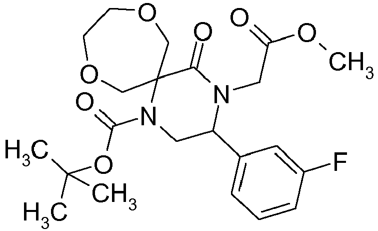

Zu 0.21 g (0.55 mmol) *tert*-Butyl 3-(3-fluorphenyl)-5-oxo-8,11-dioxa-1,4-diazaspiro[5.6]-dodecan-1-carboxylat in 20 ml DMF wurden 28 mg (0.66 mmol) Natriumhydrid zugegeben. Nach 20 min bei RT wurden bei 0°C 63 µl (0.65 mmol) Bromessigsäuremethylester zugetropft. Anschließend wurde 30 min im Eisbad und anschließend 5h bei RT gerührt. Der Reaktionsansatz wurde auf Wasser gegossen und mit Essigsäureethylester extrahiert. Die organische Phase wurde mit Wasser gewaschen getrocknet und eingeengt.

| | |
|---|---|
| Ausbeute: | 250 mg (quantitativ) |
| ESI-MS: | m/z = 453 (M+H)⁺ |
| Rₜ (HPLC): | 1.386 min (Methode A) |

### Intermediat 43:

### 2-(1-(tert-Butoxycarbonyl)-3-(3-fluorphenyl)-5-oxo-8,11-dioxa-1,4-diazaspiro[5.6]dodecan-4-yl)ethansäure

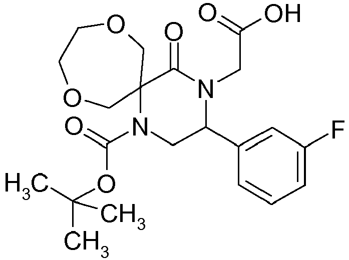

Zu 0.25 g (0.55 mmol) *tert*-Butyl 3-(3-fluorphenyl)-4-(2-methoxy-2-oxoethyl)-5-oxo-8,11-dioxa-1,4-diazaspiro[5.6]dodecan-1-carboxylat in 5 ml THF und 1 ml Wasser wurden 15 mg (0.62 mmol) Lithiumhydroxid zugegeben. Es wurde 2h bei RT gerührt und anschließend das MeOH abdestilliert. Der Rückstand wurde mit Wasser versetzt und mit Ameisensäure sauer gestellt. Die wässrige Phase wurde mit Essigsäureethylester extrahiert. Die Essigsäureethylester-Phase wurde getrocknet und eingeengt.

| | |
|---|---|
| Ausbeute: | 230 mg (quantitativ) |
| ESI-MS: | m/z = 439 (M+H)⁺ |
| Rₜ (HPLC): | 1.253 min (Methode A) |

### Intermediat 44:

### 3-Amino-5,7-dihvdrospiro[cyclopenta[b]pyridin-6,3'-pyrrolo[2,3-b]pyridin]-2'(1'H)-on

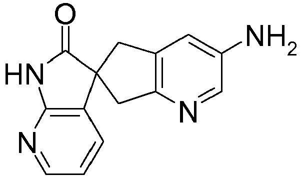

Diese Verbindung und deren Vorstufen wurden analog zu US 2007/265225 synthetisiert.

### Intermediat 45:

### (3R)-tert-Butyl 3-(3,5-difluorphenyl)-5-oxo-4-(2-oxo-2-(2'-oxo-1',2',5,7-tetrahydrospiro-[cyclopenta[b]pyridin-6,3'-pyrrolo[2,3-b]pyridin]-3-ylamino)ethyl)-8,11-dioxa-1,4-diazaspiro[5.6]dodecan-1-carboxylat

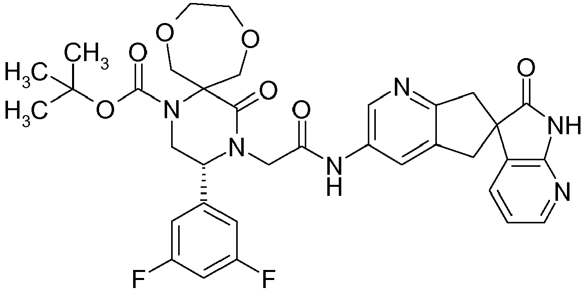

0.13 g (0.28 mmol) (*R*)-4-Carboxymethyl-3-(3,5-difluor-phenyl)-5-oxo-8,11-dioxa-1,4-diaza-spiro[5.6]dodecan-1-carbonsäure-*tert*-butylester in 2 ml DMF wurden mit 0.11 g (0.29 mmol) HATU und 0.08 ml (0.60 mmol) TEA versetzt und 3 min bei RT gerührt. Anschließend wurden 72 mg (0.28 mmol) 3-Amino-5,7-dihydrospiro-[cyclopenta-[b]pyridin-6,3'-pyrrolo[2,3-b]pyridin]-2'(1'H)-on zugegeben und über Nacht bei RT gerührt. Das Reaktionsgemisch wurde auf gesättigte Natriumhydrogencarbonat-Lösung gegossen. Der ausgefallene Niederschlag wurde abgesaugt, mit Wasser gewaschen und getrocknet.

| | |
|---|---|
| Ausbeute: | 180 mg (92% d. Th.) |
| ESI-MS: | m/z = 691 (M+H)⁺ |
| Rₜ (HPLC): | 1.30 min (Methode A) |

### Intermediat 46:

### (rac)-Benzylamino-(tetrahydro-pyran-4-yl)-essigsäureethylester

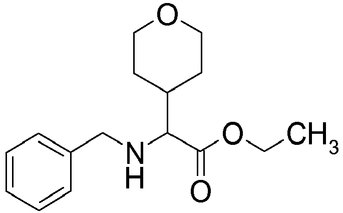

0.53 ml (4.5 mmol) Benzylbromid, 0.85 g (4.5 mmol) (*rac*)-Amino-(tetrahydropyran-4-yl)-essigsäureethylester und 1.9 g (13.7 mmol) Kaliumcarbonat in 10 mL DMF wurden 2 h bei 100°C gerührt. Nach dem Erkalten wurde der Ansatz mit Wasser versetzt und dreimal mit Ethylacetat extrahiert. Die vereinigten organischen Phasen wurden mit gesättigter, wässriger Kochsalz-Lösung, dreimal mit 0.5 M Salzsäure und abermals mit gesättigter Kochsalz-Lösung extrahiert. Die vereinigten wässrigen Phasen wurden mit konz. wässriger Ammoniak-Lösung auf pH 9 eingestellt und dreimal mit Dichlormethan extrahiert. Die vereinigten organischen Phasen wurden mit gesättigter Kochsalz-Lösung gewaschen, getrocknet und einrotiert.
- Ausbeute:: 0.39 g (31% d. Th.)
- ESI-MS:: m/z = 278 (M+H)⁺
- Rₜ (HPLC):: 0.813 min (Methode A)

### Intermediat 47:

### (rac)-E/Z-{Benzyl-[2-(3,5-difluor-phenyl)-2-hydroxyimino-ethyl]-amino}-(tetrahydro-pyran-4-yl)-essigsäureethylester

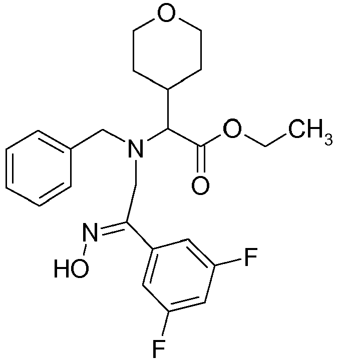

0.35 g (1.4 mmol) 2-Brom-1-(3,5-difluor-phenyl)-ethanon oxim, 0.39 g (1.4 mmol) (rac)-Benzylamino-(tetrahydro-pyran-4-yl)-essigsäureethylester und 0.25 g (1.8 mmol) Kalium-carbonat in 10 ml THF wurden über Nacht bei RT gerührt. Der Reaktionsansatz wurde mit Wasser verdünnt und viermal mit Ethylacetat extrahiert. Die vereinigten organischen Phasen wurden mit Wasser gewaschen, getrocknet, filtriert und einrotiert. Der Rückstand wurde mittels HPLC aufgereinigt. Die produktenthaltenden Fraktionen wurden vereinigt und das organische Lösungsmittel entfernt. Die wässrige Lösung wurde mit einer gesättigten Natriumhydrogencarbonat-Lösung alkalisch gestellt, zweimal mit Ethylacetat extrahiert, die organischen Phasen vereinigt, getrocknet, filtriert und einrotiert.

| | |
|---|---|
| Ausbeute: | 0.24 g (38% d. Th.) |
| ESI-MS: | m/z = 447 (M+H)⁺ |
| Rₜ (HPLC): | 1.64 / 1.68 min (Methode A) |

### Intermediat 48:

### 6-(3,5-Difluorphenyl)-3-(tetrahydro-2H-pyran-4-yl)piperazin-2-on

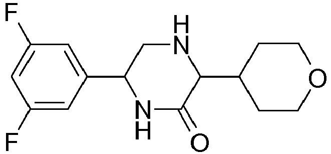

4.2 g (9.4 mmol) (*rac*)-E/Z-{Benzyl-[2-(3,5-difluor-phenyl)-2-hydroxyimino-ethyl]-amino}-(tetrahydro-pyran-4-yl)-essigsäureethylester in 100 ml Ethanol wurden unter einer Wasserstoffatmosphäre zusammen mit 0.80 g 10% Palladiumkohle 20h bei 50°C hydriert. Es wurde nochmals Katalysator hinzugefügt und bei 70°C hydriert. Der Katalysator wurde abgesaugt und das Lösemittel wurde eingeengt. Der Rückstand wurde in Ethanol gelöst, mit 2 g Natriumhydrogencarbonat versetzt und über Nacht unter Rückfluss gekocht. Nach dem Erkalten wurden die unlöslichen Bestandteile des Reaktionsansatzes abgesaugt. Das Filtrat wurde eingedampft. Der Rückstand wurde mittels flash Chromatographie gereinigt. Die Fraktionen wurden eingedampft und der Rückstand mit Diisopropylether verieben. Der Niederschlag wurde abgesaugt und getrocknet.

| | |
|---|---|
| Ausbeute: | 1.24 g (44% d. Th.) |
| ESI-MS: | m/z = 297 (M+H)⁺ |
| Rₜ (HPLC): | 0.476 / 0.635 min (Methode A) |

### Intermediat 49:

### tert-Butyl-5-(3,5-difluorphenyl)-3-oxo-2-(tetrahydro-2H-pyran-4-yl)piperazin-1-carboxylat

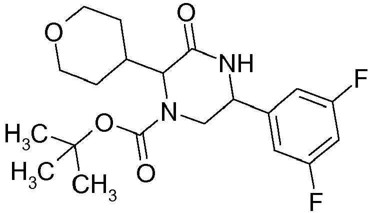

Zu 1.2 g (4.1 mmol) 6-(3,5-Difluorphenyl)-3-(tetrahydro-2H-pyran-4-yl)piperazin-2-on in 50 ml Acetonitril wurden 0.71 ml (4.1 mmol) DIPEA und 4.6 g (21.0 mmol) Di-*tert*-butyldicarbonat zugegeben. Die Reaktion wurde 2h bei 60°C gerührt. Der Reaktionsansatz wurde eingeengt. Der Rückstand wurde mittels flash-Chromatographie gereinigt.

| | |
|---|---|
| Ausbeute: | 0.3 g (19% d.Th.; Isomerengemisch 1) und 1.3 g (81% d.Th.; Isomerengemisch 2) |
| ESI-MS: | m/z = 397 (M+H)⁺ |
| Rₜ (HPLC): | 1.397/1.296 min (Methode A) |

### Intermediat 50a:

### tert-Butyl 5-(3,5-difluorphenyl)-4-(2-methoxy-2-oxoethyl)-3-oxo-2-(tetrahydro-2H-pyran-4-yl)-piperazin-1-carboxylat (Isomerengemisch 1.1)

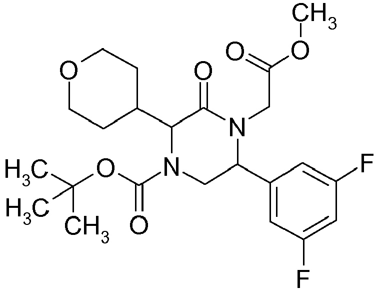

Bei 0°C wurden zu 0.30 g (0.76 mmol) *tert*-Butyl 5-(3,5-difluorphenyl)-3-oxo-2-(tetrahydro-2H-pyran-4-yl)piperazin-1-carboxylat (Isomerengemisch 1) in 10 ml DMF 38 mg (0.86 mmol) Natriumhydrid zugegeben. Nach 20 min unter Eisbadkühlung wurden 84 µl (0.86 mmol) Bromessigsäuremethylester zugetropft. Anschließend wurde 30 min im Eisbad und 5h bei RT gerührt. Der Reaktionsansatz wurde auf Wasser gegossen und mit Essigsäureethylester extrahiert. Die organische Phase wurde mit Wasser gewaschen, getrocknet und eingeengt.

| | |
|---|---|
| Ausbeute: | 310 mg (87% d.Th.) |
| ESI-MS: | m/z = 469 (M+H)⁺ |
| Rₜ (HPLC): | 1.521 min (Methode A) |

### Intermediat 50b:

### tert-Butyl 5-(3,5-difluorphenyl)-4-(2-methoxy-2-oxoethyl)-3-oxo-2-(tetrahydro-2H-pyran-4-yl)-piperazin-1-carboxylat (Isomerengemisch 2.1)

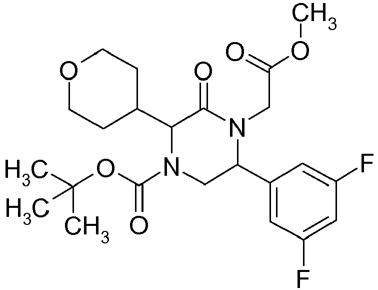

Bei 0°C wurde zu 1.30 g (3.3 mmol) *tert*-Butyl 5-(3,5-difluorphenyl)-3-oxo-2-(tetrahydro-2H-pyran-4-yl)piperazin-1-carboxylat (Isomerengemisch 2) in 20 ml DMF 0.16 g (3.7 mmol) Natriumhydrid zugegeben. Nach 20 min unter Eisbadkühlung wurden 0.36 ml (3.7 mmol) Bromessigsäuremethylester zugetropft. Anschließend wurde 30 min im Eisbad und 5h bei RT gerührt. Der Reaktionsansatz wurde auf Wasser gegossen und mit Essigsäureethylester extrahiert. Die organische Phase wurde mit Wasser gewaschen, getrocknet und eingeengt.

| | |
|---|---|
| Ausbeute: | 0.87 g (57% d.Th.) |
| ESI-MS: | m/z = 469 (M+H)⁺ |
| Rₜ (HPLC): | 1.485 min (Methode A) |

### Intermediat 51a:

### 2-(4-(tert-Butoxycarbonyl)-6-(3,5-difluorphenyl)-2-oxo-3-(tetrahydro-2H-pyran-4-yl)-piperazin-1-yl)ethansäure (Isomerengemisch 1.1.1)

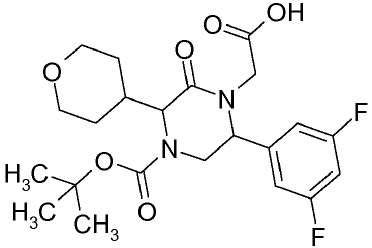

Zu 0.20 g (0.43 mmol) *tert*-Butyl 5-(3,5-difluorphenyl)-4-(2-methoxy-2-oxoethyl)-3-oxo-2-(tetrahydro-2H-pyran-4-yl)piperazin-1-carboxylat (Isomerengemisch 1.1) in 5 ml THF und 1 ml Wasser wurden 12 mg (0.48 mmol) Lithiumhydroxid zugegeben. Es wurde 2h bei RT gerührt und anschließend zur Trockne eingeengt.

| | |
|---|---|
| Ausbeute: | 200 mg (quantitativ) |
| ESI-MS: | m/z = 455 (M+H)⁺ |
| Rₜ (HPLC): | 1.361 min (Methode A) |

### Intermediat 51b:

### 2-(4-(tert-Butoxycarbonyl)-6-(3,5-difluorphenyl)-2-oxo-3-(tetrahydro-2H-pyran-4-yl)-piperazin-1-yl)ethansäure (Isomerengemisch 2.1.1)

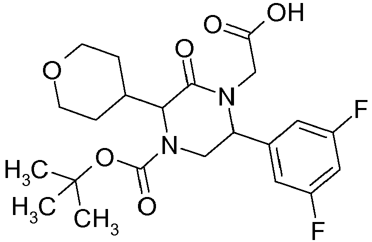

Zu 0.20 g (0.43 mmol) *tert*-Butyl 5-(3,5-difluorphenyl)-4-(2-methoxy-2-oxoethyl)-3-oxo-2-(tetrahydro-2H-pyran-4-yl)piperazin-1-carboxylat (Isomerengemisch 2.1) in 5 ml THF und 1 ml Wasser wurden 12 mg (0.48 mmol) Lithiumhydroxid zugegeben. Es wurde 2h bei RT gerührt, mit 0.1 M Salzsäure-Lösung auf pH=7 eingestellt und anschließend zur Trockne eingeengt.

| | |
|---|---|
| Ausbeute: | 200 mg (quantitativ) |
| ESI-MS: | m/z = 455 (M+H)⁺ |
| Rₜ (HPLC): | 1.291 min (Methode A) |

### Intermediat 52:

### (R)-Methyl 2-(3-(3,5-difluorphenyl)-5-oxo-8,11-dioxa-1,4-diazaspiro[5.6]dodecan-4-yl)-acetat Hydrochlorid

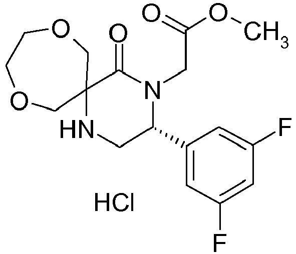

0.13 g (0.28 mmol) (*R*)-3-(3,5-Difluor-phenyl)-4-methoxycarbonylmethyl-5-oxo-8,11-dioxa-1,4-diaza-spiro[5.6]dodecan-1-carbonsäure-*tert*-butylester wurden mit 20 ml einer 1 M methanolischer Salzsäure Lösung versetzt und 2h bei 50°C gerührt. Der Reaktionsansatz wurde einrotiert und ohne weitere Aufreinung weiter umgesetzt.

| | |
|---|---|
| Ausbeute: | 100 mg (89% d. Th.) |
| Rₜ (HPLC): | 0.99 min (Methode A) |

### Intermediat 53:

### (R)-Methyl 2-(3-(3,5-difluorphenyl)-1-methyl-5-oxo-8,11-dioxa-1,4-diazaspiro[5.6]dodecan-4-yl)acetat

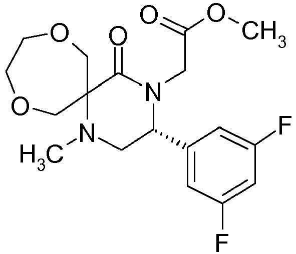

0.10 g (0.25 mmol) (R)-Methyl 2-(3-(3,5-difluorphenyl)-5-oxo-8,11-dioxa-1,4-diazaspiro-[5.6]dodecan-4-yl)acetat Hydrochlorid in 20 ml Acetonitril wurden mit 83 mg (0.60 mmol) Kaliumcarbonat und mit 0.04 ml (0.60 mmol) Methyliodid versetzt. Der Reaktionsansatz wurde 5 Tage bei RT gerührt. Anschließend wurde die Reaktion eingeengt und ohne weitere Aufreinigung umgesetzt.

| | |
|---|---|
| Ausbeute: | 75 mg (79% d. Th.) |
| Rₜ (HPLC): | 1.20 min (Methode A) |

### Intermediat 54:

### (R)-2-(3-(3,5-Difluorphenyl)-1-methyl-5-oxo-8,11-dioxa-1,4-diazaspiro[5.6]dodecan-4-yl)-ethansäure

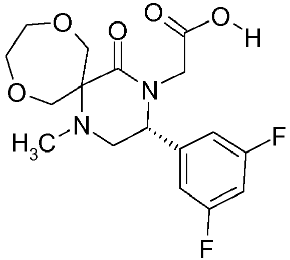

Zu 75 mg (0.20 mmol) (R)-Methyl 2-(3-(3,5-difluorphenyl)-1-methyl-5-oxo-8,11-dioxa-1,4-diazaspiro[5.6]dodecan-4-yl)acetat in 5 ml THF und 1 ml Wasser wurden 7.2 mg (0.30 mmol) Lithiumhydroxid zugegeben. Der Reaktionsansatz wurde 2h bei RT gerührt und anschließend MeOH abdestilliert. Der Rückstand wurde mit einer 0.1 M Salzäure Lösung neutral gestellt und zur Trockne eingeengt.

| | |
|---|---|
| Ausbeute: | 75 mg (quantitativ) |
| Rₜ (HPLC): | 1.0 min (Methode A) |

### Intermediat 55:

### (R)-3-Cycloheptylmethyl-6-(3,5-difluor-phenyl)-piperazin-2-on

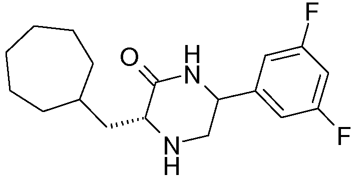

Unter einer Wasserstoffatmosphäre wurden 5.5 g (12.0 mmol) (*R*)-2-(Benzyl-{2-(3,5-difluor-phenyl)-2-[hydroxyimino]-ethyl}-amino)-3-cycloheptyl-propionsäure-methylester (diese Verbindung kann analog der hierin beschriebenen Synthese von (*S*)-2-(Benzyl-{2-(3,5-difluor-phenyl)-2-[hydroxyimino]-ethyl}-amino)-3-cycloheptyl-propionsäure-methylester unter Verwendung der entsprechenden (R)-konfigurierten Ausgangskomponente erhalten werden) und 0.70 g Palladium auf Kohle (10%) in 30 ml Methanol für 3 Tage bei 50°C bis 60°C und 3 bar Wasserstoffdruck hydriert. Nach dem Entfernen des Katalysators wurde das Filtrat einrotiert. Die Aufreinigung erfolgte mittels flash Chromatographie.

| | |
|---|---|
| Ausbeute: | 1.3 g (34% d. Th.; Isomer 3) und 1.4 g (36% d. Th.; Isomer 4) |
| ESI-MS: | m/z = 323 (M+H)⁺ |
| Rₜ (HPLC): | 1.004 min / 1.076 min (Methode A) |

### Intermediat 56a:

### (2R)-tert-Butyl-2-(cycloheptylmethyl)-5-(3,5-difluorphenyl)-3-oxopiperazin-1-carboxylat (Isomer 3.1)

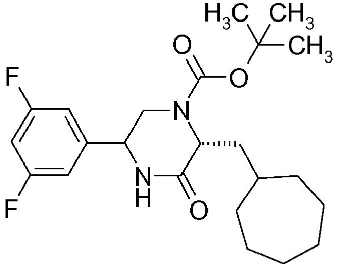

1.3 g (4.0 mmol) (3R)-3-(Cycloheptylmethyl)-6-(3,5-difluorphenyl)piperazin-2-on (Isomer 3), 0.89 g (4.1 mmol) Di-*tert-*Butyldicarbonat und 0.70 ml (4.1 mmol) DIPEA wurden in 20 ml Acetonitril über Nacht bei RT gerührt. Der Reaktionsansatz wurde eingeengt und der Rückstand zwischen Wasser und Essigsäureethylester verteilt. Die wässrige Phase wurde mit Essigsäureethylester extrahiert. Die organischen Phasen wurden vereinigt, getrocknet und eingeengt.

| | |
|---|---|
| Ausbeute: | 1.7 g (quantitativ) |
| ESI-MS: | m/z = 421 (M-H)⁻ |
| Rₜ (HPLC): | 1.791 min (Methode A) |

### Intermediat 56b:

### (2R)-tert-Butyl-2-(cycloheptylmethyl)-5-(3,5-difluorphenyl)-3-oxopiperazin-1-carboxylat (Isomer 4.1)

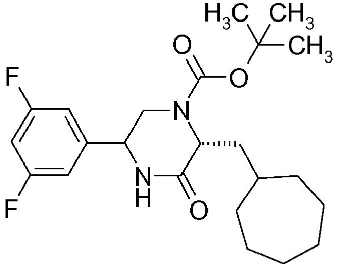

1.3 g (4.0 mmol) (3*R*)-3-(Cycloheptylmethyl)-6-(3,5-difluorphenyl)piperazin-2-on (Isomer 4), 0.89 g (4.1 mmol) Di-*tert*-Butyldicarbonat und 0.70 ml (4.1 mmol) DIPEA wurden in 20 ml Acetonitril über Nacht bei RT gerührt. Der Reaktionsansatz wurde eingeengt und der Rückstand zwischen Wasser und Essigsäureethylester verteilt. Die wässrige Phase wurde mit Essigsäureethylester extrahiert. Die organischen Phasen wurden vereinigt, getrocknet und eingeengt.

| | |
|---|---|
| Ausbeute: | 1.6 g (94 % d.Th.)) |
| ESI-MS: | m/z = 423 (M+H)⁺ |
| Rₜ (HPLC): | 1.905 min (Methode A) |

### Intermediat 57a:

### (2R)-tert-Butyl-2-(cycloheptylmethyl)-5-(3,5-difluorphenyl)-4-(2-methoxy-2-oxoethyl)-3-oxopiperazin-1-carboxylat (Isomer 3.1.1)

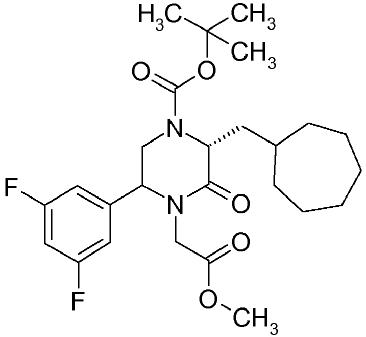

Unter einer Stickstoffatmosphäre wurden bei 0°C zu 0.50 g (1.2 mmol) (2*R*)-*tert*-Butyl-2-(cycloheptylmethyl)-5-(3,5-difluorphenyl)-3-oxopiperazin-1-carboxylat (Isomer 3.1) in 20 ml DMF 59 mg (1.4 mmol) Natriumhydrid zugegeben. Nach 20 min bei 0°C wurden 0.13 ml (1.4 mmol) Bromessigsäuremethylester zugegeben und 30 min bei 0°C gerührt. Anschließend wurde der Reaktionsansatz 5h bei RT gerührt. Der Reaktionsansatz wurde mit Wasser versetzt und zweimal mit Essigsäureethylester extrahiert. Die vereinigten organischen Phasen wurden getrocknet und eingeengt.

| | |
|---|---|
| Ausbeute: | 0.53 g (91 % d.Th) |
| ESI-MS: | m/z = 495 (M+H)⁺ |
| Rₜ (HPLC): | 1.923 min (Methode A) |

### Intermediat 57b:

### (2R)-tert-Butyl-2-(cycloheptylmethyl)-5-(3,5-difluorphenyl)-4-(2-methoxy-2-oxoethyl)-3-oxopiperazin-1-carboxylat (Isomer 4.1.1)

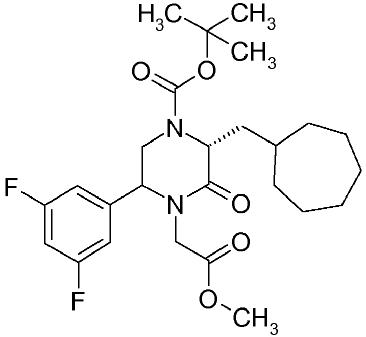

Unter einer Stickstoffatmosphäre wurden bei 0°C zu 0.50 g (1.2 mmol) (2*R*)-*tert*-Butyl-2-(cycloheptylmethyl)-5-(3,5-difluorphenyl)-3-oxopiperazin-1-carboxylat (Isomer 4.1) in 20 ml DMF 59 mg (1.4 mmol) Natriumhydrid zugegeben. Nach 20 min bei 0°C wurden 0.13 ml (1.4 mmol) Bromessigsäuremethylester zugegeben und 30 min bei 0°C gerührt. Anschließend wurde der Reaktionsansatz 5h bei RT gerührt. Der Reaktionsansatz wurde mit Wasser versetzt und zweimal mit Essigsäureethylester extrahiert. Die vereinigten organischen Phasen wurden getrocknet und eingeengt.

| | |
|---|---|
| Ausbeute: | 0.55 g (94% d.Th) |
| ESI-MS: | m/z = 495 (M+H)⁺ |
| Rₜ (HPLC): | 1.967 min (Methode A) |

### Intermediat 58a:

### 2-((3R)-4-(tert-butoxycarbonyl)-3-(cycloheptylmethyl)-6-(3,5-difluorphenyl)-2-oxopiperazin-1-yl)ethansäure (Isomer 3.1.1.1)

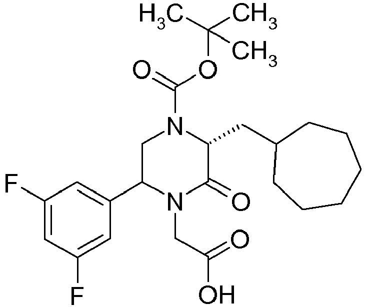

Zu 0.53 g (1.1 mmol) (2*R*)-*tert*-Butyl-2-(cycloheptylmethyl)-5-(3,5-difluorphenyl)-4-(2-methoxy-2-oxoethyl)-3-oxopiperazin-1-carboxylat (Isomer 3.1.1) in 5 ml THF wurden 29 mg (1.2 mmol) Lithiumhydroxid in 1.0 ml Wasser zugegeben. Nach 2 h bei RT wurde mit 0.1 M Salzsäure Lösung neutralisiert. Der Reaktionsansatz wurde bis zur Trockne einrotiert.

| | |
|---|---|
| Ausbeute: | 0.53 g (quantitativ) |
| Rₜ (HPLC): | 1.744 min (Methode A) |

### Intermediat 58b:

### 2-((3R)-4-(tert-butoxycarbonyl)-3-(cycloheptylmethyl)-6-(3,5-difluorphenyl)-2-oxopiperazin-1-yl)ethansäure (Isomer 4.1.1.1)

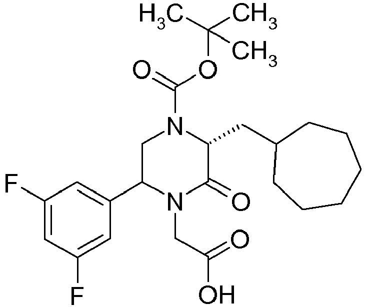

Zu 0.55 g (1.1 mmol) (2*R*)-*tert*-Butyl-2-(cycloheptylmethyl)-5-(3,5-difluorphenyl)-4-(2-methoxy-2-oxoethyl)-3-oxopiperazin-1-carboxylat (Isomer 4.1.1) in 5 ml THF wurden 24 mg (1.3 mmol) Lithiumhydroxid in 1.0 ml Wasser zugegeben. Nach 2 h bei RT wurde mit 0.1 M Salzsäure Lösung neutralisiert. Der Reaktionsansatz wurde bis zur Trockne einrotiert.

| | |
|---|---|
| Ausbeute: | 0.52 g (97% d.Th.)) |
| Rₜ (HPLC): | 1.861 min (Methode A) |

### Herstellung der Endverbindungen

### Beispiel 1a:

### 2-((R)-3-(3,5-Difluorphenyl)-5-oxo-8,11-dioxa-1,4-diazaspiro[5.6]dodecan-4-yl)-N-((R)-2'-oxo-1,1',2',3-tetrahydrospiro[inden-2,3'-pyrrolo[2,3-b]pyridin]-5-yl)acetamid

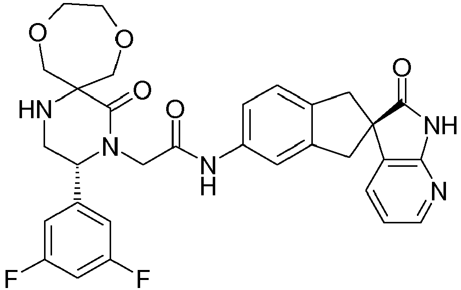

0.114 g (0.25 mmol) (*R*)-4-Carboxymethyl-3-(3,5-difluor-phenyl)-5-oxo-8,11-dioxa-1,4-diaza-spiro[5.6]dodecan-1-carbonsäure-*tert*-butylester wurden zusammen mit 67.9 mg (0.27 mmol) (*R*)-5-Amino-1,3-dihydrospiro[inden-2,3'-pyrrolo[2,3-b]pyridin]-2'(1'H)-on, 63.3 mg (0.33 mmol) 1-Ethyl-3-(3-dimethylaminopropyl)carbodiimid-Hydrochlorid, 44.6 mg (0.33 mmol) HOBt, 35 µl (0.25 mmol) TEA und 1.5 ml DMF über Nacht bei RT gerührt. Das Lösungsmittel wurde abrotiert und der Rückstand mit 15 ml einer methanolischen Salzsäure (1.25N) 1 h bei 50°C gerührt. Der Reaktionsansatz wurde einrotiert und der Rückstand mittels PR-HPLC aufgereinigt. Die produktenthaltenden Fraktionen wurden einrotiert und der Rückstand mit Diisopropylether verrieben. Nach dem Absaugen wurde der Feststoff mit Diisopropylether nachgewaschen und getrocknet.

| | |
|---|---|
| Ausbeute: | 65 mg (42% d. Th.) |
| ESI-MS: | m/z =590 (M+H)⁺ |
| Rₜ (HPLC): | 1.10 min (Methode A) |

### Beispiel 1b:

### 2-((R)-3-(3,5-Difluorphenyl)-5-oxo-8,11-dioxa-1,4-diazaspiro[5.6]dodecan-4-yl)-N-((S)-2'-oxo-1,1',2',3-tetrahydrospiro[inden-2,3'-pyrrolo[2,3-b]pyridin]-5-yl)acetamid

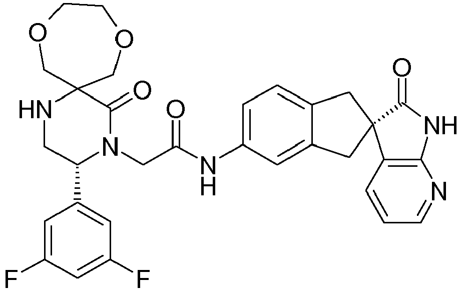

0.114 g (0.25 mmol) (*R*)-4-Carboxymethyl-3-(3,5-difluor-phenyl)-5-oxo-8,11-dioxa-1,4-diaza-spiro[5.6]dodecan-1-carbonsäure-*tert*-butylester wurden mit 67.9 mg (0.27 mmol) (*S*)-5-Amino-1,3-dihydrospiro[inden-2,3'-pyrrolo[2,3-b]pyridin]-2'(1'H)-on, 63.3 mg (0.33 mmol) 1-Ethyl-3-(3-dimethylaminopropyl)carbodiimid Hydrochlorid, 44.6 mg (0.33 mmol) HOBT, 35 µl (0.25mmol) TEA und 1.5 ml DMF über Nacht bei RT gerührt. Das Lösungsmittel wurde abrotiert und der Rückstand mit 15 ml einer methanolischen Salzsäure (1.25N) 1 h bei 50°C gerührt. Der Reaktionsansatz wurde einrotiert und der Rückstand mittels PR-HPLC aufgereinigt. Die produktenthaltenden Fraktionen wurden einrotiert und der Rückstand mit Diisopropylether verrieben. Nach dem Absaugen wurde der Feststoff mit Diisopropylether nachgewaschen und getrocknet.

| | |
|---|---|
| Ausbeute: | 50 mg (32% d. Th.) |
| ESI-MS: | m/z =590 (M+H)⁺ |
| Rₜ (HPLC): | 1.10 min (Methode A) |

### Beispiel 13:

### 2-((3S)-3-(Cycloheptylmethyl)-6-(3,5-difluorphenyl)-2-oxopiperazin-1-yl)-N-((R)-2'-oxo-1,1',2',3-tetrahydrospiro[inden-2,3'-pyrrol[2,3-b]pyridin]-5-yl)acetamid

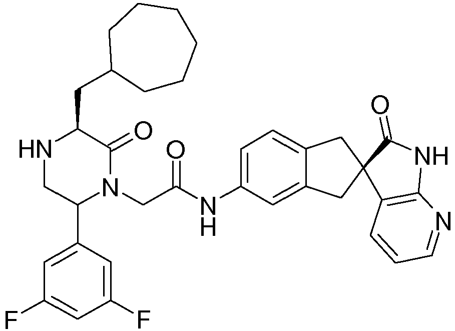

100 mg (0.21 mmol) 2-((3*S*)-4-(*tert*-butoxycarbonyl)-3-(cycloheptylmethyl)-6-(3,5-difluorphenyl)-2-oxopiperazin-1-yl)ethansäure, 58 mg (0.23 mmol) (*R*)-5-amino-1,3-dihydro-spiro[inden-2,3'-pyrrol[2,3-b]pyridin]-2'(1'H)-on, 56 mg (0.29 mmol) 1-Ethyl-3-(3-dimethyl-aminopropyl)carbodiimid-Hydrochlorid, 39 mg (0.29 mmol) HOBT, 0.03 ml (0.23 mmol) TEA und 1.50 ml DMF wurden über Nacht bei RT gerührt. Der Ansatz wurde einrotiert. Der Rückstand wurde mit 15 ml einer 1.25M methanolischer Salzsäure versetzt und 2 h bei 50°C gerührt. Die Diastereomere wurden mittels RP-HPLC getrennt. Die das Produkt enthaltenden Fraktionen wurden lyophilisiert.

**Diastereomer 1:**

| | |
|---|---|
| Ausbeute: | 16 mg (12% d. Th.) |
| ESI-MS: | m/z = 614 (M+H)⁺ |
| Rₜ (HPLC): | 1.13 min (Methode B) |

**Diastereomer 2:**

| | |
|---|---|
| Ausbeute: | 10 mg (7% d. Th.) |
| ESI-MS: | m/z = 614 (M+H)⁺ |
| Rₜ (HPLC): | 1.19 min (Methode B) |

### Beispiel 14:

### 2-((R)-3-(3,5-Difluoro-phenyl)-5-oxo-12-oxa-1,4-diaza-dispiro[5.2.5.2]hexadecan-4-yl)-N-((R)-2'-oxo-1,1',2',3-tetrahydrospiro[inden-2,3'-pyrrolo[2,3-b]pyridin]-5-yl)acetamid

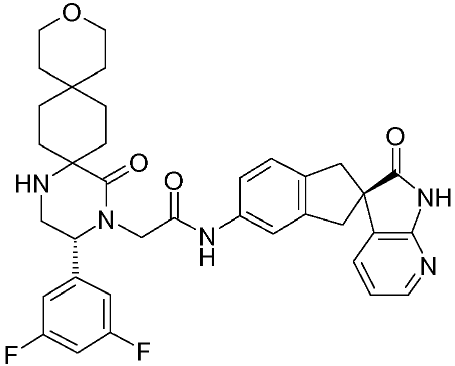

75 mg (0.15 mmol) 4-Carboxymethyl-(3R)-(3,5-difluorphenyl)-5-oxo-12-oxa-1,4-diaza-dispiro[5.2.5.2]hexadecan-1-carbonsäure-*tert*-butylester, 40 mg (0.16 mmol) (*R*)-5-Amino-1,3-dihydrospiro[inden-2,3'-pyrrol[2,3-b]pyridin]-2'(1'H)-on, 38 mg (0.20 mmol) 1-Ethyl-3-(3-dimethylaminopropyl)carbodiimid-Hydrochlorid, 27 mg (0.20 mmol) HOBT, 0.02 ml (0.16 mmol) TEA und 1.50 ml DMF wurden bei RT über Nacht gerührt. Der Reaktionsansatz wurde einrotiert. Der Rückstand wurde mit 15 ml einer 1.25M methanolischer Salzsäure 1 h bei 50°C gerührt. Der Ansatz wurde einrotiert und der Rückstand mittels RP-HPLC gereinigt. Die produktenthaltenden Fraktionen wurden vereinigt und lyophilisiert.

| | |
|---|---|
| Ausbeute: | 59 mg (62% d. Th.) |
| ESI-MS: | m/z = 642 (M+H)⁺ |
| Rₜ (HPLC): | 1.13 min (Methode A) |

### Beispiel 15a:

### 2-(6-(3,5-Difluorphenyl)-2-oxo-3-(tetrahydro-2H-pyran-4-yl)piperazin-1-yl)-N-((R)-2'-oxo-1,1',2',3-tetrahydrospiro[inden-2,3'-pyrrolo[2,3-b]pyridin]-5-yl)acetamid

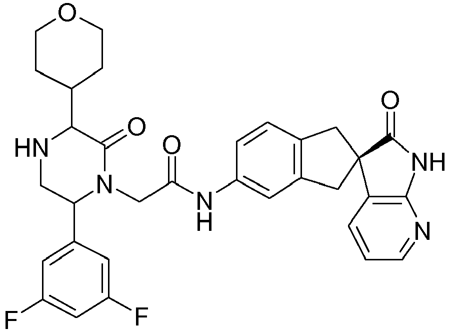

0.10 g (0.22 mmol) 2-(4-(*tert*-Butoxycarbonyl)-6-(3,5-difluorphenyl)-2-oxo-3-(tetrahydro-2H-pyran-4-yl)piperazin-1-yl)ethansäure (Isomerengemisch 1.1.1), 60 mg (0.24 mmol) (*R*)-5-Amino-1,3-dihydrospiro[inden-2,3'-pyrrolo[2,3-b]pyridin]-2'(1'H)-on, 58 mg (0.30 mmol) 1-Ethyl-3-(3-dimethylaminopropyl)carbodiimid-Hydrochlorid, 41 mg (0.30 mmol) HOBT, 0.03 ml (0.24 mmol) TEA und 1.5 ml DMF wurden bei RT über Nacht gerührt. Der Reaktionsansatz wurde einrotiert. Der Rückstand wurde mit 15 ml einer 1.25M methanolischer Salzsäure Lösung 1h bei 50°C gerührt. Der Ansatz wurde einrotiert und der Rückstand mittels HPLC gereinigt. Die produktenthaltenden Fraktionen wurden vereinigt und lyophilisiert. Das Produkt lag als Isomerengemisch vor.

| | |
|---|---|
| Ausbeute: | 52 mg (40% d. Th.) |
| ESI-MS: | m/z = 588 (M+H)⁺ |
| Rₜ (HPLC): | 1.067 min (Methode A) |

### Beispiel 15b:

### 2-(6-(3,5-Difluorphenyl)-2-oxo-3-(tetrahydro-2H-pyran-4-yl)piperazin-1-yl)-N-((R)-2'-oxo-1,1',2',3-tetrahydrospiro[inden-2,3'-pyrrolo[2,3-b]pyridin]-5-yl)acetamid

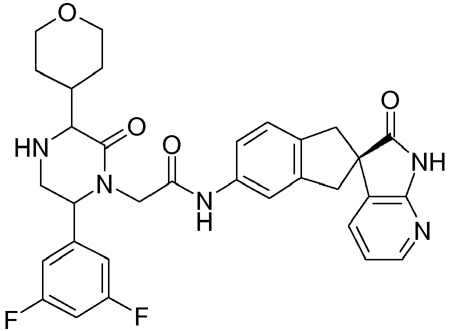

0.10 g (0.22 mmol) 2-(4-(*tert*-Butoxycarbonyl)-6-(3,5-difluorphenyl)-2-oxo-3-(tetrahydro-2H-pyran-4-yl)piperazin-1-yl)ethansäure (Isomerengemisch 2.2.2), 60 mg (0.24 mmol) (*R*)-5-Amino-1,3-dihydrospiro[inden-2,3'-pyrrolo[2,3-b]pyridin]-2'(1'H)-on, 58 mg (0.30 mmol) 1-Ethyl-3-(3-dimethylaminopropyl)carbodümid-Hydrochlorid, 41 mg (0.30 mmol) HOBT, 0.03 ml (0.24 mmol) TEA und 1.5 ml DMF wurden bei RT über Nacht gerührt. Der Reaktionsansatz wurde einrotiert. Der Rückstand wurde mit 15 ml einer 1.25M methanolischer Salzsäure Lösung 1h bei 50°C gerührt. Der Ansatz wurde einrotiert und der Rückstand mittels HPLC gereinigt. Die produktenthaltenden Fraktionen wurden vereinigt und lyophilisiert. Das Produkt lag als Isomergengemisch vor.

| | |
|---|---|
| Ausbeute: | 42 mg (33% d. Th.) |
| ESI-MS: | m/z = 588 (M+H)⁺ |
| Rₜ (HPLC): | 1.025 min (Methode A) |

### Beispiel 16a:

### 2-((3R)-3-(Cycloheptylmethyl)-6-(3,5-difluorphenyl)-2-oxopiperazin-1-yl)-N-((R)-2'-oxo-1,1',2',3-tetrahydrospiro[inden-2,3'-pyrrolo[2,3-b]pyridin]-5-yl)acetamid

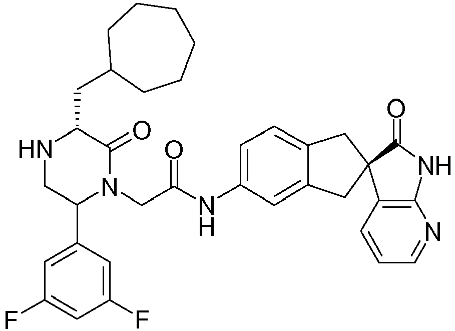

0.20 g (0.42 mmol) 2-((3*R*)-4-(*tert*-Butoxycarbonyl)-3-(cycloheptylmethyl)-6-(3,5-difluorphenyl)-2-oxopiperazin-1-yl)ethansäure (Isomer 3.1.1.1), 0.11 g (0.45 mmol) (*R*)-5-Amino-1,3-dihydrospiro[inden-2,3'-pyrrolo[2,3-b]pyridin]-2'(1'H)-on, 0.11 g (0.55 mmol) 1-Ethyl-3-(3-dimethylaminopropyl)-carbodiimid Hydrochlorid, 74 mg (0.55 mmol) HOBT, 0.06 ml (0.45 mmol) TEA und 2.0 ml DMF wurden bei RT über Nacht gerührt. Der Reaktionsansatz wurde einrotiert. Der Rückstand wurde mit 15 ml einer 1.25M methanolischer Salzsäure 2 h bei 50°C gerührt. Der Ansatz wurde einrotiert und der Rückstand mittels HPLC gereinigt. Die produktenthaltenden Fraktionen wurden vereinigt und lyophilisiert.

| | |
|---|---|
| Ausbeute: | 80 mg (31% d. Th.) |
| ESI-MS: | m/z = 614 (M+H)⁺ |
| Rₜ (HPLC): | 1.278 min (Methode A) |

### Beispiel 16b:

### 2-((3R)-3-(Cycloheptylmethyl)-6-(3,5-difluorphenyl)-2-oxopiperazin-1-yl)-N-((R)-2'-oxo-1,1',2',3-tetrahydrospiro[inden-2,3'-pyrrolo[2,3-b]pyridin]-5-yl)acetamid

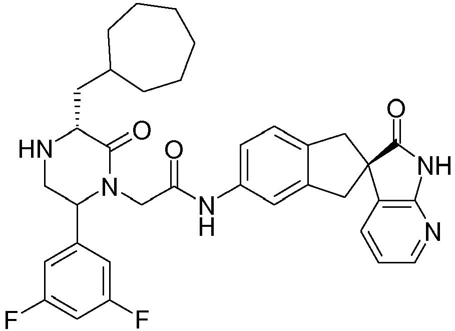

0.20 g (0.42 mmol) 2-((3*R*)-4-(*tert*-Butoxycarbonyl)-3-(cycloheptylmethyl)-6-(3,5-difluorphenyl)-2-oxopiperazin-1-yl)ethansäure (Isomer 4.1.1.1), 0.11 g (0.45 mmol) (*R*)-5-Amino-1,3-dihydrospiro[inden-2,3'-pyrrolo[2,3-b]pyridin]-2'(1'H)-on, 0.11 g (0.55 mmol) 1-Ethyl-3-(3-dimethylaminopropyl)-carbodiimid Hydrochlorid, 74 mg (0.55 mmol) HOBT, 0.06 ml (0.45 mmol) TEA und 2.0 ml DMF wurden bei RT über Nacht gerührt. Der Reaktionsansatz wurde einrotiert. Der Rückstand wurde mit 15 ml einer 1.25M methanolischer Salzsäure 1 h bei 50°C gerührt. Der Ansatz wurde einrotiert und der Rückstand mittels HPLC gereinigt. Die produktenthaltenden Fraktionen wurden vereinigt und lyophilisiert.

| | |
|---|---|
| Ausbeute: | 41 mg (16% d. Th.) |
| ESI-MS: | m/z = 614 (M+H)⁺ |
| Rₜ (HPLC): | 1.313 min (Methode A) |

### Beispiel 17:

### 2-((R)-3-(3,5-Difluorphenyl)-1-methyl-5-oxo-8,11-dioxa-1,4-diazaspiro[5.6]dodecan-4-yl)-N-((R)-2'-oxo-1,1',2',3-tetrahydrospiro[inden-2,3'-pyrrolo[2,3-b]pyridin]-5-yl)acetamid

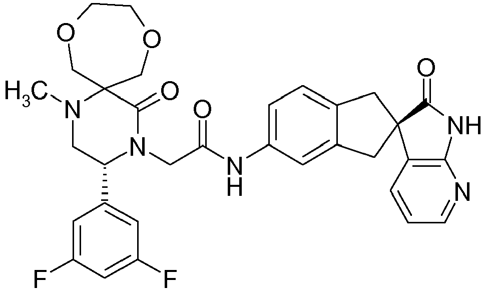

75 mg (0.20 mmol) (*R*)-2-(3-(3,5-Difluorphenyl)-1-methyl-5-oxo-8,11-dioxa-1,4-diazaspiro-[5.6]dodecan-4-yl)-ethansäure, 55 mg (0.22 mmol) (*R*)-5-Amino-1,3-dihydrospiro[inden-2,3'-pyrrolo[2,3-b]pyridin]-2'(1'H)-on, 50 mg (0.26 mmol) 1-Ethyl-3-(3-dimethylamino-propyl)carbodiimid-Hydrochlorid, 35 mg (0.26 mmol) HOBT, 0.07 ml (0.50 mmol) TEA und 1.5 ml DMF wurden bei RT über Nacht gerührt. Der Reaktionsansatz wurde einrotiert. Der Rückstand wurde mit 15 ml einer 1.25M methanolischer Salzsäure 1 h bei 50°C gerührt. Der Ansatz wurde einrotiert und der Rückstand mittels HPLC gereinigt. Die das Produkt enthaltenden Fraktionen wurden vereinigt und lyophilisiert.

| | |
|---|---|
| Ausbeute: | 16 mg (13% d. Th.) |
| ESI-MS: | m/z = 604 (M+H)⁺ |
| Rₜ (HPLC): | 1.20 min (Methode A) |

### Beispiel 18:

### 2-(3-(3-Methoxyphenyl)-5-oxo-8,11-dioxa-1,4-diazaspiro[5.6]dodecan-4-yl)-N-((R)-2'-oxo-1',2',3-tetrahydrospiro[inden-2,3'-pyrrolo[2,3-b]pyridin]-5-yl)acetamid

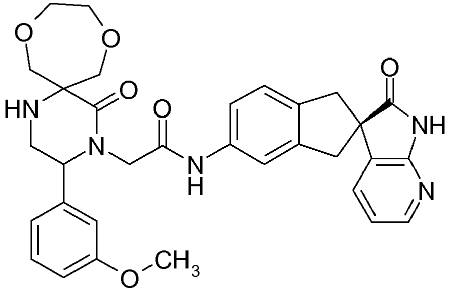

0.60 g (0.79 mmol) *tert*-Butyl 3-(3-methoxyphenyl)-5-oxo-4-(2-oxo-2-((*R*)-2'-oxo-1,1',2',3-tetrahydrospiro[inden-2,3'-pyrrolo[2,3-b]pyridin]-5-ylamino)ethyl)-8,11-dioxa-1,4-diaza-spiro[5.6]dodecan-1-carboxylat in 5 ml Methanol wurden mit 15 ml einer 1.25 M methanolischer Salzsäure versetzt und 3 h bei 50°C gerührt. Der Reaktionsansatz wurde einrotiert und mit gesättigter NaHCO₃-Lösung neutralisiert. Der ausgefallene Niederschlag wurde abgesaugt, mit Wasser gewaschen und getrocknet.

| | |
|---|---|
| Ausbeute: | 250 mg (54% d. Th.) |
| ESI-MS: | m/z =584 (M+H)⁺ |
| Rₜ (HPLC): | 1.02 min (Methode A) |

### Beispiel 19:

### N-(5-Chlor-2'-oxo-1,1',2',3-tetrahydrospiro[inden-2,3'-pyrrolo[2,3-b]pyridin]-6-yl)-2-((R)-3-(3,5-difluorphenyl)-5-oxo-8,11-dioxa-1,4-diazaspiro[5.6]dodecan-4-yl)acetamid

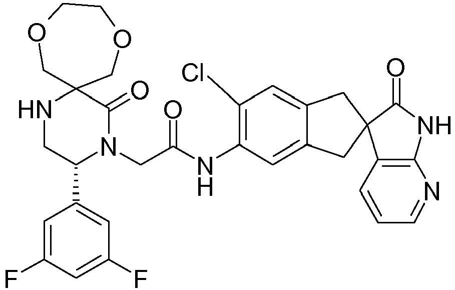

40 mg (0.06 mmol) (3*R*)-*tert*-Butyl 4-(2-(5-chlor-2'-oxo-1,1',2',3-tetrahydrospiro[inden-2,3'-pyrrolo[2,3-b]pyridin]-6-ylamino)-2-oxoethyl)-3-(3,5-difluorophenyl)-5-oxo-8,11-dioxa-1,4-diazaspiro[5.6]dodecan-1-carboxylat wurden mit 30 ml einer 1 N methanolischer Salzsäure Lösung versetzt und 1 h bei 50°C gerührt. Der Reaktionsansatz wurde einrotiert. Der Rückstand wurde in Wasser aufgenommen und mit gesättigter NaHCO₃ Lösung auf pH=8 gestellt. Der ausgefallene Niederschlag wurde abgesaugt, mit Wasser gewaschen und getrocknet.

| | |
|---|---|
| Ausbeute: | 19 mg (55% d. Th.) |
| ESI-MS: | m/z =624 (M+H)⁺ |
| Rₜ (HPLC): | 1.17 min (Methode A) |

### Beispiel 20:

### (R)-tert-Butyl 5-oxo-4-(2-oxo-2-((R)-2'-oxo-1,1',2',3-tetrahydrospiro[inden-2,3'-pyrrolo-[2,3-b]pyridin]-5-ylamino)ethyl)-3-phenyl-8,11-dioxa-1,4-diazaspiro[5.6]dodecan-1-carboxylat

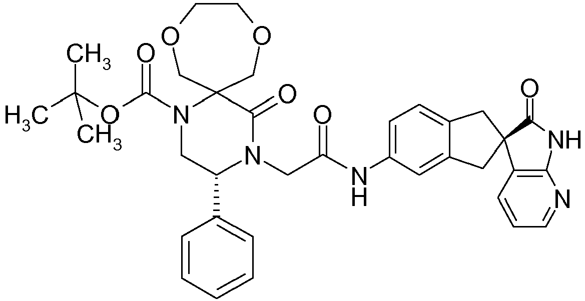

0.21 g (0.5 mmol) (*R*)-2-(1-(*tert*-Butoxycarbonyl)-5-oxo-3-phenyl-8,11-dioxa-1,4-diaza-spiro[5.6]dodecan-4-yl)ethansäure in 3 ml DMF wurden mit 0.38 g (1 mmol) HATU und 0.18 ml (1.3 mmol) TEA versetzt und 10 min bei RT gerührt. Anschließend wurden 0.13 mg (0.5 mmol) (*S*)-5-Amino-1,3-dihydrospiro[inden-2,3'-pyrrolo[2,3-b]pyridin]-2'(1'H)-on zugegeben und über Nacht bei RT gerührt. Das Reaktionsgemisch wurde auf Eiswasser gegossen. Der ausgefallene Niederschlag wurde abgesaugt, mit Wasser gewaschen und getrocknet.

| | |
|---|---|
| Ausbeute: | 320 mg (98% d. Th.) |
| ESI-MS: | m/z = 654 (M+H)⁺ |
| Rₜ (HPLC): | 1.41 min (Methode A) |

### Beispiel 21:

### N-((R)-2'-Oxo-1,1',2',3-tetrahydrospiro[inden-2,3'-pyrrolo[2,3-b]pyridin]-5-yl)-2-((R)-5-oxo-3-phenyl-8,11-dioxa-1,4-diazaspiro[5.6]dodecan-4-yl)acetamid

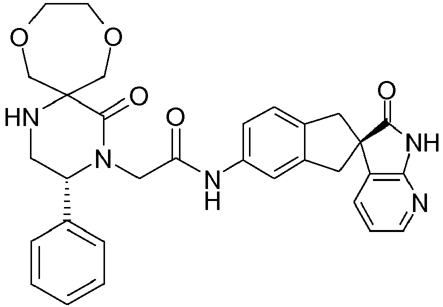

0.27 g (0.41 mmol) (*R*)*-tert-*Butyl 5-oxo-4-(2-oxo-2-((*R*)-2'-oxo-1,1',2',3-tetrahydrospiro-[inden-2,3'-pyrrolo-[2,3-b]pyridin]-5-ylamino)ethyl)-3-phenyl-8,11-dioxa-1,4-diazaspiro[5.6]-dodecan-1-carboxylat in 5 ml Methanol wurden mit 10 ml einer 1.25 M methanolischen Salzsäure versetzt und 3 h bei 50°C gerührt. Der Reaktionsansatz wurde einrotiert und mit gesättigter NaHCO₃-Lösung neutralisiert. Der ausgefallene Niederschlag wurde abgesaugt, mit Wasser gewaschen und getrocknet.

| | |
|---|---|
| Ausbeute: | 160 mg (71 % d. Th.) |
| ESI-MS: | m/z =554 (M+H)⁺ |
| Rₜ (HPLC): | 1.00 min (Methode A) |

### Beispiel 22:

### 2-((R)-3-(4-Fluorphenyl)-5-oxo-8,11-dioxa-1,4-diazaspiro[5.6]dodecan-4-yl)-N-((R)-2'-oxo-1,1',2',3-tetrahydrospiro[inden-2,3'-pyrrolo[2,3-b]pyridin]-5-yl)acetamid

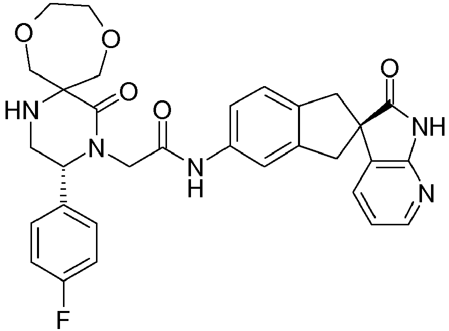

0.10 g (0.17 mmol) eines Racemats von 2-(3-(4-Fluorphenyl)-5-oxo-8,11-dioxa-1,4-diaza-spiro[5.6]dodecan-4-yl)-N-((R)-2'-oxo-1,1',2',3-tetrahydrospiro[inden-2,3'-pyrrolo-[2,3-b]-pyridin]-5-yl)acetamid wurden über eine SFC-Anlage (1xIB, Laufmittel: 30%MeOH + 0.2% DEA; Fluss:10 ml/min, c = 25mg/min) in die entsprechenden Enatiomere aufgetrennt.

| | |
|---|---|
| Ausbeute: | 30.3 mg (30% d. Th.) |
| ESI-MS: | m/z =570 (M-H)⁻ |
| Rₜ (chirale-HPLC): | 3.63 min (Methode Daicel IB, 250mmx4.6mm, 5µm, 40°C, CO₂, MeOH+DEA, isokratisch 30%,4 ml / min) |

### Beispiel 23:

### 2-(3-(3-Fluorphenyl)-5-oxo-8,11-dioxa-1,4-diazaspiro[5.6]dodecan-4-yl)-N-((R)-2'-oxo-1,1',2',3-tetrahydrospiro[inden-2,3'-pyrrolo[2,3-b]pyridin]-5-yl)acetamid

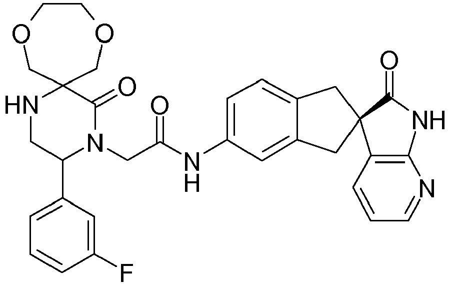

0.23 g (0.52 mmol) 2-(1-(*tert*-Butoxycarbonyl)-3-(3-fluorphenyl)-5-oxo-8,11-dioxa-1,4-diazaspiro[5.6]dodecan-4-yl)ethansäure, 0.15 mg (0.58 mmol) (*R*)-5-Amino-1,3-dihydro-spiro[inden-2,3'-pyrrolo[2,3-b]pyridin]-2'(1'H)-on, 0.13 g (0.70 mmol) 1-Ethyl-3-(3-dimethylaminopropyl)carbodiimid Hydrochlorid, 95 mg (0.7 mmol) HOBT, 0.08 ml (0.58 mmol) TEA und 2 ml DMF wurden über Nacht bei RT gerührt. Der Ansatz wurde einrotiert. Der Rückstand wurde mit 15 ml einer 1.25M methanolischer Salzsäure versetzt und 2 h bei 50°C gerührt. Das Reaktionsgemisch wurde eingeengt und mittels HPLC aufgereinigt.

| | |
|---|---|
| Ausbeute: | 51 mg (17% d. Th.) |
| ESI-MS: | m/z = 572 (M+H)⁺ |
| Rₜ (HPLC): | 1.047 min (Methode A) |

### Beispiel 24:

### 2-((R)-3-(3,5-Difluorphenyl)-5-oxo-8,11-dioxa-1,4-diazaspiro[5.6]dodecan-4-yl)-N-(2'-oxo-1',2',5,7-tetrahydrospiro[cyclopenta[b]pyridin-6,3'-pyrrolo[2,3-b]pyridin]-3-yl)acetamid

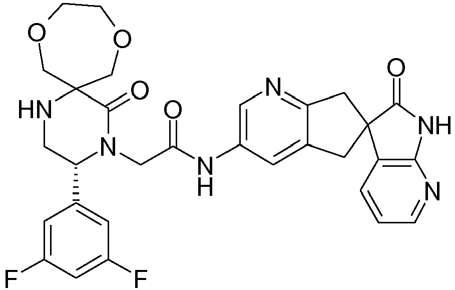

0.18 g (0.26 mmol) (3*R*)-*tert*-Butyl 3-(3,5-difluorphenyl)-5-oxo-4-(2-oxo-2-(2'-oxo-1',2',5,7-tetrahydrospiro[cyclopenta[b]pyridin-6,3'-pyrrolo[2,3-b]pyridin]-3-ylamino)ethyl)-8,11-dioxa-1,4-diazaspiro[5.6]dodecan-1-carboxylat wurden mit 30 ml einer 1 N methanolischer Salzsäurelösung versetzt und 1 h bei 50°C gerührt. Der Reaktionsansatz wurde einrotiert und mittels HPLC aufgereinigt. Die Produktfraktionen wurden eingeengt, mit Natriumhydrogencarbonat neutralisiert und die Wasserphase mit DCM extrahiert. Die organische Phase wurde einrotiert. Der Rückstand mit Diethylether verrieben, abgesaugt, mit Diethylether gewaschen und getrocknet.

| | |
|---|---|
| Ausbeute: | 14 mg (9.1% d. Th.) |
| ESI-MS: | m/z =589 (M-H)⁻ |
| Rₜ (HPLC): | 0.90 min (Methode A) |

Die nachfolgenden Beispiele beschreiben die Herstellung pharmazeutischer Anwendungsformen, die als Wirkstoff eine beliebige Verbindung der allgemeinen Formel I enthalten:

### Beispiel I

Kapseln zur Pulverinhalation mit 1 mg Wirkstoff

### Zusammensetzung:

1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

### Zusammensetzung:

1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

### Zusammensetzung:

1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

### Zusammensetzung:

1 Hub enthält:

| | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

### Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellunagverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

**Lösungsmittel für Lyophilisat:**

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel **I** 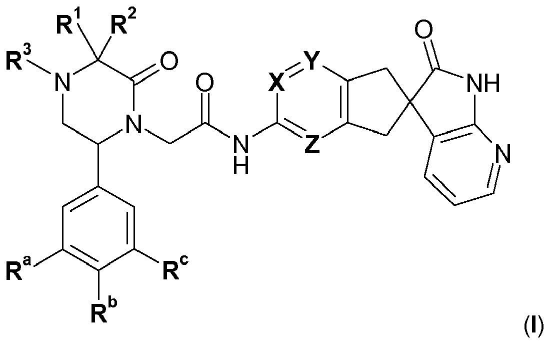 in der
**X** C-H, C-Cl oder N,
**Y, Z** unabhängig voneinander jeweils CH oder N,
wobei maximal nur ein Substituent X, Y oder Z ein Stickstoffatom bedeutet,
(a)
**R¹** H,
**R²** **R^{2.1}**-CH₂- und
**R^{2.1}** die Gruppe 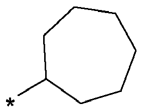 bedeutet oder
(b)
**R¹** und **R²** zusammen mit dem Kohlenstoffatom, an das sie gebunden sind, den Rest 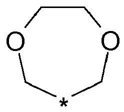 bilden und
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

2. Verbindungen der allgemeinen Formel **I** gemäß Anspruch 1, in denen
**X, Y, Z** jeweils C-H oder N, wobei maximal nur ein N im Ring enthalten ist,
(a)
**R¹** H und
**R²** die Gruppe 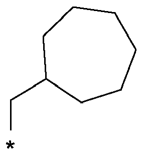 bedeutet,
(b) **R¹** und **R²** zusammen mit dem Kohlenstoffatom, an das sie gebunden sind, die Gruppe 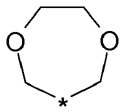 bedeutet,
und
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

3. Verbindungen der allgemeinen Formel **I** gemäß Anspruch 1, in denen
**X, Y, Z** jeweils C-H oder N, wobei maximal nur ein N im Ring enthalten ist,
**R¹** H,
**R²** die Gruppe 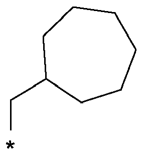 bedeutet,
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

4. Verbindungen der allgemeinen Formel **I** gemäß Anspruch 1, in denen
**X, Y, Z** jeweils C-H oder N, wobei maximal nur ein N im Ring enthalten ist,
**R¹** H,
**R²** die Gruppe 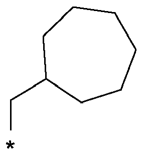 bedeutet,
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

5. Verbindungen der allgemeinen Formel **I** gemäß Anspruch 1, in denen
**X, Y, Z** jeweils C-H oder N, wobei maximal nur ein N im Ring enthalten ist,
**R¹** und **R²** zusammen mit dem Kohlenstoffatom, an das sie gebunden sind, die Gruppe 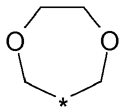 bedeutet,
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

6. Verbindungen der allgemeinen Formel **I** gemäß Anspruch 1, in denen
**X, Y,** **Z**jeweils C-H oder N, wobei maximal nur ein N im Ring enthalten ist,
**R¹** und **R²** zusammen mit dem Kohlenstoffatom, an das sie gebunden sind, eine Gruppe 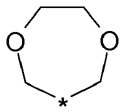 bedeutet,
**R³** H, -C(O)-O-C₁₋₄-Alkyl oder eine C₁₋₆-Alkylgruppe, die mit 1, 2, 3, 4 oder 5 Fluoratomen substituiert sein kann,
**R^{a}** H, F, -OCH₃ oder -OCF₃,
**R^{b}** H, F, -OCH₃ oder -OCF₃, und
**R^{c}** H, F, -OCH₃ oder -OCF₃ bedeutet,
deren individuelle Diastereomere, deren individuelle Enantiomere und deren Salze, insbesondere deren physiologisch verträgliche Salze mit anorganischen oder organischen Säuren oder Basen.

7. Verbindungen der allgemeinen Formel **I** gemäß Anspruch 1, die ausgewählt sind aus der Gruppe bestehend aus:
| **Nr.** | **Struktur** |
|---|---|
| (1) | 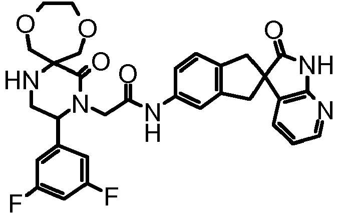 |
| (1a) | 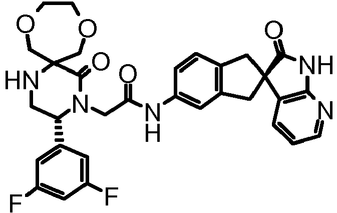 |
| (1b) | 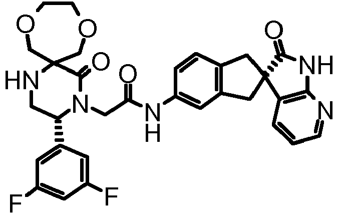 |
| (2) | 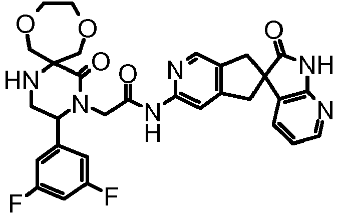 |
| (3) | 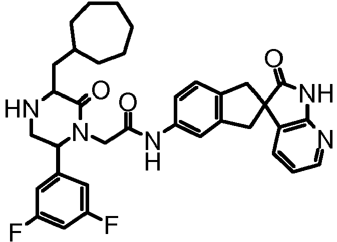 |
| (4) | 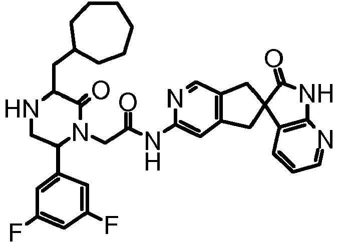 |
| (13) | 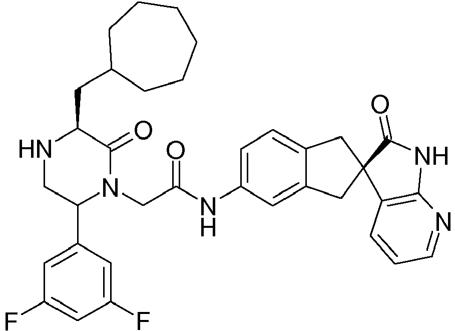 |
| (16) | 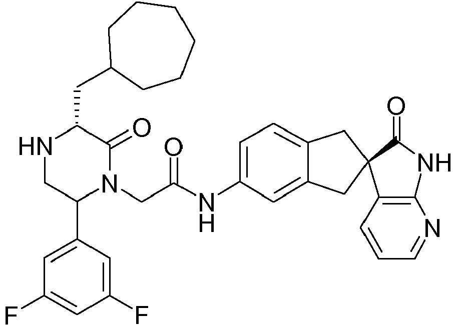 |
| (17) | 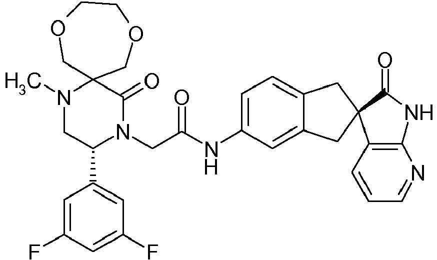 |
| (18) | 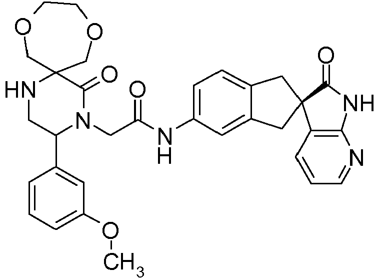 |
| (19) | 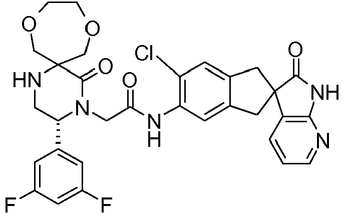 |
| (20) | 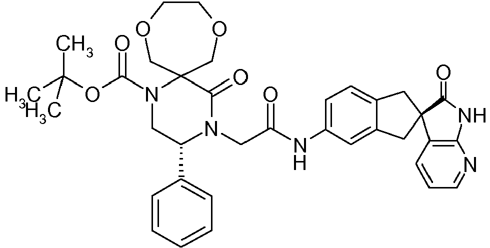 |
| (21) | 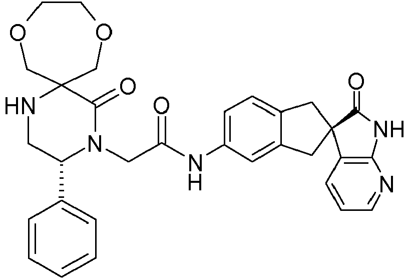 |
| (22) | 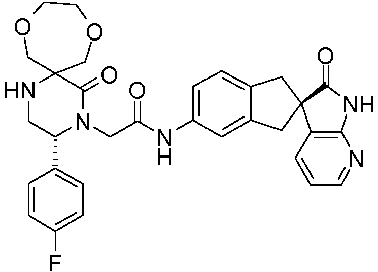 |
| (23) | 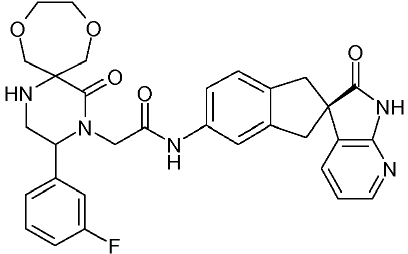 |
| (24) | 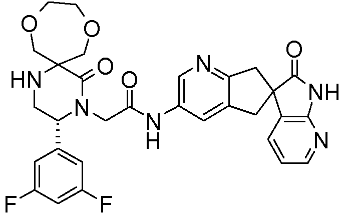 |

8. Arzneimittel, enthaltend eine Verbindung nach einem der Ansprüche 1 bis 7 oder ein physiologisch verträgliches Salz davon neben gegebenenfalls einem oder mehreren inerten Trägerstoffen und/oder Verdünnungsmitteln.

9. Eine Verbindung nach einem der Ansprüche 1 bis 7 zur Verwendung als Arzneimittel für die Behandlung von Kopfschmerzen, insbesondere Migräne- bzw. Cluster-Kopfschmerz.

10. Eine Verbindung nach einem der Ansprüche 1 bis 7 zur Verwendung als Arzneimittel für die Behandlung von nicht insulinabhängigem Diabetes mellitus ("NIDDM"), des complex regional pain syndrome (CRPS1), cardiovaskulären Erkrankungen, Morphintoleranz, Clostridiumtoxin-bedingten Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündlichen Erkrankungen, z.B. entzündlichen Gelenkerkrankungen (Osteoarthritis, rheumatoide Arthritis, neurogene Arthritis), generalisiertem Weichteilrheumatismus (Fibromyalgie), neurogenen Entzündungen der oralen Mucosa, entzündlichen Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierten Neuropathien, HIV-induzierten Neuropathien, postherpetischen Neuropathien durch Gewebetrauma induzierten Neuropathien, trigeminalen Neuralgien, temporomandibulären Dysfunktionen, CRPS (complex regional pain syndrome), Rückenschmerzen, viszeralen Erkrankungen, wie z.B. irritable bowel syndrome (IBS), inflammatory bowel syndrome, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten und Kastraten.

11. Verfahren zur Herstellung eines Arzneimittels gemäß Anspruch 8 **dadurch gekennzeichnet, dass** auf nichtchemischem Weg eine Verbindung nach einem der Ansprüche 1 bis 7 in einen oder mehrere inerte Trägerstoffe und/oder Verdünnungsmittel eingearbeitet wird.

12. Verbindungen der allgemeinen Formel **II** 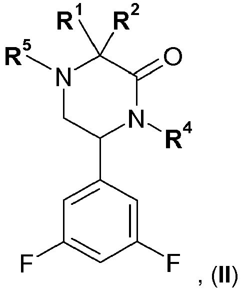 in der
**R¹** H,
**R²** die Gruppe 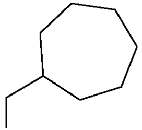
**R⁴** H oder CH₂-C(O)-O**R**^{4.1},
**R^{4.1}** H, C₁₋₆-Alkyl oder Benzyl,
**R⁵** H, Benzyl oder -C(O)-O**R**^{5.1},
**R^{5.1}** C₁₋₆-Alkyl oder Benzyl bedeutet,
deren individuelle Diastereomere, deren individuelle Enantiomere und deren Salze, insbesondere deren physiologisch verträgliche Salze mit anorganischen oder organischen Säuren oder Basen.

13. Verfahren zur Herstellung von Verbindungen der allgemeinen Formel **II** 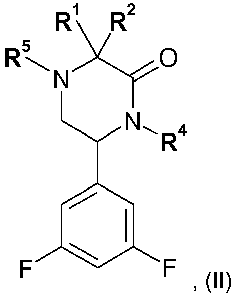 in der
**R¹** H,
**R²** die Gruppe 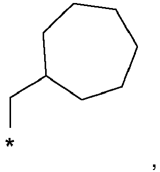
**R⁴** H oder -CH₂-C(O)-O**R^{4.1}**,
**R^{4.1}** H, C₁₋₆-Alkyl oder Benzyl,
**R⁵** H, Benzyl oder -C(O)-O**R^{5.1}**,
**R^{5.1}** C₁₋₆-Alkyl oder Benzyl bedeutet,
deren individuelle Diastereomere, deren individuelle Enantiomere und deren Salze, insbesondere deren physiologisch verträgliche Salze mit anorganischen oder organischen Säuren oder Basen,
umfassend die Schritte:
(a) Umsetzung einer Verbindung der allgemeinen Formel **III** 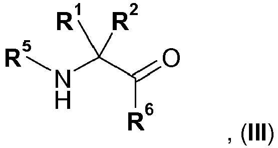 in der **R¹** und **R²** wie eingangs erwähnt definiert sind,
**R⁵** Benzyl oder -C(O)-O**R^{5.1}**,
**R^{5.1}** Benzyl und
**R⁶** -O-C₁₋₆-Alkyl bedeutet,
mit einer Verbindung der allgemeinen Formel **IV** 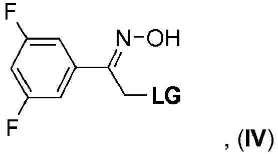 in der **LG** eine Abgangsgruppe darstellt, beispielsweise ein Brom- oder lodatom, eine Trifluormethansulfonyl-, Methansulfonyl- oder Toluolsulfonylgruppe
(b) Überführen einer unter Schritt (a) erhaltenen Verbindung der allgemeinen Formel **V** 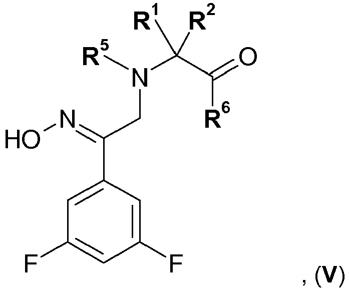 in der **R¹** und **R²** wie eingangs erwähnt definiert sind, **R⁵** Benzyl oder -C(O)-O-Benzyl und **R⁶** -die Gruppe O-C₁₋₆-Alkyl bedeutet, unter reduktiven Bedingungen, wie beispielsweise in einer Wasserstoffatmosphäre bei erhöhtem Druck und Temperatur und in Gegenwart eines Katalysators, beispielsweise in Gegenwart von Palladium auf Kohle, in eine Verbindung der allgemeinen Formel **VI** 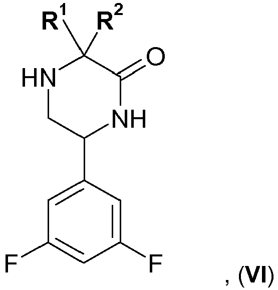 in der **R¹** und **R²** wie eingangs erwähnt definiert sind;
(c) Überführen einer unter Schritt (b) erhaltenen Verbindung der allgemeinen Formel **VI** in eine Verbindung der allgemeinen Formel **VII** 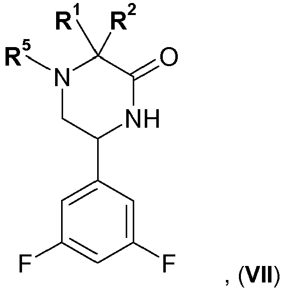 in der **R¹** und **R²** wie eingangs erwähnt definiert sind,
**R⁵** Benzyl oder -C(O)-O**R^{5.1}** und
**R^{5.1}** C₁₋₆-Alkyl oder Benzyl bedeutet;
(d) Umsetzung einer unter Schritt (c) erhaltenen Verbindung der allgemeinen Formel **VII** mit einer Verbindung der allgemeinen Formel **VIII** 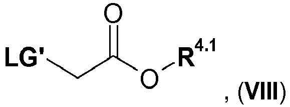 in der LG' eine Abgangsgruppe darstellt, beispielsweise ein Brom- oder lodatom, eine Trifluormethansulfonyl-, Methansulfonyl- oder Toluolsulfonylgruppe,
**R^{4.1}** H, C₁₋₆-Alkyl oder Benzyl bedeutet; und
(e) Isolieren einer unter Schritt (d) erhaltenen Verbindung der allgemeinen Formel **II,** in der **R¹**, **R², R⁴** und **R⁵** wie eingangs erwähnt definiert sind.

14. Verbindungen der allgemeinen Formel **IX** 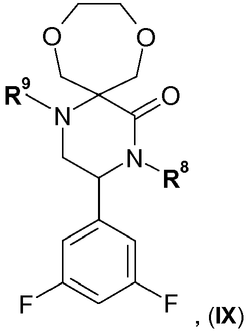 in der
**R⁸** H oder -CH₂-C(O)-O-**R^{8.1},**
**R^{8.1}** H, C₁₋₆-Alkyl oder Benzyl und
**R⁹** H, Benzyl, Benzyl-O-C(O)- oder C₁-₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen.

15. Verfahren zur Herstellung von Verbindungen der allgemeinen Formel **IX,** 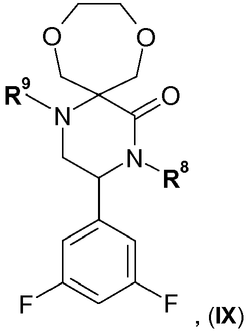 in der
**R⁸** H oder -CH₂-C(O)-O-**R^{8.1}**,
**R^{8.1}** H, C₁₋₆-Alkyl oder Benzyl und
**R⁹** H, Benzyl, Benzyl-O-C(O)- oder C₁₋₆-Alkyl-O-C(O)- bedeutet,
deren individuelle Diastereomere, deren individuelle Enantiomere und deren Salze, insbesondere deren physiologisch verträgliche Salze mit anorganischen oder organischen Säuren oder Basen,
umfassend die Schritte:
(a) Umsetzung einer Verbindung der allgemeinen Formel **X** 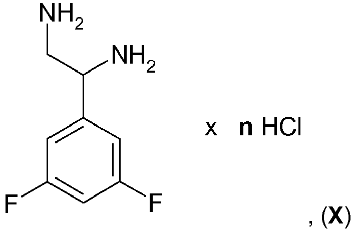 in der n eine der Ziffern 0, 1 oder 2 darstellt, mit [1,4]Dioxepan-6-on in Gegenwart eines Phasentransfer-Katalysators, wie beispielsweise Benzyltriethylammonium-chlorid, sowie in Gegenwart von Chloroform und Natronlauge;
(b) Isolieren einer unter Schritt (a) erhaltenen Verbindung der Formel **XI** 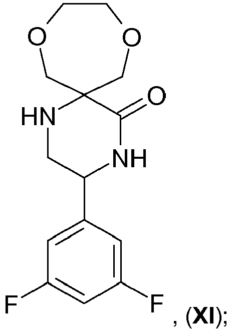
(c) Überführen einer unter Schritt (b) erhaltenen Verbindung der Formel **XI** in eine Verbindung der allgemeinen Formel **IX** 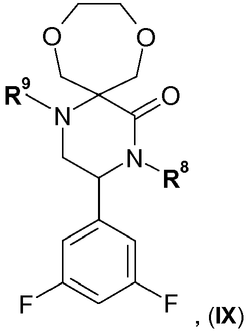 in der
**R⁸** H und
**R⁹** Benzyl, Benzyl-O-C(O)- oder C₁₋₆-Alkyl-O-C(O)- bedeutet;
(d) Umsetzung einer unter Schritt (c) erhaltenen Verbindung der allgemeinen Formel **IX** mit einer Verbindung der allgemeinen Formel **VIII** 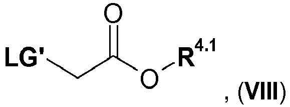 in der **LG'** eine Abgangsgruppe darstellt, beispielsweise ein Brom- oder lodatom, eine Trifluormethansulfonyl-, Methansulfonyl- oder Toluolsulfonylgruppe,
**R^{4.1}** H, C₁₋₆-Alkyl oder Benzyl bedeutet; und
(e) Isolieren einer unter Schritt (d) erhaltenen Verbindung der allgemeinen Formel **IX** 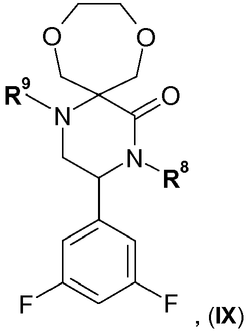 in der
**R⁸** -CH₂-C(O)-O**-R^{8.1}**,
**R^{8.1}** H, C₁₋₆-Alkyl oder Benzyl und
**R⁹** Benzyl, Benzyl-O-C(O)- oder C₁₋₆-Alkyl-O-C(O)- bedeutet.

## Claims

1. Compounds of general formula I 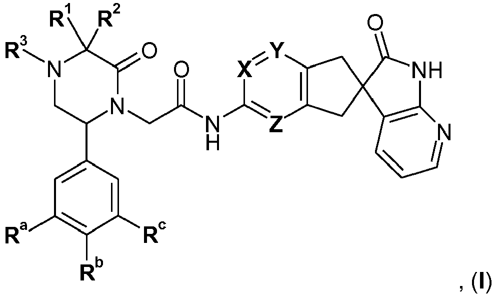 wherein
**X** denotes C-H, C-Cl or N,
**Y, Z** independently of one another each denote CH or N,
wherein at most only one substituent **X, Y or Z** denotes a nitrogen atom,
(a)
**R¹** denotes H,
**R²** denotes **R^{2.1}**-CH₂- and
**R^{2.1}** denotes the group 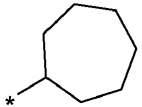 or
(b) **R¹** and **R²** together with the carbon atom to which they are attached form the group 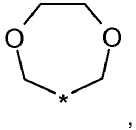 and
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

2. Compounds of general formula **I** according to claim 1, wherein
**X, Y, Z** in each case denote C-H or N, there being at most only one N in the ring,
(a)
**R¹** denotes H and
**R²** denotes the group 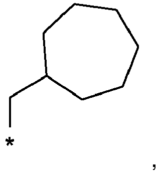
(b) **R¹** and **R²** together with the carbon atom to which they are attached denote the group 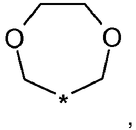 and
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

3. Compounds of general formula **I** according to claim 1, wherein
**X, Y, Z** in each case denote C-H or N, there being at most only one N in the ring,
**R¹** denotes H,
**R²** denotes the group 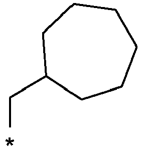
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

4. Compounds of general formula **I** according to claim 1, wherein
**X, Y, Z** in each case denote C-H or N, there being at most only one N in the ring,
**R¹** denotes H,
**R²** denotes the group 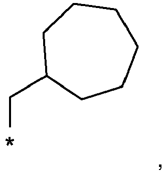
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

5. Compounds of general formula **I** according to claim 1, wherein
**X, Y, Z** in each case denote C-H or N, there being at most only one N in the ring,
**R¹** and **R²** together with the carbon atom to which they are attached denote the group 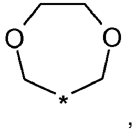
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

6. Compounds of general formula **I** according to claim 1, wherein
**X, Y, Z** in each case denote C-H or N, there being at most only one N in the ring,
**R¹** and **R²** together with the carbon atom to which they are attached denote a group 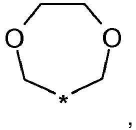
**R³** denotes H, -C(O)-O-C₁₋₄-alkyl or a C₁₋₆-alkyl group which may be substituted by 1, 2, 3, 4 or 5 fluorine atoms,
**R^{a}** denotes H, F, -OCH₃ or -OCF₃,
**R^{b}** denotes H, F, -OCH₃ or -OCF₃, and
**R^{c}** denotes H, F, -OCH₃ or -OCF₃,
the individual diastereomers, the individual enantiomers and the salts thereof, particularly the physiologically acceptable salts thereof with inorganic or organic acids or bases.

7. Compounds of general formula **I** according to claim 1, which are selected from among:
| **No.** | **Structure** |
|---|---|
| (1) | 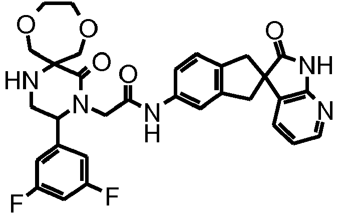 |
| (1a) | 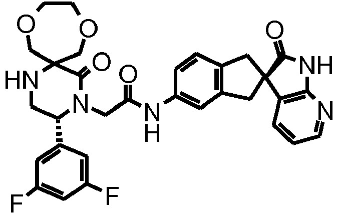 |
| (1b) | 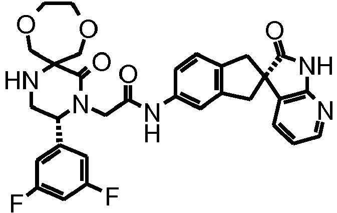 |
| (2) | 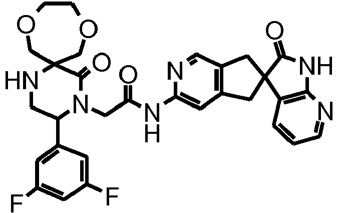 |
| (3) | 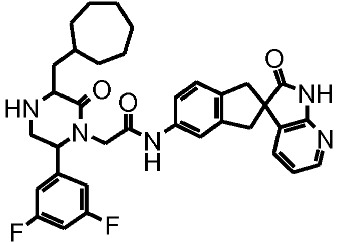 |
| (4) | 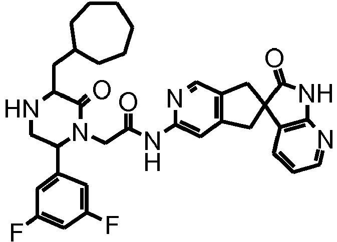 |
| (13) | 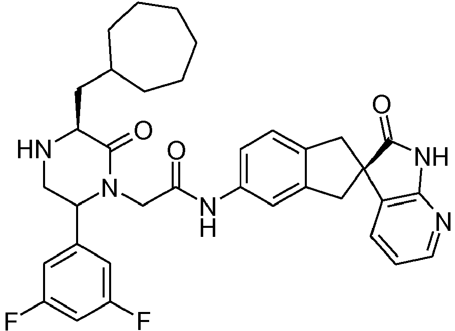 |
| (16) | 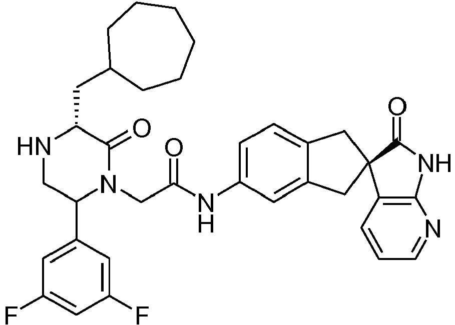 |
| (17) | 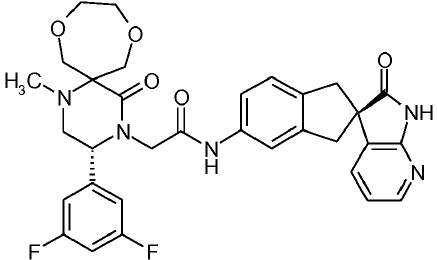 |
| (18) | 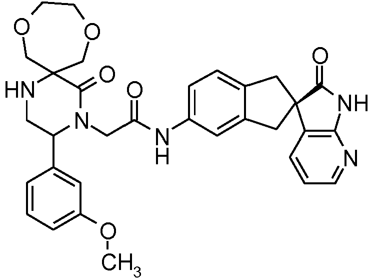 |
| (19) | 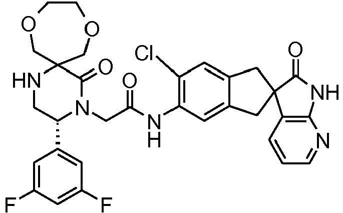 |
| (20) | 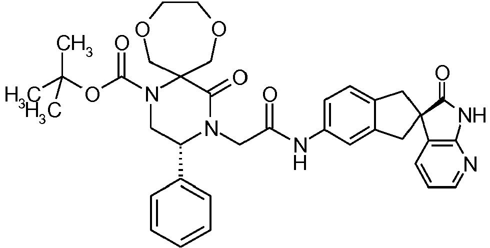 |
| (21) | 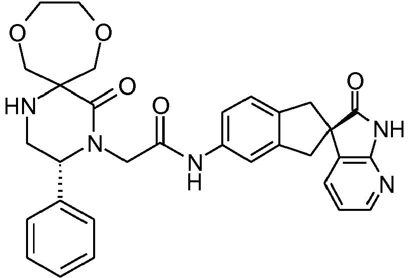 |
| (22) | 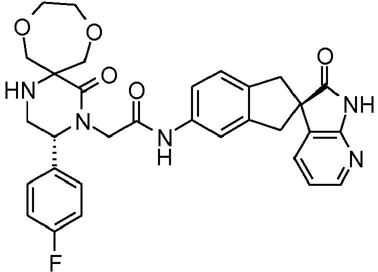 |
| (23) | 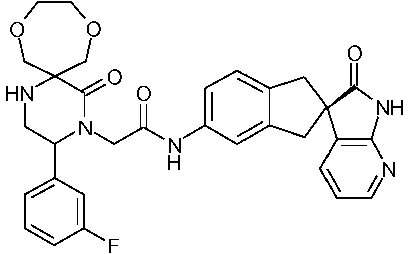 |
| (24) | 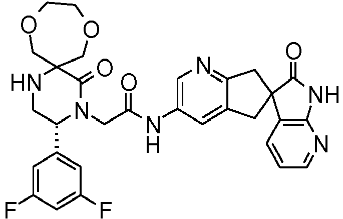 |

8. Medicaments, containing a compound according to one of claims 1 to 7 or a physiologically acceptable salt thereof, optionally together with one or more inert carriers and/or diluents.

9. A compound according to one of claims 1 to 7 for use as a medicament for the treatment of headaches, particularly migraine or cluster headaches.

10. A compound according to one of claims 1 to 7 for use as a medicament for the treatment of non-insulin-dependent diabetes mellitus ("NIDDM"), complex regional pain syndrome (CRPS1), cardiovascular diseases, morphine tolerance, diarrhoea caused by clostridium toxin, skin diseases, particularly thermal and radiation-induced skin damage including sunburn, lichen, pruritis, pruritic toxidermies and severe itching, inflammatory diseases, e.g. inflammatory diseases of the joints (osteoarthritis, rheumatoid arthritis, neurogenic arthritis), generalised soft-tissue rheumatism (fibromyalgia), neurogenic inflammation of the oral mucosa, inflammatory lung diseases, allergic rhinitis, asthma, COPD, diseases accompanied by excessive vasodilatation and resultant reduced blood supply to the tissues, e.g. shock and sepsis, chronic pain, e.g. diabetic neuropathies, neuropathies induced by chemotherapy, HIV-induced neuropathies, postherpetic neuropathies, neuropathies induced by tissue trauma, trigeminal neuralgias, temporomandibular dysfunctions, CRPS (complex regional pain syndrome), back pain, visceral complaints, such as e.g. irritable bowel syndrome (IBS), inflammatory bowel syndrome, for relieving pain, or for preventive or acute therapeutic treatment of the symptoms of menopausal hot flushes caused by vasodilatation and increased blood flow in oestrogen-deficient women and hormone-treated patients with prostate carcinoma and castrated men.

11. Method for preparing a medicament according to claim 8, **characterised in that** a compound according to one of claims 1 to 7 is incorporated by a non-chemical method in one or more inert carriers and/or diluents.

12. Compounds of general formula II 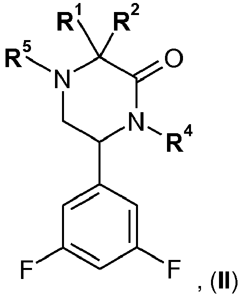 wherein
**R¹** denotes H,
**R²** denotes the group 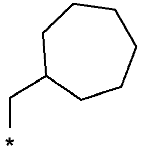
**R⁴** denotes H or CH₂-C(O)-O**R^{4.1}**,
**R^{4.1}** denotes H, C₁₋₆-alkyl or benzyl,
**R⁵** denotes H, benzyl or -C(O)-O**R^{5.1}**,
**R^{5.1}** denotes C₁₋₆-alkyl or benzyl,
the individual diastereomers, the individual enantiomers and the salts thereof, particularly the physiologically acceptable salts thereof with inorganic or organic acids or bases.

13. Process for preparing compounds of general formula **II** 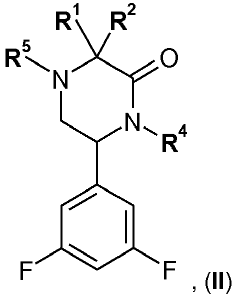 wherein
**R¹** denotes H,
**R²** denotes the group 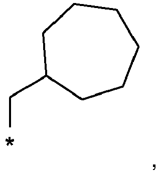
**R⁴** denotes H or -CH₂-C(O)-O**R**^{**4.**1},
**R^{4.1}** denotes H, C₁₋₆-alkyl or benzyl,
**R⁵** denotes H, benzyl or -C(O)-O**R^{5.1}**,
**R^{5.1}** denotes C₁₋₆-alkyl or benzyl,
the individual diastereomers, the individual enantiomers and the salts thereof, particularly the physiologically acceptable salts thereof with inorganic or organic acids or bases,
comprising the steps of:
(a) reacting a compound of general formula **III** 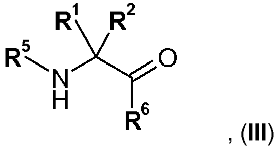 wherein **R¹** and **R²** are as hereinbefore defined,
**R⁵** denotes benzyl or -C(O)-O**R^{5.1}**,
**R^{5.1}** denotes benzyl and
**R⁶** denotes -O-C₁₋₆-alkyl,
with a compound of general formula **IV** 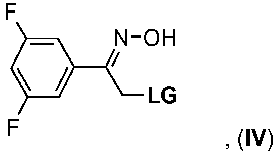 wherein **LG** denotes a leaving group, for example a bromine or iodine atom, a trifluoromethanesulphonyl, methanesulphonyl or toluenesulphonyl group
(b) converting a compound of general formula **V** obtained in step (a) 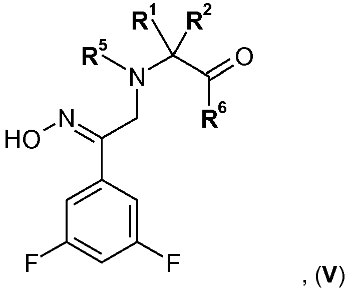 wherein **R¹** and **R²** are as hereinbefore defined, **R⁵** denotes benzyl or -C(O)-O-benzyl and **R⁶** denotes the group -O-C₁₋₆-alkyl, under reductive conditions, such as for example in a hydrogen atmosphere at elevated pressure and temperature and in the presence of a catalyst, for example in the presence of palladium on charcoal, into a compound of general formula **VI** 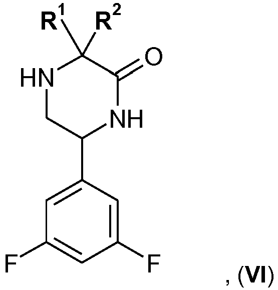 wherein **R¹** and **R²** are as hereinbefore defined;
(c) converting a compound of general formula **VI** obtained in step (b) into a compound of general formula **VII** 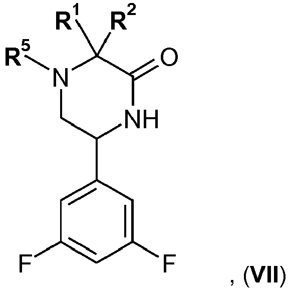 wherein **R¹** and **R²** are as hereinbefore defined,
**R⁵** denotes benzyl or -C(O)-O**R^{5.1}** and
**R^{5.1}** denotes C₁₋₆-alkyl or benzyl;
(d) reacting a compound of general formula **VII** obtained in step (c) with a compound of general formula **VIII** 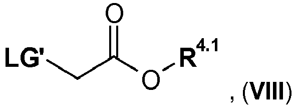 wherein LG' denotes a leaving group, for example a bromine or iodine atom, a trifluoromethanesulphonyl, methanesulphonyl or toluenesulphonyl group,
**R^{4.1}** denotes H, C₁₋₆-alkyl or benzyl; and
(e) isolating a compound of general formula **II** obtained in step (d), wherein **R¹**, **R², R⁴** and **R⁵** are as hereinbefore defined.

14. Compounds of general formula **IX** 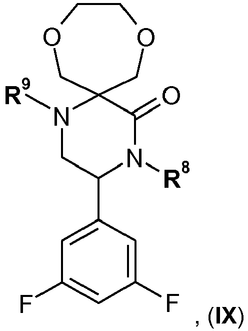 wherein
**R⁸** denotes H or -CH₂-C(O)-O-**R^{8.1}**,
**R^{8.1}** denotes H, C₁₋₆-alkyl or benzyl and
**R⁹** denotes H, benzyl, benzyl-O-C(O)- or C₁₋₆-alkyl-O-C(O)-,
the individual diastereomers, the individual enantiomers and the salts thereof, particularly the physiologically acceptable salts thereof with inorganic or organic acids or bases.

15. Process for preparing compounds of general formula IX, 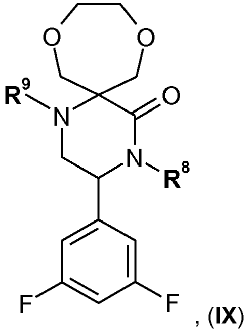 wherein
**R⁸** denotes H or -CH₂-C(O)-O-**R^{8.1}**,
**R^{8.1}** denotes H, C₁₋₆-alkyl or benzyl and
**R⁹** denotes H, benzyl, benzyl-O-C(O)- or C₁₋₆-alkyl-O-C(O)-,
the individual diastereomers, the individual enantiomers and the salts thereof, particularly the physiologically acceptable salts thereof with inorganic or organic acids or bases,
comprising the steps of:
(a) reacting a compound of general formula X 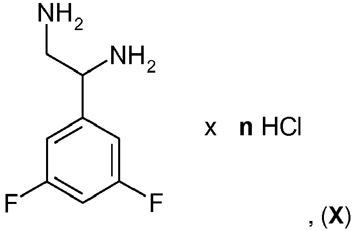 wherein n denotes one of the numbers 0, 1 or 2, with [1,4]dioxepan-6-one in the presence of a phase transfer catalyst, such as for example benzyltriethylammonium chloride, as well as in the presence of chloroform and sodium hydroxide solution;
(b) isolating a compound of formula **XI** obtained in step (a) 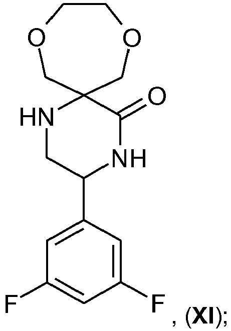
(c) converting a compound of formula **XI** obtained in step (b) into a compound of general formula **IX** 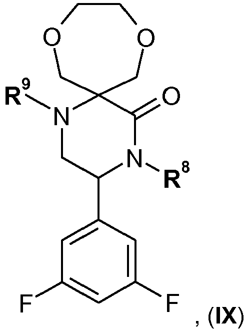 wherein
**R⁸** denotes H and
**R⁹** denotes benzyl, benzyl-O-C(O)- or C₁₋₆-alkyl-O-C(O)-;
(d) reacting a compound of general formula **IX** obtained in step (c) with a compound of general formula **VIII** 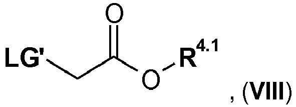 wherein LG' denotes a leaving group, for example a bromine or iodine atom, a trifluoromethanesulphonyl, methanesulphonyl or toluenesulphonyl group,
**R^{4.1}** denotes H, C₁₋₆-alkyl or benzyl; and
(e) isolating a compound of general formula **IX** obtained in step (d) 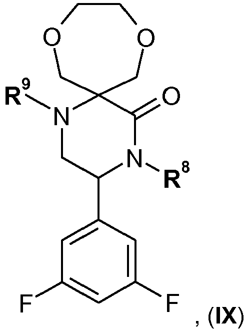 wherein
**R⁸** denotes-CH₂-C(O)-O-**R^{8.1}**,
**R^{8.1}** denotes H, C₁₋₆-alkyl or benzyl and
**R⁹** denotes benzyl, benzyl-O-C(O)- or C₁₋₆-alkyl-O-C(O)-.

## Revendications

1. Composés de formule générale I 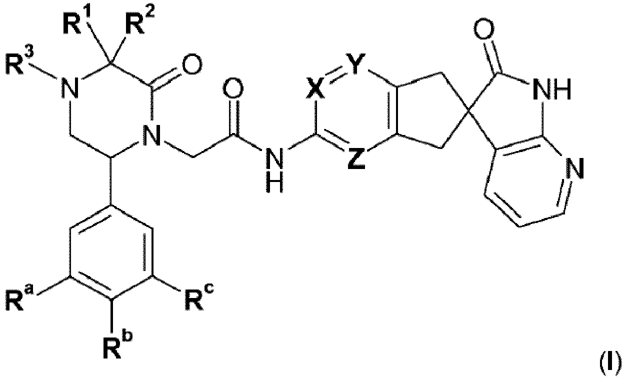 dans laquelle
X représente C-H, C-Cl ou N,
Y, Z représentent respectivement, indépendamment l'un de l'autre, CH ou N,
dans laquelle au maximum seul un substituant X, Y ou Z représente un atome d'azote,
(a)
R¹ représente H,
R² représente R^{2.1}-CH₂- et
R^{2.1} représente le groupe 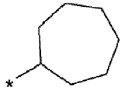 ou
(b) R¹ et R² forment conjointement avec l'atome de carbone auquel ils sont liés, le radical 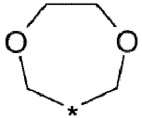 et
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

2. Composés de formule générale I selon la revendication 1, dans lesquels
X, Y, Z représentent respectivement C-H ou N, dans laquelle au maximum seul un N est contenu dans le cycle,
(a)
R¹ représente H et
R² représente le groupe 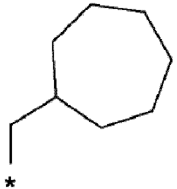
(b) R¹ et R², conjointement avec l'atome de carbone auquel ils sont liés, représentent le groupe 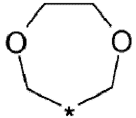 et
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

3. Composés de formule générale I selon la revendication 1, dans laquelle
X, Y, Z représentent respectivement C-H ou N, dans laquelle au maximum seul un N est contenu dans le cycle,
R¹ représente H,
R² représente le groupe 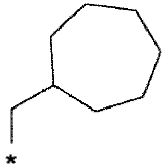
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

4. Composés de formule générale I selon la revendication 1, dans laquelle
X, Y, Z représentent respectivement C-H ou N, dans laquelle au maximum seul un N est contenu dans le cycle,
R¹ représente H
R² représente le groupe 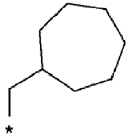
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

5. Composés de formule générale I selon la revendication 1, dans laquelle
X, Y, Z représentent respectivement C-H ou N, dans laquelle au maximum seul un N est contenu dans le cycle,
R¹ et R², conjointement avec l'atome de carbone auquel ils sont liés, représentent le groupe 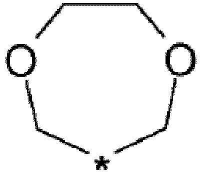
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

6. Composés de formule générale I selon la revendication 1, dans laquelle
X, Y, Z représentent respectivement C-H ou N, dans laquelle au maximum seul un N est contenu dans le cycle,
R¹ et R², conjointement avec l'atome de carbone auquel ils sont liés, représentent le groupe 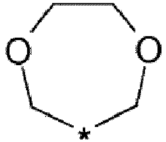
R³ représente H, un groupe -C(O)-O-C₁₋₄-alkyle ou un groupe C₁₋₆-alkyle qui peut être substitué par 1, 2, 3, 4 ou 5 atomes de fluor,
R^{a} représente H, F, -OCH₃ ou -OCF₃,
R^{b} représente H, F, -OCH₃ ou -OCF₃, et
R^{c} représente H, F, -OCH₃ ou -OCF₃,
leurs diastéréomères individuels, leurs énantiomères individuels et leurs sels, en particulier leurs sels physiologiquement acceptables avec des acides ou des bases inorganiques ou organiques.

7. Composés de formule générale I, selon la revendication 1, qui sont choisis dans le groupe constitué de :
| N° | Structure |
|---|---|
| (1) | 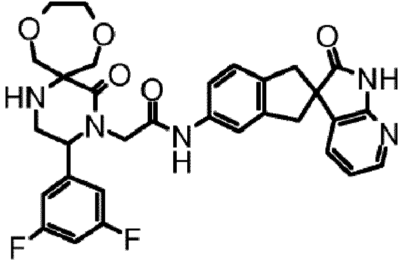 |
| (1a) | 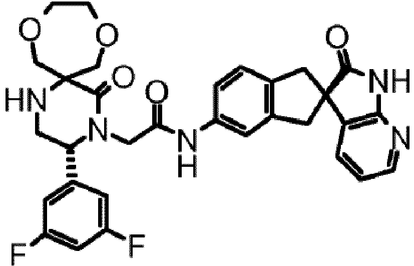 |
| (1b) | 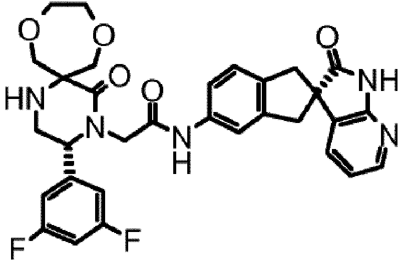 |
| (2) | 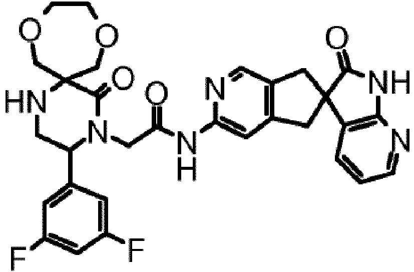 |
| (3) | 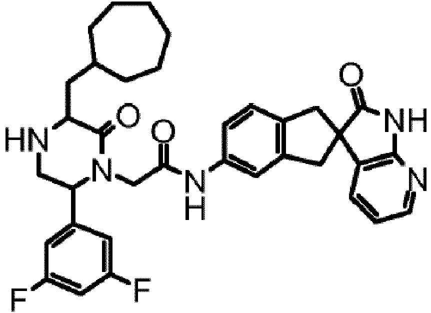 |
| (4) | 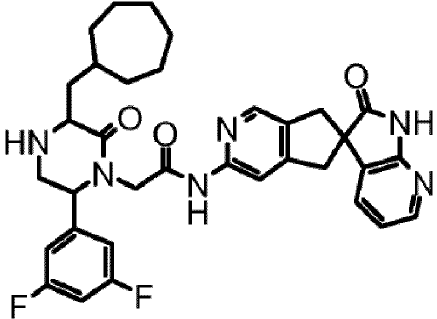 |
| (13) | 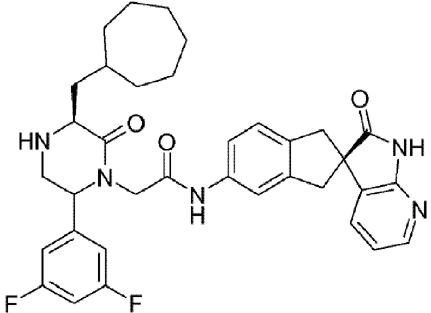 |
| (16) | 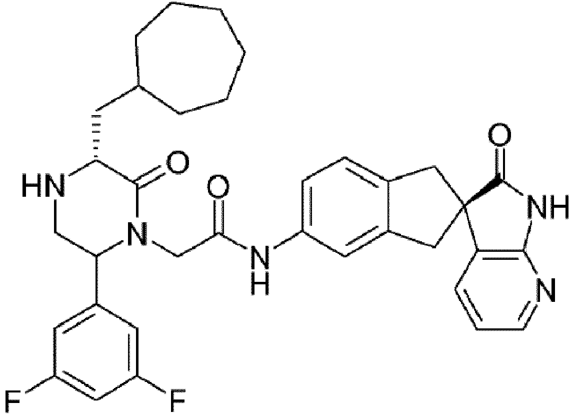 |
| (17) | 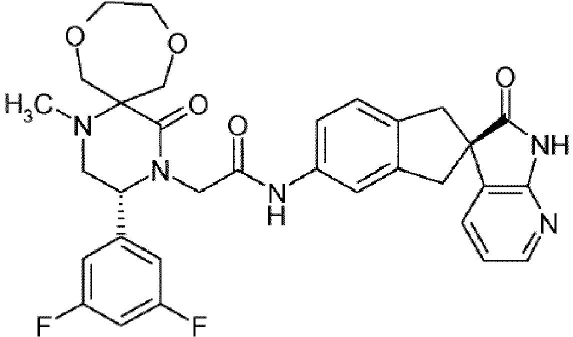 |
| (18) | 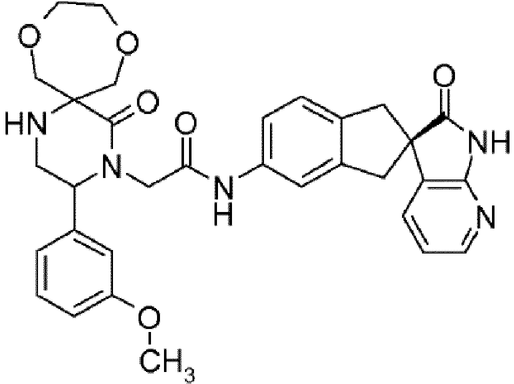 |
| (19) | 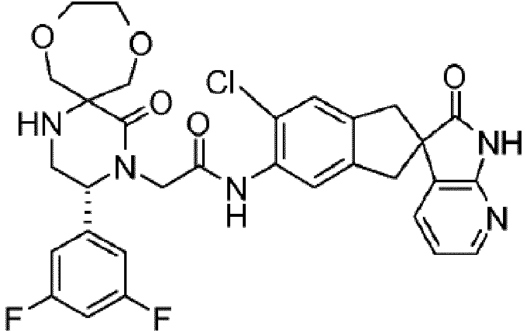 |
| (20) | 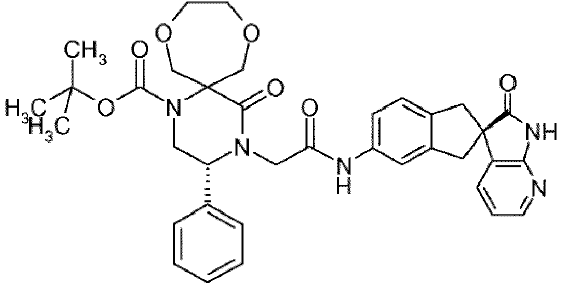 |
| (21) | 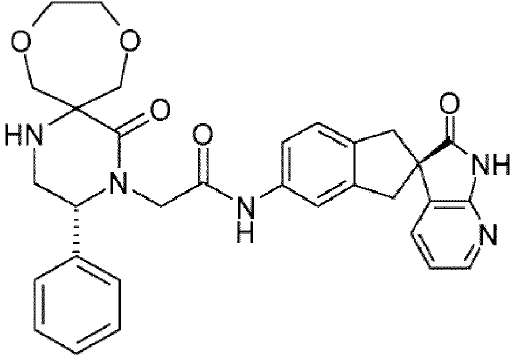 |
| (22) | 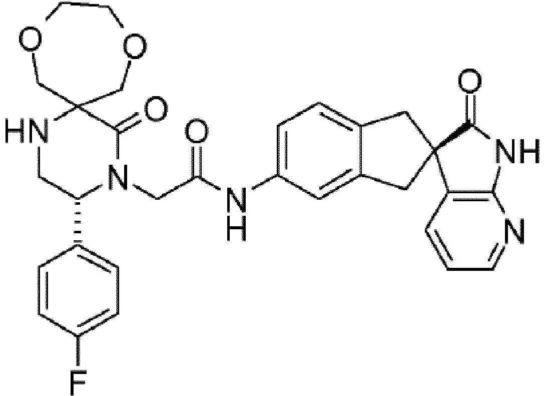 |
| (23) | 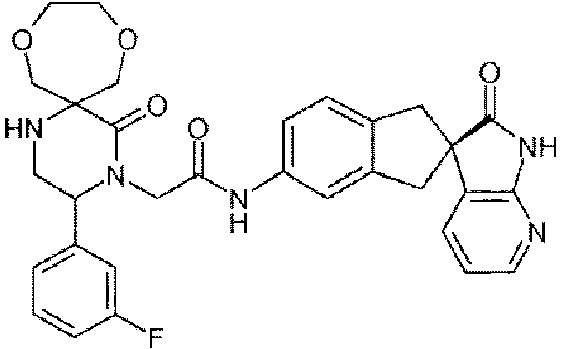 |
| (24) | 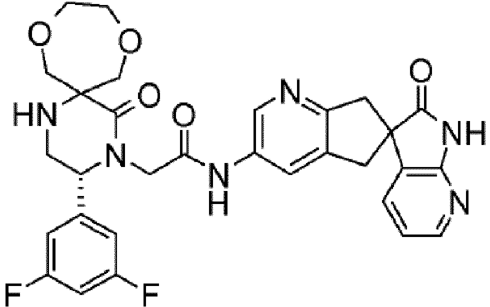 |

8. Médicament contenant un composé selon l'une des revendications 1 à 7, ou un sel physiologiquement acceptable de celui-ci, outre éventuellement un ou plusieurs véhicules et/ou diluants inertes.

9. Composé selon l'une des revendications 1 à 7,
pour son utilisation comme médicament,
pour le traitement des céphalées, en particulier des migraines ou de l'algie vasculaire de la face.

10. Composés selon l'une des revendications 1 à 7, pour son utilisation comme médicament
pour le traitement du diabète sucré non insulino-dépendant (« NIDDM »), du syndrome de douleur régionale complexe (CRPS1), de maladies cardiovasculaires, de la tolérance à la morphine, des diarrhées dues à *Clostridium,* des maladies de la peau, en particulier, des lésions cutanées thermiques et dues à un rayonnement comprenant les coup de soleil, le lichen, le prurit, les toxidermies prurigineuses et les démangeaisons sévères, des maladies inflammatoires, par exemple, des affections articulaires inflammatoires (arthrose, arthrite rhumatoïde, arthrite neurogène), des rhumatismes généralisés des tissus mous (fibromyalgie), des inflammations neurogènes de la muqueuse buccale, des maladies pulmonaires inflammatoires, la rhinite allergique, l'asthme, la BPCO, des maladies qui s'accompagnent d'une vasodilatation excessive et ainsi, d'une réduction de l'irrigation tissulaire, par exemple choc et septicémie, des maladies douloureuses chroniques telles que par exemple, les neuropathies diabétiques, les neuropathies induites par des chimiothérapies, des neuropathies induites par le VIH, des neuropathies post-herpétiques, des neuropathies induites par un traumatisme tissulaire, des névralgies du trijumeau, des dysfonctionnements temporo-mandibulaires, du CRPS (syndrome de douleur régionale complexe), des douleurs dorsales, des douleurs viscérales, par exemple le syndrome du côlon irritable (SCI), le syndrome intestinal inflammatoire, pour atténuer les états douloureux ou pour la prévention ou le traitement thérapeutique aigu des symptômes de bouffées de chaleur, induites par une vasodilatation et une augmentation du débit sanguin, chez les femmes ménopausées présentant un déficit en oestrogène et les patients souffrant d'un cancer de la prostate ou castrés, traités par hormonothérapie.

11. Procédé de production d'un médicament selon la revendication 8, **caractérisé en ce que**, de manière non chimique, un composé selon l'une des revendications 1 à 7 est incorporé dans un ou plusieurs véhicules et/ou diluants inertes.

12. Composés de formule générale II 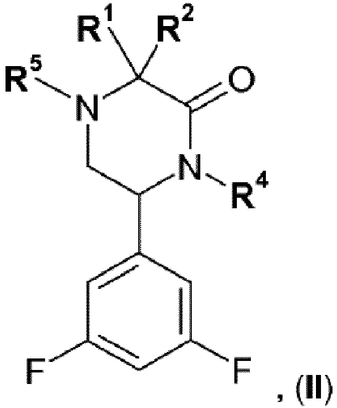 dans laquelle
R¹ représente H,
R² représente le groupe 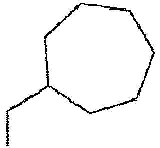
R⁴ représente H ou CH₂-C(O)-OR^{4.1},
R^{4.1} représente H, un groupe C₁₋₆-alkyle ou benzyle,
R⁵ représente H, un groupe benzyle ou -C(O)-OR^{5.1},
R^{5.1} représente un groupe C₁₋₆-alkyle ou benzyle,
leurs diastéréomères individuels, leurs énantiomères individuels et leurs sels, en particulier leurs sels physiologiquement acceptables avec des acides ou des bases inorganiques ou organiques.

13. Procédé de production de composés de formule générale II 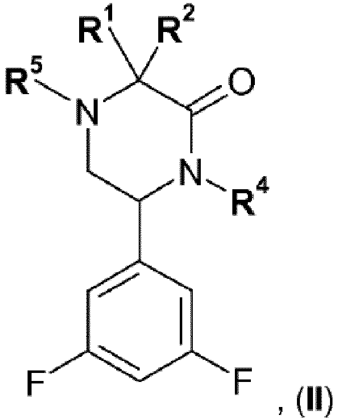 dans laquelle
R¹ représente H,
R² représente le groupe 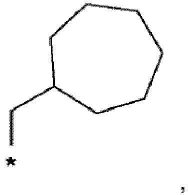
R⁴ représente H ou -CH₂-C(O)-OR^{4.1},
R^{4.1} représente H, un groupe C₁₋₆-alkyle ou benzyle,
R⁵ représente H, un groupe benzyle ou -C(O)-OR^{5.1},
R^{5.1} représente un groupe C₁₋₆-alkyle ou benzyle,
leurs diastéréomères individuels, leurs énantiomères individuels et leurs sels, en particulier leurs sels physiologiquement acceptables avec des acides ou des bases inorganiques ou organiques,
comprenant les étapes de :
(a) réaction d'un composé de formule générale III 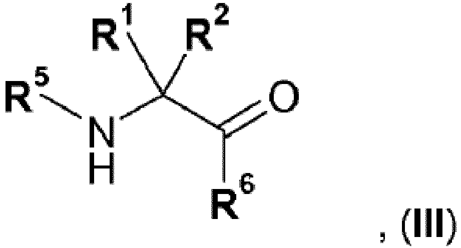 dans laquelle R¹ et R² sont tels que définis précédemment,
R⁵ représente un groupe benzyle ou -C(O)-OR^{5.1},
R^{5.1} représente un groupe benzyle et
R⁶ représente un groupe -O-C₁₋₆-alkyle,
avec un composé de formule générale IV 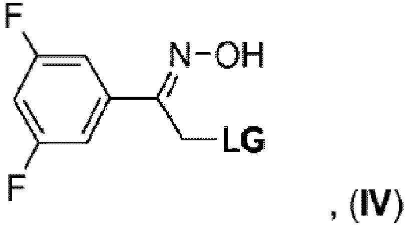 dans laquelle LG désigne un groupe partant, par exemple, un atome de brome ou d'iode, un groupe trifluorométhanesulfonyle, méthanesulfonyle ou toluènesulfonyle,
(b) conversion d'un composé de formule générale V obtenu à l'étape (a) 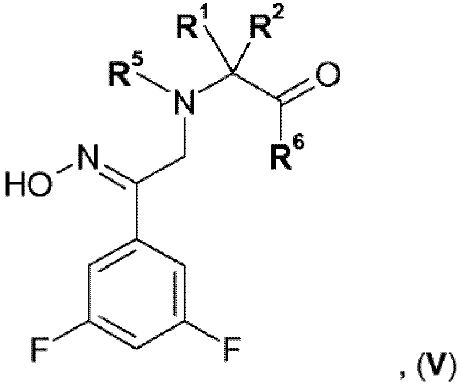 dans laquelle R¹ et R² sont tels que définis précédemment, R⁵ représente un groupe benzyle ou-C(O)-O-benzyle et R⁶ représente le groupe O-C₁₋₆-alkyle, dans des conductions réductrices, telles que par exemple, dans une atmosphère d'hydrogène, à une pression et une température élevée et en présence d'un catalyseur, par exemple, en présence de palladium sur charbon, en un composé de formule générale VI 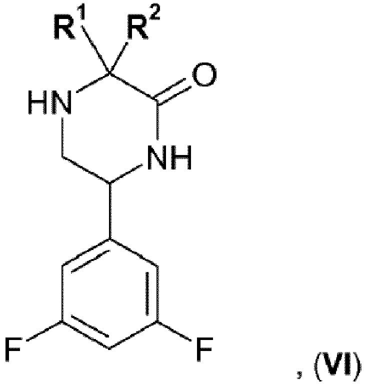 dans laquelle R¹ et R² sont tels que définis précédemment ;
(c) conversion d'un composé de formule générale VI obtenu à l'étape (b), en un composé de formule générale VII 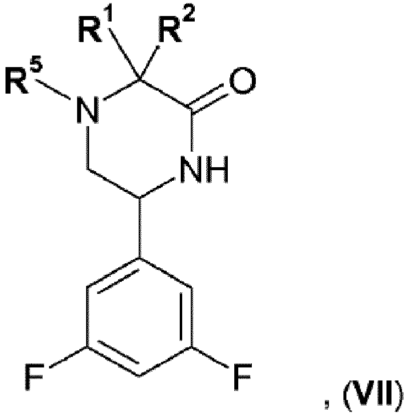 dans laquelle R¹ et R² sont tels que définis précédemment ;
R⁵ représente un groupe benzyle ou -C(O)-OR^{5.1} et
R^{5.1} représente un groupe C₁₋₆-alkyle ou benzyle ;
(d) réaction d'un composé de formule générale VII obtenu à l'étape (c) avec un composé de formule générale VIII 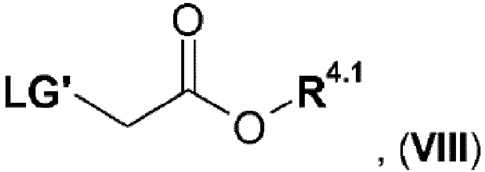 dans laquelle LG' désigne un groupe partant, par exemple, un atome de brome ou d'iode, un groupe trifluorométhanesulfonyle, méthanesulfonyle ou toluènesulfonyle,
(e) isolement d'un composé de formule générale II obtenu à l'étape (d), dans laquelle R¹, R², R⁴ et R⁵ sont tels que définis précédemment.

14. Composés de formule générale IX 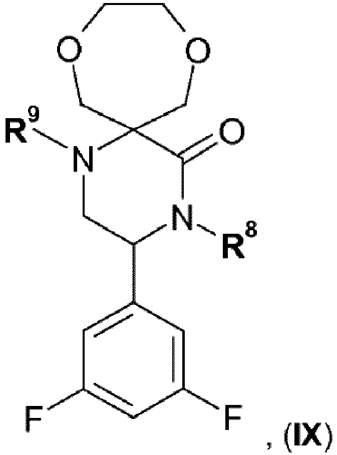 dans laquelle
R⁸ représente H ou -CH₂-C(O)-O-R^{8.1},
R^{8.1} représente H, un groupe C₁₋₆-alkyle ou benzyle et
R⁹ représente H, un groupe benzyl-O-C(O)- ou C₁₋₆-alkyl-O-C(O)-,
leurs diastéréomères individuels, leurs énantiomères individuels et leurs sels, en particulier leurs sels physiologiquement acceptables avec des acides ou des bases inorganiques ou organiques.

15. Procédé de production de composés de formule générale IX 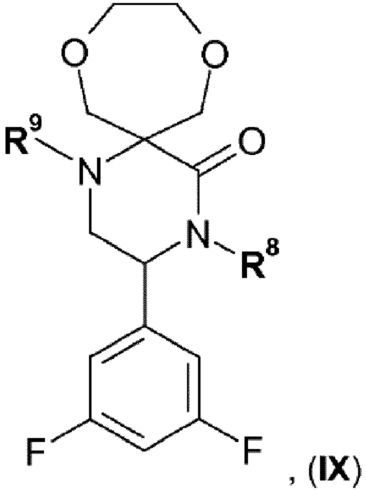 dans laquelle
R⁸ représente H ou -CH₂-C(O)-O-R^{8.1},
R^{8.1} représente H, un groupe C₁₋₆-alkyle ou benzyle et
R⁹ représente H, un groupe benzyl-O-C(O)- ou C₁₋₆-alkyl-O-C(O)-,
leurs diastéréomères individuels, leurs énantiomères individuels et leurs sels, en particulier leurs sels physiologiquement acceptables avec des acides ou des bases inorganiques ou organiques,
comprenant les étapes de :
(a) réaction d'un composé de formule générale X 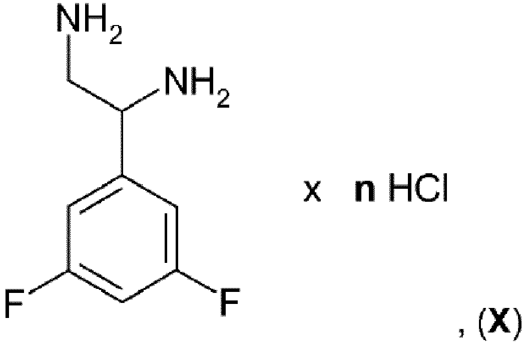 dans laquelle n désigne les chiffres 0, 1 ou 2, avec de la [1,4]-dioxépan-6-one en présence d'un catalyseur de transfert de phase, comme par exemple, le chlorure de benzyltriéthylammonium, et en présence de chloroforme et de soude caustique :
(b) isolement d'un composé de formule XI obtenu à l'étape (a) 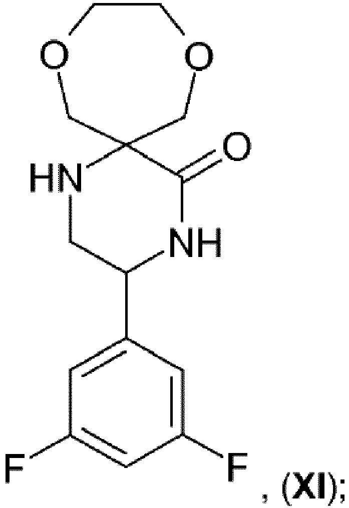
(c) conversion d'un composé de formule XI obtenu à l'étape (b) en un composé de formule générale IX 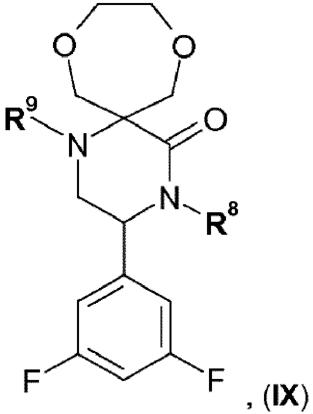 dans laquelle
R⁸ représente H et,
R⁹ représente un groupe benzyle, benzyl-O-C(O)-ou C₁₋₆-alkyl-O-C(O)- ;
(d) réaction d'un composé de formule générale IX obtenu à l'étape (c) avec un composé de formule générale VIII 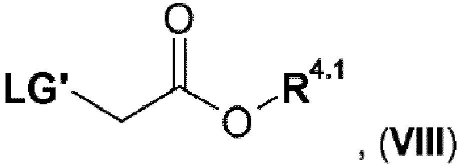 dans laquelle LG' désigne un groupe partant, par exemple, un atome de brome ou d'iode, un groupe trifluorométhanesulfonyle, méthanesulfonyle ou toluènesulfonyle,
R^{4.1} représente H, un groupe C₁₋₆-alkyle ou benzyle ; et
(e) isolement d'un composé de formule générale IX obtenu à l'étape (d) 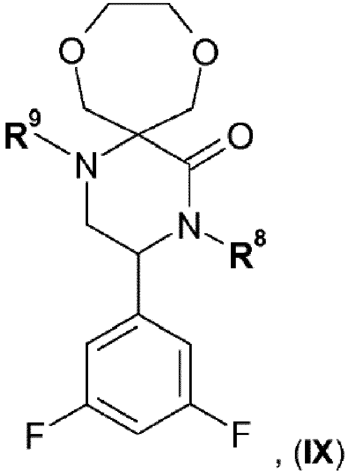 dans laquelle
R⁸ représente -CH₂-C(O)-O-R^{8.1},
R^{8.1} représente H, un groupe C₁₋₆-alkyle ou benzyle et
R⁹ représente un groupe benzyle, benzyl-O-C(O)- ou C₁₋₆-alkyl-O-C(O)-.
